(19) Europäisches Patentamt
European Patent Office
Office européen des brevets

(11) **EP 2 968 679 B1**

(12) **EUROPEAN PATENT SPECIFICATION**

(45) Date of publication and mention
of the grant of the patent:
**10.01.2018 Bulletin 2018/02**

(21) Application number: **14771007.3**

(22) Date of filing: **14.03.2014**

(51) Int Cl.:
***A61L 27/36*** *(2006.01)*      ***A61L 27/38*** *(2006.01)*

(86) International application number:
**PCT/US2014/027102**

(87) International publication number:
**WO 2014/152234 (25.09.2014 Gazette 2014/39)**

(54) **IMPROVED METHOD OF MAKING EXTRACELLULAR MATRIX COMPOSITIONS**

VERBESSERTES VERFAHREN ZUR HERSTELLUNG EXTRAZELLULÄRER MATRIXZUSAMMENSETZUNGEN

PROCÉDÉ AMÉLIORÉ DE FABRICATION DE COMPOSITIONS DE MATRICE EXTRACELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.03.2013 US 201361800532 P**

(43) Date of publication of application:
**20.01.2016 Bulletin 2016/03**

(60) Divisional application:
**17204887.8**

(73) Proprietor: **Anthrogenesis Corporation**
**Warren, NJ 07059 (US)**

(72) Inventors:
• **BHATIA, Mohit, B.**
**Manalapan, NJ 07726 (US)**
• **KAPLUNOVSKY, Aleksandr**
**Budd Lake, NJ 07828 (US)**
• **GUO, Xuan**
**Whitehouse Station, NJ 08889 (US)**

(74) Representative: **Jones Day**
**Rechtsanwälte, Attorneys-at-Law, Patentanwälte**
**Prinzregentenstrasse 11**
**80538 München (DE)**

(56) References cited:
**WO-A2-2012/142569**      **WO-A2-2013/009595**
**US-A- 5 336 616**      **US-A1- 2004 048 796**
**US-A1- 2009 138 074**      **US-A1- 2011 165 676**
**US-A1- 2012 225 484**

Remarks:
The file contains technical information submitted after the application was filed and not included in this specification

**Description**

**1. FIELD**

Provided herein are methods of producing extracellular matrix (ECM) that are superior to previously-described methods.

**2. BACKGROUND**

Extracellular matrix (ECM) comprises protein that forms many structures in the body including tendons, ligaments, and sheets that support skin and internal organs. There remains a need in the art for ECM compositions that have improved cell attachment characteristics and methods of making such ECM compositions.

**3. SUMMARY**

In one aspect, provided herein is an improved method of producing an extracellular matrix (ECM) composition from a tissue, comprising a double-drying method wherein ECM from a tissue is first dehydrated by lyophilization, then rehydrated, then heat-dried (e.g., using a vacuum heat dryer). ECM produced according to the methods provided herein is referred to herein as "double-dried ECM."

In one embodiment, provided herein is a method of producing double-dried extracellular ECM, comprising, in order: decellularizing a tissue to produce decellularized ECM; freezing the ECM; lyophilizing the ECM; rehydrating the ECM; and drying the ECM at a temperature of, e.g., 40°C to 70°C to produce said ECM composition. In certain embodiments, said double-drying method does not comprise modification of the ECM with an exogenously introduced chemical, e.g., a protease. In certain embodiments, the ECM used to produce double-dried ECM is decellularized by osmotic shock. In specific embodiments, the ECM used to produce double-dried ECM is decellularized using a detergent, detergent and osmotic shock, detergent and a base, or osmotic shock, base and detergent. In certain specific embodiments, said detergent is deoxycholic acid. The base can be, e.g., ammonium hydroxide, potassium hydroxide, or sodium hydroxide. In specific embodiments, said base is used at a concentration of less than 0.5M, 0.4M, 0.3M, 0.2M, 0.1M or 0.5M. In certain embodiments, said drying of the ECM is performed at between 50°C to 80°C. In certain embodiments, the double-dried ECM comprises collagen and one or more of laminin, fibronectin, elastin, and glycosaminoglycan.

Further provided herein is a method of culturing cells on double-dried ECM, comprising contacting the cells with double-dried ECM, and culturing the cells under conditions that allow the cells to proliferate.

Further provided herein is an extracellular matrix (ECM) for use in a method of treating a lesion in an individual, wherein the method comprises administering double-dried ECM to the individual, or contacting the individual with double-dried ECM, wherein the double-dried ECM is prepared by the method of any of claims 1-6. In certain embodiments, the individual has an oral lesion. The oral lesion may be caused by a dental procedure. In certain other embodiments, the oral lesion is not caused by a dental procedure. In specific embodiments, said oral lesion is caused by or is associated with administration of a chemotherapeutic agent to said individual. In a specific embodiment, said chemotherapeutic agent is an alkylating agent, e.g., one or more of melphalan, busulfan, cisplatin, carboplatin, cyclophosphamide, dacarbazine, ifosfamide, or mechlorethamine. In specific embodiments, the chemotherapeutic agent is an anti-metabolite, e.g., one or more of 5-fluorouracil, methotrexate, gemcitabine, cytarabine, or fludarabine. In other specific embodiments, the chemotherapeutic agent is an antibiotic having anti-tumor effect, e.g., antibiotic having anti-tumor effect is one or more of bleomycin, dactinomycin, daunorubicin, doxorubicin, or idarubicin. In other specific embodiments, said chemotherapeutic agent is a mitotic inhibitor, e.g., one or more of paclitaxel, docetaxel, etoposide, vinblastine, vincristine or vinorelbine. In certain embodiments, the oral lesion, or a plurality of said oral lesions, has caused or is expected to cause premature termination of a course of therapy comprising said chemotherapeutic agent. In certain other embodiments, the oral lesion is caused by or is associated with administration of an antibody to said individual, e.g., one or more of rituximab, of a tumumab, veltuzumab, ocrelizumab, adalimumab, etanercept, infliximab, certolizumab pegol, natalizumab or golimumab. In certain other embodiments, the oral lesion is caused by or is associated with hematopoietic stem cell transplantation, or bone marrow transplantation, to said individual. The oral lesion may be caused by or associated with graft-versus-host disease in said individual, or radiation that has been administered to said individual. In certain embodiments, administration of said double-dried ECM results in an improvement of said oral lesion of at least one Grade according to the World Health Organization Oral Toxicity (WHO-OT) score within 7 days post-administration; an improvement of said oral lesion of at least one Grade according to the National Cancer Institute Common Toxicity Criteria (NCI-CTC) for Oral Mucositis within 7 days post-administration; an improvement of said oral lesion of at least 1 point in any subscore of the Oral Mucositis Assessment Scale (OMAS) 7 days post-administration, and/or an improvement of said oral lesion of at least one Stage according to the Western Consortium for Cancer Nursing Research (WCCNR) score within 7 days post-administration.

In certain embodiments, the double-dried ECM is formed as a sheet or tube, is used as a nerve guide, tendon wrap, or dural replacement.

In certain embodiments the double-dried ECM provided herein comprises a plurality of stem cells, e.g., CD10+, CD34-CD105+, CD100+ placental stem cells. The double-dried ECM may comprise other stem cells or differentiated cells, as described elsewhere herein, e.g., embryonic stem cells, embryonic germ cells, mesenchymal stem cells, bone marrow-derived stem cells, hematopoietic progenitor cells (e.g., hematopoietic stem cells from peripheral blood, fetal blood, placental blood, umbilical cord blood, placental perfusate, etc.), somatic stem cells, neural stem cells, hepatic stem cells, pancreatic stem cells, endothelial stem cells, cardiac stem cells, muscle stem cells, adipose stem cells, and the like.

In specific embodiments, the stem cells are placental stem cells. In a more specific embodiment, said placental stem cells are CD34- and/or CD200+. In a specific embodiment, the placental stem cells are CD34-, CD10+, CD105+ and CD200+. In certain specific embodiments, the placental stem cells can express one or more of CD10, CD73, CD105, CD200, and/or OCT-4, and lack expression of one or more of CD34, CD38, CD45, and/or HLA-G. In certain other specific embodiments, the placental stem cells can also express HLA-ABC (MHC-1) and lack expression of HLA-DR. In another specific embodiment, the placental stem cells are CD200+ and HLA-G-. In another specific embodiment, the placental stem cells are CD73+, CD105+, and CD200+. In another specific embodiment, the placental stem cells are CD200+ and OCT-4+. In another specific embodiment, the placental stem cells are CD73+, CD105+ and HLA-G-. In another specific embodiment, the placental stem cells are CD73+ and CD105+, and, when in a population of placental cells, facilitate formation of one or more embryoid-like bodies under conditions that allow formation of embryoid-like bodies. In another specific embodiment, the placental stem cells are OCT-4+ and, when in a population of placental cells, facilitate formation of one or more embryoid-like bodies in a population of isolated placental stem cells comprising said stem cell when cultured under conditions that allow formation of embryoid-like bodies.

In another specific embodiment, the stem cells are adhered to the composition. In a specific embodiment of any of the above embodiments, the stem cells secrete IL-6, IL-8 and/or MCP-1 (monocyte chemotactic protein-1) when contacted with the composition.

In another aspect, provided herein are kits for administering double-dried ECM to an individual having an oral lesion. The kits typically comprise double-dried ECM in a package convenient for distribution to a practitioner of skill in the art.

## 4. BRIEF DESCRIPTION OF THE FIGURES

FIG. 1: Proliferation of AMDACs on double-dried ECM, as measured by MTS assay, with optical density read at 490nm; greater density indicates more rapid proliferation (greater cell number) over 1, 3, 7 and 10 days of culture.

FIG. 2: Migration of keratinocytes as measured in a transwell assay.

FIG. 3: Production of soluble cytokines by AMDACs cultures on double-dried ECM, or on tissue culture plastic (Plate) as determined by ELISA. 3A: TGF-$\beta$; 3B: IL-6; 3C: bFGF; 3D: IL-8; 3E: vascular endothelial growth factor (VEGF); 3F: IL-10; 3G: granulocyte-macrophage colony stimulating factor (GM-CSF).

FIG. 4: AMDACs-hpECM cultures showed increasing secretion of soluble fibronectin from day 1 to day 6 of culture.

## 5. DETAILED DESCRIPTION

### 5.1. Double-Dried Extracellular Matrix Composition

In one embodiment, provided herein is a method of producing double-dried extracellular matrix (ECM), comprising, in order: decellularizing a tissue to produced decellularized ECM (e.g., as provided in Sections 5.3-5.5, below); freezing the ECM; lyophilizing the ECM; rehydrating the ECM; and drying the ECM at a temperature of 40°C to 70°C to produce said ECM composition. In certain embodiments, said method does not comprise modification of the ECM with an exogenously introduced chemical, e.g., a protease. In certain embodiments, said drying the ECM is performed at between 50°C to 70°C. Drying of the ECM at room temperature after rehydration (e.g., by application of a vacuum) does not suffice to produce the double-dried ECM provided herein that has the requisite cell attachment and proliferation characteristics.

Said freezing of the ECM can take place at any temperature below 0°C, e.g., at -10°C, -15°C, -20°C, -25°C, -30°C, -35°C, -40°C, -45°C, -50°C, -55°C, -60°C, -65°C, -70°C, -75°C, -80°C, or -85°C, or colder. In certain embodiments, said freezing takes place at a temperature of 0°C to -10°C, -10°C to -20°C, -20°C to -30°C, -30°C to -40°C, -40°C to -50°C, -50°C to -60°C, -60°C to -70°C, or -70°C, to -80°C.

Lyophilization can be accomplished by any means known in the art, and generally proceeds until the ECM composition is substantially dry, e.g., less than about 30%, 25%, 20%, 25%, 20%, 5%, 4%, 3%, 2% or 1% water by weight.

Rehydration of the lyophilized ECM can, for example, be accomplished with any therapeutically or medically-acceptable liquid, buffer, cell culture media, excipient, or the like. In certain embodiments, the liquid used to rehydrate the ECM comprises one or more biomolecules that, e.g., assist cells subsequently added to the ECM to proliferate, assist repair of a tissue in a recipient of the ECM, or both.

After rehydration, and prior to heat drying, the ECM may be formed into any useful shape, e.g., block, sheet, tube (e.g., a closed tube or a tube with a lengthwise slit along part or all of the length of the tube), or other shape. The ECM may then be heat dried to complete formation of the ECM into the desired shape. If the ECM is to be modified or derivatized, in certain embodiments, such modification or derivatizationis performed after the double drying procedure is complete.

In certain embodiments, the double dried ECM is prepared as sheets, e.g., 3.5"x3.5" sheets.

### 5.1.1. Extracellular Matrix Compositions

ECM compositions used in the double drying method provided above may, in certain embodiments, be obtained from a mammalian source, e.g., a human, bovine, ovine, sheep, rat source. The ECM compositions may be obtained from a marsupial, e.g., a kangaroo. In certain embodiments, the ECM is obtained from a non-mammalian source, e.g., the ECM is obtained from fish.

The ECM can be obtained from any portion of the source. In certain embodiments, the ECM can be obtained from, e.g., bovine skin, calf skin, rat tail, kangaroo tail or fish skin. In particular embodiments, the ECM is obtained from placenta, e.g., bovine placental ECM, ovine placental ECM or human placental ECM.

A principal component of the ECM, e.g., human placental ECM, is collagen. The collagen can be any type of collagen known to those of skill in the art or a mixture of such collagens. In certain embodiments, the collagen is in the form of a collagen composition that comprises one or more types of collagen. Particular collagens include type I collagen, type II collagen, type III collagen and type IV collagen. In certain embodiments, ECM comprises particular amounts of these collagens. In a particular embodiment, the ECM comprises a substantial amount of type I collagen while also being enriched in type IV collagen. In certain embodiments, the ECM comprises between 1% and 15% type IV collagen, between 2% and 13% type IV collagen, between 3% and 12% type IV collagen or between 4% and 11% type IV collagen by dry weight. In certain embodiments, the ECM comprises at least 75%, at least 80%, at least 85%, at least 90%, at least 95%, or at least 99% type I collagen by dry weight. In certain embodiments, the ECM comprises between 70% and 95% type I collagen, between 74% and 92% type I collagen or between 80% and 90% type I collagen by dry weight. In certain embodiments, the ECM comprises type III collagen, for instance up to 1%, up to 2%, up to 3%, up to 4%, up to 5%, up to 6% or up to 7% type III collagen by dry weight. In certain embodiments, the ECM comprises between 2% and 15% type IV collagen, between 70% and 95% type I collagen and up to 6% type III collagen, by dry weight.

In certain embodiments, the ECM comprises one or more extracellular matrix proteins or components in addition to collagens. For example, in specific embodiments, the ECM comprises one or more of fibronectin, laminin, elastin, and/or glygosaminoglycan. In other specific embodiments, the ECM comprises no detectable fibronectin, or no detectable laminin, or no detectable laminin or fibronectin. In another specific embodiment, the ECM comprises detectable amounts of fibronectin and laminin. In another specific embodiment, the ECM comprises about 5% or more elastin by dry weight. In another specific embodiment, the ECM comprises about 10% or more elastin by dry weight. In another specific embodiment, the ECM comprises no more than about 5% elastin by dry weight.

In certain embodiments, the ECM comprises less than 1%, less than 0.5%, less than 0.1%, less than 0.05%, or less than 0.01% laminin, or comprise between 0.01%-0.05%, 0.05%-0.1%, 0.1%-0.5%, or 0.5%-1.0% laminin, as compared to the total amount of protein in the composition (e.g., by dry weight). In certain embodiments, the ECM comprises less than 1%, less than 0.5%, less than 0.1%, less than 0.05%, or less than 0.01% fibronectin, or comprise between 0.01%-0.05%, 0.05%-0.1%, 0.1%-0.5%, or 0.5%-1.0% fibronectin, as compared to the total amount of protein in the composition (e.g., by dry weight). In certain embodiments, the ECM comprises about 1%, 2%, 3%, 4%, 5%, 6%, 7%, 8%, 9%, 10%, 11%, 12%, 13%, 14%, or 15% elastin or between 1-5%, 5-10%, or 10-15% elastin, and comprises less than 1%, less than 0.5%, less than 0.1%, less than 0.05%, or less than 0.01% laminin, or comprises between 0.01%-0.05%, 0.05%-0.1%, 0.1%-0.5%, or 0.5%-1.0% laminin; and/or less than 1%, less than 0.5%, less than 0.1%, less than 0.05%, or less than 0.01% fibronectin, or comprises between 0.01%-0.05%, 0.05%-0.1%, 0.1%-0.5%, or 0.5%-1.0% fibronectin, as compared to the total amount of protein in the composition (e.g., by dry weight). In a specific embodiment, the ECM comprises greater than 80% collagen, between 10-15% elastin, less than 0.01% laminin, and less than 0.01% fibronectin, as compared to the total amount of protein in the composition (e.g., by dry weight).

In certain embodiments, the ECM utilized in the double-drying methods can be obtained by one of the processes described in the sections below.

In certain embodiments, at least the collagen in the ECM to be double-dried is cross-linked, e.g., with a cross-linker. In certain embodiments, the cross-linker is glutaraldehyde. See, e.g., U.S. Pat. Nos. 4,852,640, 5,428,022, 5,660,692 and 5,008,116, and in McPherson et al., 1986, J. Biomedical Materials Res. 20: 79-92. Further exemplary cross-linkers and methods of cross-linking collagen are described in U.S. Pat. Nos. 5,880,242 and 6,117,979 and in Zeeman et al., 2000, J Biomed Mater Res. 51(4):541-8, van Wachem et al., 2000, J Biomed Mater Res. 53(1):18-27, van Wachem et al., 1999, J Biomed Mater Res. 47(2):270-7, Zeeman et al., 1999, J Biomed Mater Res. 46(3):424-33, Zeeman et al., 1999, Biomaterials 20(10):921 -31.

In further embodiments collagen in the ECM to be double-dried is cross-linked with 1,4-butanediol diglycidyl ether. In further embodiments collagen in the ECM is cross-linked with genipin, a non-toxic, naturally occurring crosslinking agent. Genipin can be obtained from its parent compound, geniposide, which may be isolated from the fruits of *Gardenia jasminoides.* Genipin may be obtained commercially from Challenge Bioproducts Co., Ltd., 7 Alley 25, Lane 63, TzuChiang St. 404 Taichung Taiwan R.O.C., Tel 886-4-3600852. The use of genipin as a cross-linking reagent is described extensively in U.S. Patent Application Publication No. 2003/004930 1.

The collagen in the ECM to be double-dried can be cross-linked with a single cross-linker or with a mixture of cross-linkers. In certain embodiments, ECM comprises base-treated, detergent treated human placental collagen cross-linked with glutaraldehyde.

The collagen in the ECM to be double-dried can be cross-linked with any enzyme-mediated crosslinking technique known to those of skill in the art. For instance, the collagen in the ECM can be cross-linked by transglutaminase, e.g., by the methods described, for example, in Orban et al., 2004, J. Biomedical Materials Res. 68(4):756-62.

### 5.1.2. Processes for Preparation of ECM

In certain embodiments, the ECM useful for producing double-dried ECM is prepared from human placenta according to the methods described herein. The placental tissue can be from any part of the placenta including the amnion, whether soluble or insoluble or both, the chorion, the umbilical cord or from the entire placenta. In certain embodiments, the ECM is prepared from whole human placenta without the umbilical cord. In certain other embodiments, the ECM is prepared from whole placenta without the amniotic membrane or umbilical cord.

The placenta from which ECM is obtained is preferably taken as soon as possible after normal delivery, or after cesarean section delivery, of a normal healthy infant. Advantageously, the placenta is collected under aseptic conditions. The placenta, in certain embodiments, is stored for 48 hours from the time of delivery prior to any further treatment. In other embodiments, the placenta is stored for up to 5 days from the time of delivery prior to any further treatment.

The placenta and optionally umbilical cord can be transported from the delivery or birthing room to another location, e.g., a laboratory, for further processing. The placenta can be transported in a sterile, transport device such as a sterile bag or a container, which is optionally thermally insulated. In some embodiments, the placenta is stored at room temperature until further treatment. In other embodiments, the placenta is refrigerated until further treatment, i.e., stored at a temperature of about 2°C to 8°C. The placenta may be stored under sterile conditions for up to 5 days before further treatment. The placenta is preferably handled and processed under aseptic conditions, as known to persons skilled in the art, e.g., in a laboratory can be equipped with an HEPA filtration system (as defined by clean room classification, having a class 1000 or better).

The placenta is preferably exsanguinated, i.e., completely drained of the cord blood remaining after birth, prior to obtaining the ECM. In some embodiments, the placenta is 70% exsanguinated, 80% exsanguinated, 90% exsanguinated, 95% exsanguinated or 99% exsanguinated.

The expectant mother may be screened for known pathogens prior to the time of birth, using standard techniques known to one skilled in the art, for communicable diseases including but not limited to, HIV, HBV, HCV, HTLV, syphilis, CMV, and other viral pathogens known to contaminate placental tissue, e.g., following FDA regulations. The expectant mother may be screened (e.g., a blood sample is taken for diagnostic purposes) within one month of birth, particularly within two weeks of birth, within one week of birth, or at the time of birth. Preferably, only tissues collected from donors who tested negative or non-reactive to the above-mentioned pathogens are used to produce ECM. Advantageously, a thorough paternal and medical and social history of the donor of the placental membrane is obtained, including for example, a detailed family history.

In certain embodiments, the donor is screened using standard serological and bacteriological tests known to persons skilled in the art. For example, donor screening can encompass using standard antigen-detection techniques known to one skilled in the art using, e.g., antibody screen (ATY); alanine amino transferase screening (ALT); Hepatitis Core Antibody (nucleic acid and ELISA); Hepatitis B Surface Antigen; Hepatitis C Virus Antibody; HIV-1 and HIV-2; HTLV-1 and HTLV-2; Syphilis test (RPR); CMV antibody test; and/or Hepatitis C and HIV test. The assays used may be nucleic acid based assays or ELISA based assays as known to one skilled in the art.

Blood from the umbilical cord of the newborn using standard techniques known to one skilled in the art (See, e.g., Cotorruelo et al., 2002, Clin. Lab. 48(5 6):271 81; Maine et al., 2001, Expert Rev. Mol. Diagn., 1(1):19 29; Nielsen et al., 1987, J. Clin. Microbiol. 25(8):1406 10). In one embodiment, blood from the umbilical cord of the newborn is tested for bacterial pathogens (including but not limited to gram positive and gram negative bacteria) and/or fungi using standard techniques known to persons skilled in the art. The blood type and Rh factor of the blood of the umbilical cord of the newborn can be determined using standard techniques known to those skilled in the art. In another embodiment, complete blood count (CBC) with differential is obtained from the blood from the umbilical cord of the newborn using standard methods known to one skilled in the art. In yet another embodiment, an aerobic bacterial culture is taken from the blood from the umbilical cord of the newborn, using standard methods known to one skilled in the art. Only tissues collected from donors that have a CBC within a normal limit (e.g., no gross abnormality or deviation from the normal level), test negative for serology and bacteriology, and test negative or non-reactive for infectious disease and contamination are used to produce the ECM.

Once the human placental tissue is obtained, it can be treated according to the following steps in order to prepare the ECM. Although the following steps are presented in sequential order, one of skill in the art will recognize that the order of several steps can be interchanged. It is assumed that techniques readily apparent to those of skill in the art such as buffer exchange, precipitation, centrifugation, resuspension, dilution and concentration of protein compositions need not be explained in detail. Exemplary preparation is described in the examples below.

Any portion of the placenta, or the entire placenta, can be used in the processes described herein. In certain embodiments, ECM is prepared from whole placenta, or from chorionic or amnionic portions of the placenta.

The umbilical cord may be separated from the placental disc, and the amniotic membrane may be separated from the chorionic membrane. The amniotic membrane may be separated from the chorionic membrane prior to cutting the placental membrane. Separation of the amniotic membrane from the chorionic membrane can be done starting from the edge of the placental membrane, and can be separated from the chorionic membrane using blunt dissection, e.g., with gloved fingers. Following separation of the amniotic membrane from the chorionic membrane and placental disc, the umbilical cord stump may be cut, e.g., with scissors, and detached from the placental disc. In certain embodiments, when separation of the amniotic and chorionic membranes is not possible without tearing the tissue, the amniotic and chorionic membranes may be cut from the placental disc as one piece and then peeled them apart.

The amniotic membrane, chorionic membrane or whole placenta can be stored prior to use in the methods described herein. Exemplary storage techniques are described in U.S. Patent Application Publication Nos. 2004/0048796 and 2003/018751 5.

The placental tissue may be decellularized prior to obtaining the ECM. The placental tissue can be decellularized according to any technique known to those of skill in the art, e.g., techniques described in U.S. Patent Application Publication Nos. 2004/0048796 and 2003/01875 15.

In certain embodiments, the placental tissue is subjected to an osmotic shock. Although not intending to be bound by any particular theory of operation, it is believed that the osmotic shock can burst cells in the tissue and thereby facilitating removal of cells, cellular components and blood components. Osmotic shock can be in addition to any clarification step or it can be the sole clarification step.

The osmotic shock can be carried out in any osmotic shock conditions known to those of skill in the art. Such conditions include incubating the tissue in solutions of high osmotic potential, or of low osmotic potential or of alternating high and low osmotic potential. The high osmotic potential solution can be any high osmotic potential solution known to those of skill in the art such as a solution comprising one or more of NaCl (e.g., 0.2M to 1.0M), KCl (e.g., 0.2M to 1.0M or 2.0M), ammonium sulfate, a monosaccharide, a disaccharide (e.g., 20% sucrose), a hydrophilic polymer (e.g., polyethylene glycol), glycerol, etc. In certain embodiments, the high osmotic potential solution is a sodium chloride solution. In some embodiments, the sodium chloride solution is at least 0.25M, 0.5M, 0.75M, 1.0M, 1.25M, 1.5M, 1.75M, 2M, 2.25M or 2.5M NaCl. In some embodiments, the sodium chloride solution is about 0.25M to 5M, about 0.5M to 4M, about 0.75M to 3M, or about 1.0M to 2.0M NaCl.

The low osmotic potential solution can be any low osmotic potential solution known to those of skill in the art, such as water, for example water deionized according to any method known to those of skill. In some embodiments, the osmotic shock solution comprises water with an osmotic shock potential less than that of 50 mM NaCl.

In certain embodiments, the osmotic shock is accomplished using a sodium chloride solution followed by a water solution. In various embodiments, the sodium chloride solution is at least 0.5M NaCl, at least 0.75M NaCl, at least 1.0M NaCl, at least 1.5M NaCl, or at least 2.0M NaCl. In certain embodiments, one 0.5M NaCl treatment is followed by a water wash. In certain embodiments, two consecutive 0.5M NaCl treatments are followed by a water wash. In certain embodiments, one 2M NaCl treatment is followed by a water wash. These sequences can be repeated according to the judgment of one of skill in the art.

In certain embodiments, the collagen composition resulting from the osmotic shock can be incubated with a detergent. The detergent can be any detergent known to those of skill in the art to be capable of disrupting cellular or subcellular membranes. In certain embodiments, the detergent is ionic. For instance, in certain embodiments, the detergent is deoxycholate, deoxycholic acid or sodium dodecylsulfate. In certain embodiments, the detergent is zwitterionic.

In certain embodiments, the detergent is nonionic. For instance, in certain embodiments, the detergent can be a TWEEN® detergent, such as TWEEN®-20, or a Triton X detergent, such as Triton X 100. The collagen composition can be contacted with the detergent under conditions judged by one of skill in the art to be suitable for removing unwanted components from the composition. Exemplary conditions are provided in the working examples below.

The detergent treatment can be carried out at any temperature according to the judgment of those of skill in the art. In certain embodiments, the detergent treatment is carried out at about 0°C to 30°C., about 5°C to 25°C, about 5°C to 20°C, or about 5°C to 15°C. In certain embodiments, the detergent treatment is carried out at about 0°C, about 5°C, about 10°C, about 15°C, about 20°C, about 25°C, or about 30°C. In particular embodiments, the detergent treatment is carried out at about 5°C to 15°C.

The detergent treatment can be carried out for a suitable time according to the judgment of those of skill in the art. In certain embodiments, the detergent treatment can be carried out for about 1-24 hours, about 2-20 hours, about 5-15 hours, about 8-12 hours, or about 2-5 hours.

In certain embodiments, the ECM resulting from the detergent treatment can be optionally treated one or more times with a base, e.g., by washing the ECM in a basic solution. In certain embodiments, the base removes endotoxins and/or viral particles. Exemplary bases for the basic treatment include biocompatible bases, volatile bases or bases known to those of skill in the art to be easily and safely removed from the ECM. The base can be any organic or inorganic bases known to those of skill in the art at a concentration of, for example, 0.2M to 1.0M. In certain embodiments, the base is ammonium hydroxide, potassium hydroxide or sodium hydroxide, e.g., an ammonium hydroxide solution, potassium hydroxide solution or sodium hydroxide solution. The sodium hydroxide solution can be 0.1M NaOH, 0.25M NaOH, 0.5M NaOH, or 1M NaOH. In particular embodiments, the basic treatment is carried out in 0.1M or 0.5M NaOH.

The base treatment can be carried out at any temperature according to the judgment of those of skill in the art. In certain embodiments, the basic treatment is carried out at about 0°C to 30°C, about 5°C to 25°C, about 5°C to 20°C, or about 5°C to 15°C. In certain embodiments, the basic treatment is carried out at about 0°C, about 5°C, about 10°C, about 15°C, about 20°C, about 25°C, or about 30°C. In particular embodiments, the basic treatment is carried out at about 5°C to 15°C.

The basic treatment can be carried out for a suitable time according to the judgment of those of skill in the art. In certain embodiments, the basic treatment can be carried out for about 1-24 hours, about 2-20 hours, about 5-15 hours, about 8-12 hours, or about 2-5 hours.

Variations of the detergent and NaOH wash steps can be used to generate a number of variations of the final ECM material. For example, in certain embodiments, the ECM-containing tissue can be treated with about 0.1M, 0.2M, 0.3M, 0.4M, or about 0.5M NaOH over about 3, 4, 5, 6, 7, 8, 9, 10, 11, 12, 13, 14, 15, 16, 17, 18, 19, 20, 21, 22, 23, or about 24 hours.

In certain other embodiments, the ECM is produced without treatment by a base. Where the process is applied to placental tissue, omission of a base treatment step typically results in a ECM comprising relatively higher amounts of elastin, fibronectin and/or laminin than the ECM produced with inclusion of the basic treatment.

In certain embodiments, any or all of the above steps are carried out under sterile conditions. In particular embodiments, the basic treatment, and all subsequent steps, are carried out under sterile conditions. In further embodiments, any collagen composition prepared according to the methods described herein can be further sterilized according to techniques apparent to one of skill in the art.

In certain embodiments, a method of preparing the ECM comprises osmotic shock, freeze dry, detergent treatment, water wash, freeze dry, basic treatment, water wash and freeze dry steps described above, carried out in order. In certain embodiments, the detergent is 1% deoxycholate. In certain embodiments, the basic treatment is 0.5 N NaOH for four hours. In certain embodiments, the first water wash is repeated (two total washes). In certain embodiments, the second water wash is repeated twice (three total washes). In certain embodiments, the detergent is 1% deoxycholate, the basic treatment is 0.5 N NaOH for four hours, the first water wash is repeated (two total washes) and the second water wash is repeated twice (three total washes). In certain embodiments, such a process can provide a composition comprising about 0.59% glycosaminoglycans, about 3.5% elastin, little or no fibronectin and little or no laminin.

In certain embodiments, ECM is obtained by subjecting tissue, e.g., placental tissue, to osmotic shock, base treatment, and water wash, e.g., as described above. In certain embodiments, these steps are carried out in order. In certain embodiments, the base treatment is 0.5 N NaOH for four hours. In certain embodiments, the ECM resulting from such preparation comprises about 0.28% to about 0.38% glycosaminoglycans, about 3.2% to about 4.7% elastin, little or no (e.g., less than 1%) fibronectin and little or no (e.g., less than 1%) laminin, by dry weight.

In certain embodiments, ECM is obtained by subjecting tissue, e.g., placental tissue, to osmotic shock, detergent treatment and water wash steps, e.g., as described above. In certain embodiments, these steps are carried out in order. In certain embodiments, the detergent is 1% deoxycholate. In certain embodiments, such a process can provide a composition comprising about 0.4% glycosaminoglycans, about 12% elastin, about 0.6% fibronectin and about 0.16% laminin.

### 5.1.3. Optional Further Treatment

In certain embodiment the ECM to be double-dried comprises telopeptide collagen. In certain embodiments, the ECM to be double-dried comprises atelopeptide collagen. In yet other embodiments, an ECM composition comprising telopeptide collagen can be used as a source for an atelopeptide collagen composition. The atelopeptide collagen composition can be used for any purpose apparent to those of skill in the art for atelopeptide collagen.

In such embodiments, ECM can be contacted with an enzyme capable or partially or completely removing telopeptides from the collagen contained therein. The enzyme can be any proteolytic enzyme known to those of skill in the art that is capable of removing telopeptides from collagen. In certain embodiments, the enzyme is pepsin or papain. Generally, the enzyme is contacted with the collagen composition under conditions suitable for removal of telopeptide known to those of skill in the art. Methods of treating collagen compositions with enzymes to remove telopeptides are described in, e.g., U.S. Pat. Nos. 4,511,653, 4,582,640, 5,436,135 and 6,548,077.

In certain embodiments, the collagen composition is contacted with pepsin at about 15°C to 40°C, about 20°C to 35°C, about 25°C to 30°C, about 20°C to 30°C, or about 23°C to 27°C. In particular embodiments, the collagen composition is contacted with pepsin at about 23°C to 27°C. for a time sufficient to remove telopeptide. The collagen composition may be contacted with the enzyme for a time sufficient to remove telopeptide. In certain embodiments, for example, the collagen is contacted with pepsin for at least 5, 10, 15, 20, 25 or 30 hours. In certain embodiments, the is contacted with pepsin for about 5 to 30 hours, about 10 to 25 hours or about 20 to 25 hours. In certain embodiments, the is contacted with pepsin for about 8, 16, 24 or 32 hours.

The collagen composition is, in certain embodiments, contacted with the enzyme in an amount suitable to remove substantially all telopeptide from the ECM. In some embodiments, about 0.1 g, 0.5 g, 1.0 g, 2.0 g or 5.0 g pepsin per kg ECM dry weight is contacted with the ECM. In other embodiments, about 0.1 g, 0.5 g, 1.0 g, 2.0 g or 5.0 g pepsin/placenta is contacted with the ECM. In certain embodiments, the collagen composition is contacted with about 0.1 to 10.0 g/L, about 0.5 to 5/L, about 1 to 2.5 g/L, or about 0.5 1.5 g/L pepsin. In some embodiments, the collagen composition is contacted with about 0.1 g/L, about 0.2 g/L, about 0.5 g/L, about 1.0 g/L, about 2.0 g/L, 5 g/L or 10 g/L pepsin. In particular embodiments, the collagen composition is contacted with about 0.5 to 1.0 g/L pepsin in acetic acid solution with pH about 2-3, at about 23°C to 27°C for about 16-24 hours.

The ECM may be contacted with the enzyme in a suitable solution volume:placenta to remove telopeptide. It is observed that a high volume ratio to placenta can maximize the effect by pepsin. In certain embodiments, about 1, 2, 4, or 8 volumes of acetic acid solution per placenta is used. In particular embodiments, about 2 volumes of acetic acid solution per placenta is used.

If desired, the ECM can be further processed by fibrillation, e.g., as described in U.S. Pat. Nos. 4,511,653, 4,582,640 and 5,436,135. If necessary, the collagen composition can be concentrated according to standard techniques prior to fibrillation.

Where desired, the ECM can be cross-linked. In certain embodiments, the ECM is fibrillated prior to cross-linking. The cross-linking can be with any cross-linker known to those of skill in the art, for instance, the cross-linkers described above. In certain embodiments, the cross-linker is glutaraldehyde, and the cross-linking can be carried out according to methods of glutaraldehyde cross-linking of collagen known to those of skill in the art. In other embodiments, the cross-linker is 1,4-butanediol diglycidyl ether or genipin.

In some embodiments, a covalent bond between a cross-linker and a collagen can be reduced, for example to improve stability. The reduction can be accomplished by contacting the ECM, e.g., the collagen in the ECM, with any reducing agent known to those of skill in the art. In certain embodiments, the reducing agent is sodium borohydride, sodium bisulfite, β-mercaptoethanol, mercaptoacetic acid, mercaptoethylamine, benzyl mercaptan, thiocresol, dithiothreitol or a phosphine such as tributylphosphine. In certain embodiments, the collagen in the ECM is cross-linked prior to reduction with the reducing agent. Reduction of collagen compositions and cross-linked collagen compositions is described in U.S. Pat. Nos. 4,185,011, 4,597,762, 5,412,076 and 5,763,5 79.

In certain embodiments, the ECM can be further processed by mechanical shearing according to methods known to those of skill in the art. Exemplary shearing techniques are described in U.S. Pat. No. 4,642,11 7. In certain embodiments, the ECM is sheared with a tissue homogenizer.

In certain embodiments, steps can be taken to limit native protease activity in the ECM. Suboptimal conditions for proteases may be achieved by formulating the compositions to eliminate or limit the amount of calcium and zinc ions available in solution. Many proteases are active in the presence of calcium and zinc ions and lose much of their activity in calcium and zinc ion free environments. Additives such as metal ion chelators, for example 1,10-phenanthroline and ethylenediaminetetraacetic acid (EDTA), therefore create an environment unfavorable to many proteolytic enzymes. Advantageously, ECM can be prepared selecting conditions of pH, reduced availability of calcium and zinc ions, presence of metal ion chelators and the use of proteolytic inhibitors specific for collagenase. For example, ECM may include a buffered solution of water, pH 5.5 to 8, or pH 7 to 8, free from calcium and zinc ions and including a metal ion chelator such as EDTA. Additionally, control of temperature and time parameters during the treatment of a collagen composition may also be employed to limit the activity of proteases.

### 5.2. Characterization of ECM

### 5.2.1. Biochemical Characterization

Biochemical based assays known in the art and exemplified herein may be used to determine the biochemical compositions of the ECM, either before or after the double-drying method of Section 5.2. Absorbance based assays, e.g., for protein content, include but are not limited to assays that measure absorbance at 280 nm (see, e.g., Layne, E, Spectrophotometric and Turbidimetric Methods for Measuring Proteins, Methods in Enzymology 3: 447-455, (1957); Stoscheck, C M, Quantitation of Protein, Methods in Enzymology 182: 50-69, (1990)), 205 nm, and assays based on the extinction coefficient of the sample (see, e.g., Scopes, R K, Analytical Biochemistry 59: 277, (1974); Stoscheck, C M. Quantitation of Protein, Methods in Enzymology 182: 50-69, (1990)). ECM may be characterized using known assays for, e.g., one or more of collagen type I, collagen type II, collagen type III, collagen type IV, laminin, elastin, fibronectin, and/or glycosaminoglycan.

Colorimetric based assays included but are not limited to modified Lowry assay, biuret assay, Bradford assay, Bicinchoninic Acid (Smith) assay (see, e.g., Stoscheck, C M, Quantitation of Protein, Methods in Enzymology 182: 50-69 (1990)).

In a specific embodiment, measuring the total protein content of ECM comprises use of a Bradford dye-binding assay (Bradford, M., Analytical Biochemistry, 72, 248 (1976). A Bradford assay may be carried out, e.g., using the Bradford dye-binding assay available through BIO-RAD, Hercules, Calif., USA. The protein assay is based on the change in color of the dye Coomassie Brilliant Blue R-250 in response to different concentrations of protein. The assay involves developing a standard calibration curve by measuring absorbance (at 595 nanometers) of a series of human collagen standards of known concentrations. The concentration of collagen in an ECM test sample, for example, sample of the amniotic membrane, is determined by referencing to the standard curve. The assay is developed in a standard format that allows measurement of collagen concentration in the range of 0.2-1.4 mg/mL and as a microassay that measures protein concentration up to 25 $\mu$g. For the standard assay, ECM dissolved in 100 mM citric acid (pH 2.4) is aliquoted into 1.5 mL microcentrifuge tubes at concentrations of 0.1-1 mg/mL at a total volume of 0.1 mL. To each tube, 1 mL of the Coomassie blue dye is added. Samples are vortexed and allowed to stand at room temperature for 10 minutes. Absorbance is measured at 595 nanometers (nm). For the microassay, ECM dissolved in 100 mM citric acid (pH 2.4) is aliquoted into wells of a 96-well plate at a total volume of 0.1 mL (2.5-30 $\mu$g/mL). To each well, 10 $\mu$L of dye reagent is added. Samples are vortexed, incubated at room temperature for ten minutes before measuring absorbance in a plate reader at 595 nm. Test samples can be assayed in triplicate. Protein concentrations are determined by referencing to the standard curve. Protein concentration is calculated as a percentage of the total dry weight of the ECM. Within a margin of error of about 10%, the protein content in each of the ECM is essentially 95% or more of the total dry weight of the ECM. Water content may be low and within the experimental error (approximately 10%).

Estimation of the total collagen content of the ECM may be characterized using methods known to one skilled in the art and exemplified herein. In a specific embodiment the collagen content of ECM is measured using a quantitative dye-based assay kit, e.g., the SIRCOL™ kit manufactured by Biocolor Ltd, UK. The assay utilizes Sirius Red (or Direct Red 80) as a specific collagen binding dye. Dye bound to collagen displays a concentration dependent increase in absorbance at 540 nm in a UV-Vis spectrophotometer. The assay involves developing a standard calibration curve by measuring absorbances of a series of bovine collagen standards of known concentrations. The concentration of collagen in a test sample, for example, amniotic membrane sample, is determined by referencing to the standard curve. In an exemplary assay, collagen (1 mg/mL) is aliquoted into 1.5 mL microcentrifuge tubes at concentrations from 5-100 $\mu$g/100 $\mu$L. Sample volumes are adjusted to a 100 $\mu$L with water. To each sample 1 mL of SIRCOL™ dye reagent is added at room temperature. Sample tubes are capped and allowed to incubate at room temperature with mechanical shaking for 30 mm. The samples are then centrifuged at 12,000Xg for 15 minutes and liquid drained using a pipetter. The reddish precipitate at the bottom of each tube is dissolved in 1 mL of 0.5M NaOH (sodium hydroxide). UV absorbance for the samples is measured at 540 nm using, e.g., a Beckman DU-7400 UV-VIS spectrophotometer. A standard calibration curve is plotted using the concentration of collagen in each sample versus the absorbance (OD) at 540 nm. To determine experimental error the assay may be repeated (n=10) at a single low concentration of collagen standard (10 $\mu$g/100 $\mu$L). The membrane sample is assayed using the same protocol, the sample being added in a total volume of 100 $\mu$L.

In yet other embodiments, collagen types in ECM may be determined using standard methods known in the art and exemplified herein, e.g., ELISA assay. An exemplary assay for determining the types of collagen, e.g., collagen Types I, III and IV, in the ECM comprises using a sandwich ELISA assay provided, for example, as a kit by Arthrogen-CIA® Collagen-I from Chondrex, Inc., Redmond, Wash., USA. For the Type III and Type IV studies, the primary (Capture Antibody) and secondary antibodies (Detection Antibody) and collagen standards may be obtained from Rockland Immunochemicals, Gilbertsville, Pa. The detection antibody is a biotinylated antibody to human collagen Type I, III or IV, which binds streptavidin peroxidase. The enzymatic reaction with a chromogenic substrate and urea and $H_2O_2$ gives a yellow color, which is detected via UV-Vis spectroscopy at 490 nm. To quantitate the amount of collagen-type, a standard calibration curve is developed with a sample of a series of human collagen standards of known concentrations. The concentration of collagen in a test sample of amniotic membrane is determined by referencing to the standard curve. Assay protocols are developed as per the recommendations of the ELISA kit. To develop a standard calibration curve, 10-12 wells in a 96-well tray are coated with the capture antibody (anti-human type-I collagen antibody, unconjugated) by adding 100 μL of a 100X-diluted Capture Antibody provided with the kit. After overnight incubation, the wells are washed with three times with a wash buffer to remove unbound antibody. Human collagen Type I is then added to the wells in increasing concentration from 0-5 μg/mL in a 100 μL volume. After a two hour incubation at room temperature, the wells are washed with the wash buffer three times to remove unbound collagen. The biotinylated Collagen-I antibody is then added to the antibody-collagen complex in the wells in a 100 μL volume and allowed to bind at room temperature for two hours. Unbound antibody is washed out with three washes with the wash buffer. The detection enzyme streptavidin peroxidase is then bound to the antibody-collagen-antibody complex by addition of a 200X-diluted sample of the enzyme provided with the kit and allowing it to incubate at room temperature for one hour. The 96-well plate is washed repeatedly (six times) to remove any unbound enzyme. The chromogenic substrate+urea/$H_2O_2$ is added to each of the wells in a 100 μL volume. The reaction is allowed to proceed for 30 minutes at room temperature. The reaction is terminated by addition of 50 L of 2.5 N sulfuric acid. Absorbance is measured at 490 nm.

In yet other embodiments, total elastin content of the ECM may be accomplished using methods known in the art. An exemplary assay for measuring the elastin content of ECM may comprise a quantitative dye-based assay kit (FASTIN) manufactured by Biocolor Ltd, UK. The assay utilizes 5,10,15,20-tetraphenyl-21,23-porphrine (TPPS) as a specific elastin binding dye (see, e.g., Winkleman, J. (1962), Cancer Research, 22, 589-596). Dye bound to elastin displays a concentration dependent increase in absorbance at 513 nm in a UV-Vis spectrophotometer. The assay involves developing a standard calibration curve by measuring absorbances of a series of bovine elastin standards of known concentrations. The concentration of elastin in a test sample, for example, a sample of the ECM, is determined by referencing to the standard curve. ECM (1 mg/mL) is aliquoted into 1.5 mL microcentrifuge tubes at concentrations from 5-100 μg/100 μL. Sample volumes are adjusted to 100 μL with water. To each sample 1 mL of Elastin precipitation Reagent (trichloroacetic acid+arginine) is added at 4°C and stored overnight at the same temperature. Following overnight precipitation, the samples are centrifuged at 12,000Xg for 15 minutes and liquid is drained using a pipetter. To each sample, 1 mL of the FASTIN dye reagent (TPPS) is added with a 100 μL of 90% saturated ammonium sulfate. Sample tubes are capped and allowed to incubate at room temperature with mechanical shaking for 1 hr. The ammonium sulfate serves to precipitate the elastin-dye complex. After the 1 hr mixing step, the samples are centrifuged at 12,000Xg for 15 minutes and liquid is drained using a pipetter. The brown precipitate at the bottom of each tube is dissolved into 1 mL of FASTIN dissociation reagent which is a solution of guanidine-HCl in I-propanol. UV absorbance for the samples is measured at 513 nm using, e.g., a Beckman DU-7400 UV-VIS spectrophotometer. A standard calibration curve is plotted using the concentration of elastin in each sample versus the absorbance (OD) at 513 nm. To determine experimental error in the assay, the assay may be repeated (n=10) at a single low concentration of elastin standard (10 μg/100 μL). The membrane sample is assayed using the same protocol, the sample being added in a total volume of 100 μL. Each sample is assayed in triplicate.

In yet other embodiments, total glycosaminoglycan (GAG) content of the ECM may be determined using methods known in the art. The presence of GAGs in ECM may be measured using, e.g., a quantitative dye-based assay kit (BLYSCAN) manufactured by Biocolor Ltd, UK. The assay utilizes 1,9-dimethyl-methylene blue as a specific GAG binding dye. Dye bound to GAG displays a concentration dependent increase in absorbance at 656 nm in a UV-Vis spectrophotometer. The assay involves developing a standard calibration curve by measuring absorbances of a series of bovine GAG standards of known concentrations. The concentration of GAG in a test sample of amniotic membrane is determined by referencing to the standard curve. Bovine GAG (0.1 mg/mL) is aliquoted into 1.5 mL microcentrifuge tubes at concentrations from 0.5-5 μg/100 μL. Sample volumes are adjusted to a 100 μL with water. To each sample 1 mL of the 1,9-dimethyl-methylene dye reagent is added at room temperature. Sample tubes are capped and allowed to incubate at room temperature with mechanical shaking for 30 minutes. The samples are then centrifuged at 12,000Xg for 15 minutes and liquid drained using a pipetter. The reddish precipitate at the bottom of each tube is dissolved in 1 mL of a dye dissociation reagent. UV absorbance for the samples is measured at 656 nm using, e.g., a Beckman DU-7400 UV-VIS spectrophotometer. A standard calibration curve is plotted using the concentration of GAG in each sample versus the absorbance (OD) at 540 nm. To determine experimental error in the assay, the assay may be repeated (n=8) at a single low concentration of GAG standard (1 μg/100 μL). The membrane sample is assayed using the same protocol, the sample being added in a total volume of 100 μL. Each sample is assayed in triplicate.

In yet other embodiments, total laminin content of the ECM may be assayed using methods known in the art. An exemplary assay for determining the total laminin content in ECM may comprise, e.g., a sandwich ELISA assay, e.g., as provided as a kit from Takara Bio Inc., Shiga, Japan (Cat # MKIO7). The kit includes a 96-well plate pre-coated with the primary (Capture Antibody), which is a murine monoclonal antibody to human laminin. The secondary antibodies (Detection antibody) and human laminin standards are provided with the kit. The detection antibody is a conjugated human laminin antibody with peroxidase. The enzymatic reaction with a chromogenic substrate tetramethylbenzidine and $H_2O_2$ gives a blue color, which is detected via UV-Vis spectroscopy at 450 nm. To quantitate the amount of laminin, a standard calibration curve is developed with a sample of a series of human laminin standards of known concentrations (provided with kit). The concentration of laminin in a test sample of ECM is determined by referencing to the standard curve. Assay protocols are developed as per the recommendations of the kit. To develop a standard calibration curve, the human laminin standard is added in increasing concentrations of 5 ng/mL to 160 ng/mL in a final volume of 100 $\mu$L to individual wells of an antibody pre-coated 96-well tray provided with the kit. After an hour incubation at room temperature, the wells are washed with the wash buffer 3 times (PBS containing 0.05% TWEEN™) to remove unbound laminin. The peroxidase-conjugated laminin antibody is then added to the antibody-laminin complex in the wells in a 100 $\mu$L volume and allowed to bind at room temperature for 1 hour. The 96-well plate is washed 4X to remove any unbound enzyme/antibody conjugate. The chromogenic substrate+$H_2O_2$ is added to each of the wells in a 100 $\mu$L volume. The reaction is allowed to proceed for 30 minutes at room temperature. The reaction is terminated by addition of 100 $\mu$L of 2.5N sulfuric acid. Absorbance is measured at 450 nm. Samples of solubilized membrane are tested at a concentration of 1000 ng/mL. Each membrane sample is tested in triplicate. Laminin concentration is presented as a concentration of total membrane weight as shown below.

In yet other embodiments, total fibronectin content of the ECM may be assayed using methods known in the art and exemplified herein. An exemplary assay for determining total fibronectin content of ECM may comprise, e.g., the following: a sandwich ELISA assay provided as a kit from Takara Blo Inc., Shiga, Japan (Cat # MK1 15) may be used. The kit includes a 96-well plate pre-coated with the primary (Capture Antibody), a murine monoclonal antibody to human fibronectin. The secondary antibodies (Detection antibody) and human fibronectin standards are provided with the kit. The detection antibody is a conjugated human fibronectin antibody with horseradish peroxidase. The enzymatic reaction with a chromogenic substrate tetramethylbenzidine and $H_2O_2$ gives a blue color, which is detected via UV-Vis spectroscopy at 450 nm. To quantitate the amount of fibronectin, a standard calibration curve is developed with a sample of a series of human fibronectin standards of known concentrations (provided with kit). The concentration of fibronectin in a test sample is determined by referencing to the standard curve. Assay protocols are developed as per the recommendations of the ELISA kit. To develop a standard calibration curve, the human fibronectin standard is added in increasing concentrations of 12.5 ng/mL to 400 ng/mL in a final volume of 100 $\mu$L to individual wells of an antibody pre-coated 96-well tray provided with the kit. After a 1 hr incubation at room temperature, the wells are washed with the wash buffer 3 times (PBS containing 0.05% TWEEN™) to remove unbound fibronectin. The peroxidase-conjugated fibronectin antibody is then added to the antibody-fibronectin complex in the wells in a 100 $\mu$L volume and allowed to bind at room temperature for 1 hour. The 96-well plate is washed repeatedly (4X) to remove any unbound enzyme/antibody conjugate. The chromogenic substrate+$H_2O_2$ is added to each of the wells in a 100 $\mu$L volume. The reaction is allowed to proceed for 30 minutes at room temperature. The reaction is terminated by addition of 100 $\mu$L of 2.5N sulfuric acid. Absorbance is measured at 450 nm. Samples of solubilized membrane are tested at a concentration of 1000 $\mu$g/mL. Each membrane sample is tested in triplicate.

### 5.2.2. Biocompatibility Studies

The ECM used in the double drying method provided herein is substantially non-immunogenic. Because non-immunogenic human tissue is inherently biocompatible with other human tissue, it is not necessary to perform several of the standard biocompatibility tests (e.g., dermal irritation and sensitization, acute systemic toxicity). Biocompatibility as used herein refers to the property of being biologically compatible by not producing a toxic, injurious, or immunological response or rejection in living tissue. Bodily response to unknown materials is a principal concern when using artificial materials in the body and hence the biocompatibility of a material is an important design consideration in such materials. Biocompatibility assays include but are not limited to cytotoxicity assays, rabbit eye irritation tests, hemolysis assays and pyrogencity assays. Biocompatibility assays useful for assessing the ECM may be cell-based or cell-free.

Cytotoxicity of the ECM may be determined using an ISO MEM Elution test (see Example 5.4.2.2). The purpose of this study is to evaluate the ability of ECM to elicit a cytotoxic response in cultured mouse fibroblast cells. In an exemplary assay, Eagle's Minimal Essential medium (E-MEM) supplemented with 5% Fetal Bovine Serum (FBS) is used to extract test samples. The medium is also supplemented with one or more of the following: L-glutamine, HEPES, gentamicin, penicillin, vancomycin, and amphotericin B (fungizone). Cultures of L-929 cells (mouse fibroblasts) are grown and used as monolayers in disposable tissue culture labware at $37\pm1$°C in a humidified atmosphere of $5\pm1$% carbon dioxide in air. Test samples are extracted intact using a ratio equivalent of 120 cm$^2$ sample and 20 ml-E-MEM plus 5% FBS. Test samples are extracted in E-MEM plus 5% FBS at $37\pm1$°C in $5\pm1$% carbon dioxide for 24-25 hours. After the extraction period, the maintenance culture medium is removed from test culture wells and replaced with 1 ml of the test media/extract and control media/extracts and positive control media spiked with cadmium chloride. Positive, intermediate and negative controls are run in parallel with the test samples. The test media/extract and control media/extract and positive control media spiked with cadmium chloride are plated in triplicate and incubated 72±4 hours at 37±1°C in a humidified atmosphere of 5±1% carbon dioxide in air. Cultures are evaluated for cytotoxic effects by microscopic observation at 24, 48 and 72±4 hour incubation periods. Criteria for evaluating cytotoxicity can include morphological changes in cells, such as granulation, crenation or rounding, and loss of viable cells from the monolayer by lysis or detachment. The validity of the test requires that negative control cultures maintain a healthy normal appearance throughout the duration of the test. Degrees of toxicity are scored, as follows:

0 None: Discrete intracytoplasmic granules; no cell lysis

1 Slight: Not more than 20% of the cells are round, loosely attached, and without intracytoplasmic granules; occasional lysed cells are present

2 Mild: Not more than 50% of the cells are round and devoid of intra-cytoplasmic granules; no extensive cell lysis and empty areas between cells

3 Moderate: Not more than 70% of the cell layers contain rounded cells and/or are lysed

4 Severe: Nearly complete destruction of the cell layers.

According to the USP, test articles scoring "0", "1" or "2" will be considered non-toxic. Test articles scoring "3" or "4" will be considered toxic. The positive control sample must have a score of "3" or "4" and the negative control sample must have a score of "0" for a valid test.

The ocular surface of the rabbit is known to be more sensitive than human skin, therefore rabbit eye irritation studies are used to assess the biocompatibility of ECM. In an exemplary assay, samples are screened for primary ocular irritation. The ECM is cleaned using an aqueous solution of 0.05% deoxycholic acid monohydrate sodium salt (D-Cell). The test can be conducted in accordance with the guidelines of the Federal Hazardous Substances Act (FHSA) Regulations, 16 CFR 1500. In an exemplary assay, control eyes are judged clinically normal for rabbits by gross examination with an auxiliary light source. To detect any pre-existing corneal injury the eyes are treated with fluorescein stain, flushed with 0.9% USP physiological saline solution (PSS), and observed with ultraviolet light in a darkened room. A sample is instilled into the lower conjunctival sac of one eye of each rabbit according to standard techniques. The opposite eye of each rabbit remains untreated and serves as the comparative control. Animals are returned to their cages following treatment. At 24, 48, and 72 hours after dosing the test eye of each rabbit is examined with an auxiliary light source and appropriate magnification compared to the untreated control eye, and graded for ocular irritation. To detect or confirm corneal injury the test eyes are treated with fluorescein stain, flushed with PSS, and examined in darkened conditions with an ultraviolet lamp at 24 hours. Reactions are scored in accordance with the FHSA-modified Draize scoring criteria. One of three animals exhibiting a significant positive reaction is a borderline finding. Two of three animals exhibiting a significant positive reaction is a significant positive response and the test article is considered an irritant.

Hemolytic properties of the ECM may be assayed using methods known in the art and exemplified herein (See Example 5.4.2.4). Hemolysis describes the hemolytic properties of a test sample that will contact blood. In an exemplary assay, the procedure involves exposing the test material to a blood cell suspension and then determining the amount of hemoglobin released. The test is run under static conditions with direct contact of the test sample of ECM with human blood. The amount of hemoglobin released by the red blood cells is measured spectrophotometrically at 540 nm (following conversion to cyanomethemoglobin) concurrently with negative and positive controls. The hemolytic index for the samples and controls is calculated as follows:

$$\text{Hemolytic Index} = \text{Hemoglobin Released(mg/mL)} \times 100$$

Hemoglobin Present(mg/mL)

Where:

$$\text{Hemoglobin Released(mg/ml)} = (\text{Constant} + \text{X Coefficient}) \times \text{Optical Density} \times 16$$

$$\text{Hemoglobin Present(mg/mL)} = \text{Diluted Blood } 10 \pm 1 \text{ mg/mL}$$

Pyrogenicity of the ECM may be assayed using methods known in the art and exemplified herein (See Example 5.4.2.5). In one embodiment, the pyrogenicity of ECM is determined by measuring the presence of bacterial endotoxin in ECM using, for example, the Limulus Amebocyte Lysate (LAL) test. This test is an in vitro assay for detection and quantification of bacterial endotoxin. In an exemplary test, ninety-eight samples of ECM (n=1 per lot), each measuring 1x2 cm, are tested individually for extraction. The extractions are performed by washing each sample in 30 mL of extraction fluid for 40 to 60 minutes at 37 to 40°C with intermittent swirling on an orbital shaker. The pH of each sample extract is between 6 and 8 as verified with pH paper. Pyrogen levels are measured by a Kinetic Turbidimetric Colorimetric Test with a test sensitivity of 0.05 Endotoxin Units (EU) per mL. Total endotoxin level per sample is calculated by multiplying the detected endotoxin value (EU/mL) by 30 mL (extraction volume per device) and again by twenty-four (to simulate a 6x8 cm-sized device).

"Biocompatibility" or "biocompatible" as used herein refers to the property of being biologically compatible by not producing a toxic, injurious, or immunological response or rejection in living tissue.

"Non-pyrogenic" as used herein refers to a material has been tested and found to contain less than or equal to 0.5 EU/mL of a pyrogen, e.g., endotoxin. One EU is approximately 0.1 to 0.2 ng of endotoxin per milliliter and varies according to the reference consulted.

### 5.2.3. Microbiological Studies

Presence of microbiological organisms in ECM, either before or after the double-drying method, including but not limited to Escherichia coli, Klebsiella pneumoniae, Staphylococcus aureus, Enterococcus faecalis, Candida albicans, Proteus vulgaris, Staphylococcus viridans, and Pseudomonas aeruginosa may be determined by art-known methods. Such methods may be used at any step of the preparation of the collagen composition. An exemplary process for microbiology studies during processing comprises the following: Samples of ECM are immersed for five minutes in saline spiked with, e.g., Escherichia coli, Klebsiella pneumoniae, Staphylococcus aureus, Enterococcus faecalis, Candida albicans, Proteus vulgaris, Staphylococcus viridans, and Pseudomonas aeruginosa to deliberately contaminate the sample. Advantageously, decellularization and rinsing act to reduce the number of microorganisms on ECM.

Bioburden of the ECM may also be assayed using art-known methods. As used herein, "bioburden" is a measure of the contaminating organisms found on a given amount of material before it undergoes an industrial sterilization process. In an exemplary method, the minimum E-beam radiation dose that would achieve sterility with a Sterilization Assurance Level of 10-6 is determined. Membranes are extracted by immersion and manual shaking using PEPTONE-TWEEN™ Solution. Plating method is membrane filtration using soybean-casein digest agar. For aerobic conditions, plates are incubated 4 days at 30-35°C, then enumerated. For fungi, plates are incubated four days at 20-25°C, then enumerated. For spore-forming bacteria, the extract portion is heat shocked, filtered and plated as for aerobic bacteria. Plates are incubated 4 days at 30-35°C, then enumerated. For anaerobic bacteria, plates are incubated under anaerobic conditions for 4 days at 30-35°C, then enumerated. Microorganisms utilized are *Clostridium sporogenes, Pseudomonas aeruginosa,* and *Bacillus atrophaeus.*

In particular embodiments, the ECM, e.g., a gram of ECM, has less than 2 colony forming units (cfu) for aerobes and fungi, less than 1, or zero cfu for aerobes and fungi. In yet other embodiments, the ECM has less than 5.1 Colony Forming Units (cfu), less than 2, or less than 1 cfu for anaerobes and spores.

In particular embodiments, ECM is not bacteriostatic or fungistatic as determined using methods exemplified herein and known to one skilled in the art (See Example 5.4.3.2). As used herein bacteriostatic refers to an agent that inhibits bacterial growth or reproduction but does not kill bacteria. As used herein fungistatic refers to an agent that prevents the growth of a fungus by the presence of a non-fungicidal chemical or physical agency.

### 5.2.4. Storage and Handling of ECM

ECM, e.g., double-dried ECM, may be stored at room temperature (e.g., 25°C). In certain embodiments, the ECM can be stored at a temperature of at least 0°C, at least 4°C, at least 10°C, at least 15°C, at least 20°C, at least 25°C, at least 30°C, at least 35°C or at least 40°C, no more than 4°C, no more than 10°C, no more than 15°C, no more than 20°C, no more than 25°C, no more than 30°C, no more than 35°C or no more than 40°C. In some embodiments, the ECM is not refrigerated. In some embodiments, ECM may be refrigerated at a temperature of about 2 to 8°C. In other embodiments, ECM can be stored at any of the above-identified temperatures for an extended period of time. In a particular embodiment, ECM is stored under sterile and non-oxidizing conditions. In certain embodiments, the ECM produced according to the methods described herein can be stored at any of the specified temperatures for 12 months or more with no alteration in biochemical or structural integrity (e.g., no degradation), without any alteration of the biochemical or biophysical properties of the collagen composition. In certain embodiments, the ECM can be stored for several years with no alteration in biochemical or structural integrity (e.g., no degradation), without any alteration of the biochemical or biophysical properties of the collagen composition. The ECM may be stored in any container suitable for long-term storage. Advantageously, ECM can be stored in a sterile double peel-pouch package.

### 5.2.5. Sterilization

ECM, e.g., double-dried ECM, can be sterilized according to techniques known to those of skill in the art for sterilizing such compositions. In a specific embodiment, ECM, e.g., double-dried ECM, is sterilized by radiation. In certain embodiments, the ECM is filtered through a filter that allows passage of endotoxins and retains the collagen composition. Any filter of a size, for example 30 kDa, known to those of skill in the art for filtration of endotoxins can be used. In certain embodiments, the collagen composition is contacted with the filter under conditions that allow endotoxins to pass through the filter while retaining a collagen composition. The conditions can be any conditions for filtration known to those of skill in the art, for instance, centrifugation or pumping. The filter should be of a size that retains collagen while allowing endotoxins to pass the filter. In certain embodiments, the filter is between 5 kDa and 100 kDa. In particular embodiments, the filter is about 5 kDa, about 10 kDa, about 15 kDa, about 20 kDa, about 30 kDa, about 40 kDa, about 50 kDa, about 60 kDa, about 70 kDa, about 80 kDa, about 90 kDa or about 100 kDa. The filter can be of any material known to those of skill in the art to be compatible with a collagen composition such as cellulose, polyethersulfone and others apparent to those of skill. The filtration can be repeated as many times as desired by one of skill in the art. Endotoxin can be detected according to standard techniques to monitor clearance.

In certain embodiments, the double-dried ECM can be filtered to generate ECM free of, or reduced in, viral particles. Advantageously, the filter retains a collagen composition while allowing viral particles to pass through. Any filter known to those of skill in the art to be useful for clearing viruses can be used. For instance, a 1000 kDa filter can be used for clearance, or reduction, of parvovirus, hepatitis A virus and HIV. A 750 kDa filter can be used for clearance, or reduction, of parvovirus and hepatitis A virus. A 500 kDa filter can be used for clearance, or reduction, of parvovirus.

ECM, e.g., double-dried ECM, can be prepared which is free of viral particles, or reduced in number of viral particles, by a method comprising the step of contacting the ECM with a filter of a size that allows one or more viral particles to pass through the filter while retaining the ECM. In certain embodiments, the collagen composition is contacted with the filter under conditions that allow one or more viral particles to pass through the filter while retaining a collagen composition. The conditions can be any conditions for filtration known to those of skill in the art, for instance, centrifugation or pumping. The filter should be of a size that retains collagen while allowing one or more viral particles to pass the filter. In certain embodiments, the filter is between 500 kDa and 1000 kDa. In particular embodiments, the filter is about 500 kDa, about 750 kDa or about 1000 kDa. The filter can be of any material known to those of skill in the art to be compatible with a collagen composition such as cellulose, polyethersulfone and others apparent to those of skill. The filtration can be repeated as many times as desired by one of skill in the art. Viral particles can be detected according to standard techniques to monitor filtration.

Sterilization of ECM can also be carried out by electron beam irradiation using methods known to one skilled in the art, e.g., Gorham, D. Byrom (ed.), 1991, Biomaterials, Stockton Press, New York, 55-122. Any dose of radiation sufficient to kill at least 99.9% of bacteria or other potentially contaminating organisms is within the scope of the method. In a particular embodiment, a dose of at least 18-25 kGy is used to achieve the terminal sterilization of ECM.

### 5.3. Formulations of double-dried ECM

ECM, e.g., double-dried ECM, can be formulated in water or phosphate buffered saline, e.g., as a solution or suspension, e.g., a mouthwash. In particular embodiments, the collagen is formulated in phosphate buffered saline. The ECM can be present in the solution or suspension at any concentration useful to those of skill in the art. In certain embodiments, the formulations comprise 0.1-100 mg/ml, 1-100 mg/ml, 1-75 mg/ml, 1-50 mg/ml, 1-40 mg/ml, 10-40 mg/ml or 20-40 mg/ml ECM. In certain embodiments, the ECM solution or suspension comprises about 5 mg/ml, 10 mg/ml, 15 mg/ml, 20 mg/ml, 25 mg/ml, 30 mg/ml, 35 mg/ml, 40 mg/ml, 45 mg/ml or 50 mg/ml collagen. In a particular embodiment, provided herein are formulations comprising about 35 mg/ml ECM.

ECM, as extracted from the placenta, is typically a white paste. This paste can be used in the methods of treatment provided elsewhere herein as a paste, or can be shaped according to any methods known in the art for shaping such materials, e.g., during the double-drying method after rehydration but prior to the second drying step. For example, the composition can be forced into a mold, or formed around a mold, to produce specific shapes. The shape can be any useful shape including sheets, tubes, plugs, spheres and the like. In certain embodiments, the ECM is shaped to fit a site of a wound or injury. The shaped ECM can be used for any purpose apparent to those of skill in the art. Exemplary methods of using shaped ECM are provided below.

In certain embodiments, double-dried ECM prepared according to the methods described herein may be combined with pharmaceutically or cosmetically acceptable carriers and administered as compositions in vitro or in vivo. Forms of administration include, but are not limited to, injections, solutions, creams, gels, implants, pumps, ointments, emulsions, suspensions, microspheres, particles, microparticles, nanoparticles, liposomes, pastes, patches, tablets, transdermal delivery devices, sprays, aerosols, or other means familiar to one of ordinary skill in the art. Such pharmaceutically or cosmetically acceptable carriers are commonly known to one of ordinary skill in the art. Pharmaceutical formulations can be prepared by procedures known in the art using well known and readily available ingredients. For example, the compounds can be formulated with common excipients, diluents, or carriers, and formed into tablets, capsules, suspensions, powders, and the like. Examples of excipients, diluents, and carriers that are suitable for such formulations include the following: fillers and extenders (e.g., starch, sugars, mannitol, and silicic derivatives); binding agents (e.g., carboxymethyl cellulose and other cellulose derivatives, alginates, gelatin, and polyvinyl-pyrrolidone); moisturizing agents (e.g., glycerol); disintegrating agents (e.g., calcium carbonate and sodium bicarbonate); agents for retarding dissolution (e.g., paraffin); resorption accelerators (e.g., quaternary ammonium compounds); surface active agents (e.g., cetyl alcohol, glycerol monostearate); adsorptive carriers (e.g., kaolin and bentonite); emulsifiers; preservatives; sweeteners; stabilizers; coloring agents; perfuming agents; flavoring agents; lubricants (e.g., talc, calcium and magnesium stearate); solid polyethyl glycols; and mixtures thereof.

The terms "pharmaceutically or cosmetically acceptable carrier" or "pharmaceutically or cosmetically acceptable vehicle" are used herein to mean, without limitations, any liquid, solid or semi-solid, including, but not limited to, water or saline, a gel, cream, salve, solvent, diluent, fluid ointment base, ointment, paste, implant, liposome, micelle, giant micelle, and the like, which is suitable for use in contact with living animal or human tissue without causing adverse physiological or cosmetic responses, and which does not interact with the other components of the composition, *e.g.*, the ECM, e.g., double-dried ECM, in a deleterious manner. Other pharmaceutically or cosmetically acceptable carriers or vehicles known to one of skill in the art may be employed to make compositions for delivering the molecules.

The double-dried ECM formulations can be so constituted that they release any active ingredient, e.g., an active ingredient in addition to ECM, only or preferably in a particular location, possibly over a period of time. Such combinations provide yet a further mechanism for controlling release kinetics. The coatings, envelopes, and protective matrices may be made, for example, from polymeric substances or waxes.

Methods of in vivo administration of ECM, e.g., double-dried ECM,, or of formulations comprising ECM, e.g., double-dried ECM, and optionally other materials such as carriers that are particularly suitable for various forms include, but are not limited to, oral administration (e.g. buccal or sublingual administration), topical application, aerosol application, transdermal administration, intradermal administration, subdermal administration, intramuscular administration, or surgical administration at the location of a lesion. Techniques useful in the various forms of administrations above include but are not limited to, topical application, surgical administration, injections, sprays, transdermal delivery devices, osmotic pumps, electrodepositing directly on a desired site, or other means familiar to one of ordinary skill in the art.

The double-dried ECM can be applied in the form of creams, gels, solutions, suspensions, liposomes, particles, or other means known to one of skill in the art of formulation and delivery of therapeutic and cosmetic compounds. Some examples of appropriate formulations for subcutaneous administration include but are not limited to implants, depot, needles, capsules, and osmotic pumps. Some examples of appropriate formulations for oral administration include but are not limited to: pastes, patches, sheets, liquids, syrups, suspensions, aerosols and mists. Some examples of appropriate formulations for transdermal administration include but are not limited to creams, pastes, patches, sprays, and gels. Some examples of appropriate delivery mechanisms for subcutaneous administration include but are not limited to implants, depots, needles, capsules, and osmotic pumps.

Embodiments in which the double-dried ECM is combined with, for example, one or more pharmaceutically or cosmetically acceptable carriers or excipients may conveniently be presented in unit dosage form and may be prepared by conventional pharmaceutical techniques. Such techniques include the step of bringing into association the compositions containing the active ingredient and the pharmaceutical carrier(s) or excipient(s). In general, the formulations are prepared by uniformly and intimately bringing into association the active ingredient with, e.g., a liquid carrier. Particular unit dosage formulations are those containing a dose or unit, or an appropriate fraction thereof, of the administered ingredient. It should be understood that in addition to the ingredients particularly mentioned above, formulations comprising the double dried ECM compositions provided herein may include other agents commonly used by one of ordinary skill in the art. The volume of administration will vary depending on the route of administration.

The double-dried ECM may be administered to persons or animals in any dose range that will produce desired physiological or pharmacological results. Dosage will depend upon the substance or substances administered, the therapeutic endpoint desired, the desired effective concentration at the site of action or in a body fluid, and the type of administration. Information regarding appropriate doses of substances are known to persons of ordinary skill in the art and may be found in references such as L. S. Goodman and A. Gilman, eds, The Pharmacological Basis of Therapeutics, Macmillan Publishing, New York, and Katzung, Basic & Clinical Pharmacology, Appleton & Lang, Norwalk, Conn., (6th Ed. 1995). A clinician skilled in the art of the desired therapy may chose specific dosages and dose ranges, and frequency of administration, as required by the circumstances and the substances to be administered.

The double-dried ECM may comprise one or more compounds or substances, e.g., active ingredients or active agents, that are not double-dried ECM components, e.g., collagen, elastin, laminin and/or glycosaminoglycan. For example, the double-dried ECM may be impregnated, either during production or during preparation for surgery, with a biomolecule. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), hyaluronic acid as a wound healing agent, wound sealants (such as fibrin with or without thrombin), cellular attractant and scaffolding reagents (such as fibronectin) and the like. In a specific example, the double-dried ECM may be impregnated with at least one growth factor, for example, fibroblast growth factor, epithelial growth factor, etc. The double-dried ECM may also be impregnated with small organic molecules such as specific inhibitors of particular biochemical processes e.g., membrane receptor inhibitors, kinase inhibitors, growth inhibitors, anticancer drugs, antibiotics, etc.

In yet other embodiments, double-dried ECM may be combined with a hydrogel. Any hydrogel known to one skilled in the art may be used, e.g., any of the hydrogel compositions disclosed in Graham, 1998, Med. Device Technol. 9(1): 18-22; Peppas et al., 2000, Eur. J. Pharm. Biopharm. 50(1): 27-46; Nguyen et al., 2002, Biomaterials, 23(22): 4307-14; Henincl et al., 2002, Adv. Drug Deliv. Rev 54(1): 13-36; Skelhorne et al., 2002, Med. Device. Technol. 13(9): 19-23; or Schmedlen et al., 2002, Biomaterials 23: 4325-32. In a specific embodiment, the hydrogel is applied onto the ECM, i.e., discharged on the surface of the collagen composition. The hydrogel for example, may be sprayed onto the double-dried ECM, saturated on the surface of the double-dried ECM, soaked with the double-dried ECM, bathed with the double-dried ECM or coated onto the surface of the double-dried ECM. The hydrogels useful in the methods and compositions provided herein can be made from any water-interactive, or water soluble polymer known in the art, including but not limited to, polyvinylalcohol (PVA), polyhydroxyehthyl methacrylate, polyethylene glycol, polyvinyl pyrrolidone, hyaluronic acid, dextran or derivatives and analogs thereof.

In some embodiments, the double-dried ECM is further impregnated with one or more biomolecules prior to being combined with a hydrogel. In other embodiments, the hydrogel is further impregnated with one or more biomolecules prior to being combined with double-dried ECM. Such biomolecules include but are not limited to, antibiotics (such as Clindamycin, Minocycline, Doxycycline, Gentamycin), hormones, growth factors, anti-tumor agents, anti-fungal agents, anti-viral agents, pain medications, anti-histamines, anti-inflammatory agents, anti-infectives including but not limited to silver (such as silver salts, including but not limited to silver nitrate and silver sulfadiazine), elemental silver, antibiotics, bactericidal enzymes (such as lysozome), wound healing agents (such as cytokines including but not limited to PDGF, TGF; thymosin), hyaluronic acid as a wound healing agent, wound sealants (such as fibrin with or without thrombin), cellular attractant and scaffolding reagents (such as fibronectin) and the like. In a specific example, the double-dried ECM or the hydrogel may be impregnated with at least one growth factor, for example, fibroblast growth factor, epithelial growth factor, etc. Advantageously, the biomolecule can be a therapeutic agent. In some embodiments, the hydrogel is combined with a laminate comprising double-dried ECM.

In some embodiments, the hydrogel/ double-dried ECM is applied topically to a subject, i.e., on the surface of the oral mucosa, e.g., at the site of a lesion. In some embodiments, the hydrogel formulated to be non-biodegradable. In yet other embodiments, the hydrogel is formulated to be biodegradable. In a specific embodiment, the hydrogel is formulated to degrade within days, e.g., less than 7 days, less than 14 days, less than 21 days, or less than 28 days after administration to an individual. In another specific embodiment, the hydrogel composition is formulated to degrade within months after administration to an individual.

In some embodiments, the double-dried ECM is populated with cells. Cells that can be used to populate double-dried ECM include, but are not limited to, stem cells (e.g., human stem cells), human differentiated adult cells, pluripotent stem cells, multipotent stem cells, tissue specific stem cells, embryonic like stem cells, committed progenitor cells, fibroblastoid cells, and the like. In other embodiments, the double-dried ECM may be populated with specific classes of progenitor cells including but not limited to chondrocytes, hepatocytes, hematopoietic cells, pancreatic parenchymal cells, neuroblasts, and muscle progenitor cells.


### 5.4. Stem Cells

In certain embodiments, the double-dried ECM comprises a plurality of stem cells. The stem cells can be any stem cells suitable for a given purpose, and can be pluripotent stem cells, or can be progenitor cells. Preferably, the composition comprises placental stem cells such as those described in U.S. Patent Nos. 7,045,148; 7,468,276; 8,057,788 and 8,202, 703. However, the double-dried ECM can comprise stem or progenitor cells, preferably mammalian stem or progenitor cells, from any tissue source, e.g., embryonic stem cells, mesenchymal stem cells, bone marrow-derived stem cells, hematopoietic progenitor cells (e.g., hematopoietic stem cells from peripheral blood, fetal blood, placental blood, umbilical cord blood, placental perfusate, etc.), somatic stem cells, neural stem cells, hepatic stem cells, pancreatic stem cells, endothelial stem cells, cardiac stem cells, muscle stem cells, adipose stem cells, and the like. The double-dried ECM can comprise any combination of types of stem cells. In preferred embodiments, the stem cells are human stem cells, e.g., human placental stem cells.

In certain embodiments, the double-dried ECM is contacted with a plurality of stem or progenitor cells for a time sufficient for a plurality of said stem or progenitor cells to attach to the ECM. In preferred embodiments, the double-dried ECM is shaped into a useful configuration, e.g., sheet, plug, tube, or other configuration, prior to contacting with the stem or progenitor cells. Contacting the stem or progenitor cells with the double-dried ECM can be effected by any method known in the art, and may comprise, e.g., dispensing medium comprising the stem or progenitor cells onto the surface of the double-dried ECM; immersing a part or a whole of the double-dried ECM in a suspension of the stem or progenitor cells; culturing a plurality of the stem or progenitor cells on the surface of the double-dried ECM for a time sufficient for the plurality to proliferate for at least one cell division; and the like. The stem cells, preferably placental stem cells, can be present on the double-dried ECM, e.g., a shaped form of the double-dried ECM, on the entirety or a portion of the ECM's surface, e.g., can be present randomly on the surface, present confluently, etc.

The number of stem or progenitor cells contacted with the double-dried ECM in any embodiment may vary, but in various embodiments can be at least $1 \times 10^6$, $3 \times 10^6$, $1 \times 10^7$, $3 \times 10^7$, $1 \times 10^8$, $3 \times 10^8$, $1 \times 10^9$, $3 \times 10^9$, $1 \times 10^{10}$, $3 \times 10^{10}$, $1 \times 10^{11}$, $3 \times 10^{11}$, or $1 \times 10^{12}$; or may be no more than $1 \times 10^6$, $3 \times 10^6$, $1 \times 10^7$, $3 \times 10^7$, $1 \times 10^8$, $3 \times 10^8$, $1 \times 10^9$, $3 \times 10^9$, $1 \times 10^{10}$, $3 \times 10^{10}$, $1 \times 10^{11}$, $3 \times 10^{11}$, or $1 \times 10^{12}$ stem or progenitor cells.

In certain other embodiments, the double-dried ECM comprises one or more types of extracellular matrix protein deposited by a stem cell or population of stem cells. In one embodiment, for example, double-dried ECM is made to comprise extracellular matrix proteins by contacting double-dried ECM with a plurality of stem cells; culturing the stem cells on the composition for a time sufficient for the stem cells to deposit a detectable amount of at least one type of extracellular matrix protein; and decellularizing the composition to produce an double-dried ECM comprising at least one type of extracellular matrix protein. In one embodiment, therefore, the ECM comprises a decellularized extracellular matrix, wherein the decellularized extracellular matrix is deposited or produced by stem cells. In various embodiments, the extracellular matrix protein comprises one or more of collagen (e.g., one or more of Type I, II, III, and/or IV collagen), elastin and/or fibronectin. In another embodiment, the extracellular matrix protein is produced by a plurality of stem cells that are proliferating and not differentiating. In another embodiment, the extracellular matrix is produced by a plurality of stem cells that are differentiating, or by a plurality of cells that have differentiated from a plurality of stem cells.

### 5.4.1. Placental Stem Cells

In one embodiment, a composition provided herein comprises double-dried ECM and a plurality of CD34- placental stem cells. CD34- placental stem cells are stem cells, obtainable from placental tissue, that adhere to a tissue culture substrate and have the capacity to differentiate into non-placental cell types. Placental stem cells can be either fetal or maternal in origin (that is, can have the genotype of either the mother or fetus). Populations of placental stem cells, or populations of cells comprising placental stem cells, can comprise placental stem cells that are solely fetal or maternal in origin, or can comprise a mixed population of placental stem cells of both fetal and maternal origin. The placental stem cells, and populations of cells comprising the placental stem cells, can be identified and selected by the morphological, marker, and culture characteristic discussed below.

The placental stem cells, when cultured in primary cultures or in cell culture, adhere to the tissue culture substrate, e.g., tissue culture container surface (e.g., tissue culture plastic). Placental stem cells in culture assume a generally fibroblastoid, stellate appearance, with a number of cytoplasmic processes extending from the central cell body. The placental stem cells are, however, morphologically differentiable from fibroblasts cultured under the same conditions, as the placental stem cells exhibit a greater number of such processes than do fibroblasts. Morphologically, placental stem cells are also distinguishable from hematopoietic stem cells, which generally assume a more rounded, or cobblestone, morphology in culture.

The placental stem cells generally express the markers CD10, CD73, CD105, CD200, HLA-G, and/or OCT-4, and do not express CD34, CD38, or CD45. Placental stem cells can also express HLA-ABC (MHC-1) and HLA-DR. Thus, in one embodiment, the stem cells that can be combined with the ECM are CD200+ or HLA-G+. In another embodiment, the placental stem cells are CD73+, CD105+, and CD200+. In another embodiment, the placental stem cells are CD200+ and OCT-4+. In another embodiment, the placental stem cells are CD73+, CD105+ and HLA-G+. In another embodiment, the placental stem cells are CD73+ and CD105+, and, when in a population of placental cells, facilitate formation of one or more embryoid-like bodies under conditions that allow formation of embryoid-like bodies. In another embodiment, the placental stem cells are OCT-4+ and, when in a population of placental cells, facilitate formation of one or more embryoid-like bodies in a population of isolated placental stem cells comprising said stem cell when cultured under conditions that allow formation of embryoid-like bodies.

In certain embodiments, the isolated placental stem cells are isolated placental stem cells. In certain other embodiments, the isolated placental stem cells are isolated placental multipotent cells. In one embodiment, the isolated placental stem cells, e.g., PDACs, are CD34-, CD10+ and CD105+ as detectable by flow cytometry. In another specific embodiment, the isolated CD34-, CD10+, CD105+ placental cells have the potential to differentiate into cells of a neural phenotype, cells of an osteogenic phenotype, and/or cells of a chondrogenic phenotype. In another specific embodiment, the isolated CD34-, CD10+, CD105+ placental cells are additionally CD200+. In another specific embodiment, the isolated CD34-, CD10+. CD105+ placental cells are additionally CD45- or CD90+. In another specific embodiment, the isolated CD34-, CD10+, CD105+ placental cells are additionally CD45- and CD90+, as detectable by flow cytometry. In another specific embodiment, the isolated CD34-, CD10+, CD105+, CD200+ placental cells are additionally CD90+ or CD45-, as detectable by flow cytometry. In another specific embodiment, the isolated CD34-, CD10+, CD105+, CD200+ placental cells are additionally CD90+ and CD45-, as detectable by flow cytometry, i.e., the cells are CD34-, CD10+, CD45-, CD90+, CD105+ and CD200+. In another specific embodiment, said CD34-, CD10+, CD45-, CD90+, CD105+, CD200+ cells are additionally CD80- and CD86-.

In certain embodiments, said placental stem cells are CD34-, CD10+, CD105+ and CD200+, and one or more of CD38-, CD45-, CD80-, CD86-, CD133-, HLA-DR,DP,DQ-, SSEA3-, SSEA4-, CD29+, CD44+, CD73+, CD90+, CD105+, HLA-A,B,C+, PDL1+, ABC-p+, and/or OCT-4+, as detectable by flow cytometry. In other embodiments, any of the CD34-, CD10+, CD105+ cells described above are additionally one or more of CD29+, CD38-, CD44+, CD54+, SH3+ or SH4+. In another specific embodiment, the cells are additionally CD44+. In another specific embodiment of any of the isolated CD34-, CD10+, CD105+ placental cells above, the cells are additionally one or more of CD117-, CD133-, KDR- (VEGFR2-), HLA-A,B,C+, HLA-DP,DQ,DR-, or Programmed Death-1 Ligand (PDL1) +, or any combination thereof.

In another embodiment, the CD34-, CD10+, CD105+ cells are additionally one or more of CD13+, CD29+, CD33+, CD38-, CD44+, CD45-, CD54+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin$_{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1)+, or any combination thereof. In another embodiment, the CD34-, CD10+, CD105+ cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherin$^{low}$, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In another specific embodiment, any of the placental stem cells described herein are additionally ABC-p+, as detectable by flow cytometry, or OCT-4+ (POU5F1), as determined by reverse-transcriptase polymerase chain reaction (RT-PCR), wherein ABC-p is a placenta-specific ABC transporter protein (also known as breast cancer resistance protein (BCRP) and as mitoxantrone resistance protein (MXR)), and OCT-4 is the Octamer-4 protein (POU5F1). In another specific embodiment, any of the placental stem cells described herein are additionally SSEA3- or SSEA4-, as determined by flow cytometry, wherein SSEA3 is Stage Specific Embryonic Antigen 3, and SSEA4 is Stage Specific Embryonic Antigen 4. In another specific embodiment, any of the placental stem cells described herein are additionally SSEA3- and SSEA4-.

In another specific embodiment, any of the placental cells described herein are additionally one or more of MHC-I+ (e.g., HLA-A,B,C+), MHC-II- (e.g., HLA-DP,DQ,DR-) or HLA-G-. In another specific embodiment, any of the placental stem cells described herein are additionally one or more of MHC-I+ (e.g., HLA-A,B,C+), MHC-IV (e.g., HLA-DP,DQ,DR-) and HLA-G-.

Also provided herein are populations of the isolated placental stem cells, or populations of cells, e.g., populations of placental cells, comprising, e.g., that are enriched for, the isolated placental stem cells, that are useful in the methods and compositions disclosed herein. Preferred populations of cells comprising the isolated placental stem cells, wherein the populations of cells comprise, e.g., at least 10%, 15%, 20%, 25%, 30%, 35%, 40%, 45%, 50%, 55%, 60%, 65%, 70%, 75%, 80%, 85%, 90%, 95% or 98% isolated placental stem cells that are CD10+, CD105+ and CD34-; that is, at least 10%, 15%, 20%, 25%, 30%, 35%, 40%, 45%, 50%, 55%, 60%, 65%, 70%, 75%, 80%, 85%, 90%, 95% or 98% of cells in said population are isolated placental stem cells that are CD10+, CD105+ and CD34-. In a specific embodiment, the isolated CD34-, CD10+, CD105+ placental stem cells are additionally CD200+. In another specific embodiment, the isolated CD34-, CD10+, CD105+, CD200+ placental stem cells are additionally CD90+ or CD45-, as detectable by flow cytometry. In another specific embodiment, the isolated CD34-, CD10+, CD105+, CD200+ placental stem cells are additionally CD90+ and CD45-, as detectable by flow cytometry. In another specific embodiment, any of the isolated CD34-, CD10+, CD105+ placental stem cells described above are additionally one or more of CD29+, CD38-, CD44+, CD54+, SH3+ or SH4+. In another specific embodiment, the isolated CD34-, CD10+, CD105+ placental stem cells, or isolated CD34-, CD10+, CD105+, CD200+ placental stem cells, are additionally CD44+. In a specific embodiment of any of the populations of cells comprising isolated CD34-, CD10+, CD105+ placental stem cells above, the isolated placental stem cells are additionally one or more of CD13+, CD29+, CD33+, CD38-, CD44+, CD45-, CD54-, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherinlow, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, or Programmed Death-1 Ligand (PDL1) +, or any combination thereof. In another specific embodiment, the CD34-, CD10+, CD105+ placental stem cells are additionally CD13+, CD29+, CD33+, CD38-, CD44+, CD45-, CD54/ICAM+, CD62E-, CD62L-, CD62P-, SH3+ (CD73+), SH4+ (CD73+), CD80-, CD86-, CD90+, SH2+ (CD105+), CD106/VCAM+, CD117-, CD144/VE-cadherinlow, CD184/CXCR4-, CD200+, CD133-, OCT-4+, SSEA3-, SSEA4-, ABC-p+, KDR- (VEGFR2-), HLA-A,B,C+, HLA-DP,DQ,DR-, HLA-G-, and Programmed Death-1 Ligand (PDL1)+.

In certain embodiments, the isolated placental stem cells useful in the methods and compositions described herein are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, wherein said isolated placental stem cells are obtained by physical and/or enzymatic disruption of placental tissue. In a specific embodiment, the isolated placental stem cells are OCT-4+ and ABC-p+. In another specific embodiment, the isolated placental stem cells are OCT-4+ and CD34-, wherein said isolated placental stem cells have at least one of the following characteristics: CD10+, CD29+, CD44+, CD45-, CD54+, CD90+, SH3+, SH4+, SSEA3-, and SSEA4-. In another specific embodiment, the isolated placental stem cells are OCT-4+, CD34-, CD10+, CD29+, CD44+, CD45-, CD54+, CD90+, SH3+, SH4+, SSEA3-, and SSEA4-. In another embodiment, the isolated placental stem cells are OCT-4+, CD34-, SSEA3-, and SSEA4-. In another specific embodiment, the isolated placental stem cells are OCT-4+ and CD34-, and either SH2+ or SH3+. In another specific embodiment, the isolated placental stem cells are OCT-4+, CD34-, SH2+, and SH3+. In another specific embodiment, the isolated placental stem cells are OCT-4+, CD34-, SSEA3-, and SSEA4-, and are either SH2+ or SH3+. In another specific embodiment, the isolated placental stem cells are OCT-4+ and CD34-, and either SH2+ or SH3+, and are at least one of CD10+, CD29+, CD44+, CD45-, CD54+, CD90+, SSEA3-, or SSEA4-. In another specific embodiment, the isolated placental stem cells are OCT-4+, CD34-, CD10+, CD29+, CD44+, CD45-, CD54+, CD90+, SSEA3-, and SSEA4-, and either SH2+ or SH3+.

In another embodiment, the isolated placental stem cells useful in the methods and compositions disclosed herein are SH2+, SH3+, SH4+ and OCT-4+. In another specific embodiment, the isolated placental stem cells are CD10+, CD29+, CD44+, CD54+, CD90+, CD34-, CD45-, SSEA3-, or SSEA4-. In another embodiment, the isolated placental stem cells are SH2+, SH3+, SH4+. SSEA3- and SSEA4-. In another specific embodiment, the isolated placental stem cells are SH2+, SH3+, SH4+, SSEA3- and SSEA4-, CD10+, CD29+, CD44+, CD54+, CD90+, OCT-4+, CD34- or CD45-.

In another embodiment, the isolated placental stem cells useful in the methods and compositions disclosed herein are CD10+, CD29+, CD34-, CD44+, CD45-, CD54+, CD90+, SH2+, SH3+, and SH4+; wherein said isolated placental stem cells are additionally one or more of OCT-4+, SSEA3- or SSEA4-.

In certain embodiments, isolated placental stem cells useful in the methods and compositions disclosed herein are CD200+ or HLA-G-. In a specific embodiment, the isolated placental stem cells are CD200+ and HLA-G-. In another specific embodiment, the isolated placental stem cells are additionally CD73+ and CD105+. In another specific embodiment, the isolated placental stem cells are additionally CD34-, CD38- or CD45-. In another specific embodiment, the isolated placental stem cells are additionally CD34-, CD38- and CD45-. In another specific embodiment, said placental stem cells are CD34-, CD38-, CD45-, CD73+ and CD105+. In another specific embodiment, said isolated CD200+ or HLA-G- placental cells facilitate the formation of embryoid-like bodies in a population of placental cells comprising the isolated placental stem cells, under conditions that allow the formation of embryoid-like bodies. In another specific embodiment, the isolated placental stem cells are isolated away from placental cells that are not stem or multipotent cells. In another specific embodiment, said isolated placental stem cells are isolated away from placental cells that do not display these combination of markers.

In another embodiment, a cell population useful in the methods of treatment and ECM compositions described herein is a population of cells comprising, e.g., that is enriched for, CD200+, HLA-G- placental stem cells. In a specific embodiment, said population is a population of placental cells. In various embodiments, at least about 10%, at least about 20%, at least about 30%, at least about 40%, at least about 50%, or at least about 60% of cells in said cell population are isolated CD200+, HLA-G- placental stem cells. Preferably, at least about 70% of cells in said cell population are isolated CD200+, HLA-G- placental stem cells. More preferably, at least about 90%, 95%, or 99% of said cells are isolated CD200+, HLA-G- placental stem cells. In a specific embodiment of the cell populations, said isolated CD200+, HLA-G- placental stem cells are also CD73+ and CD105+. In another specific embodiment, said isolated CD200+, HLA-G- placental stem cells are also CD34-, CD38- or CD45-. In another specific embodiment, said isolated CD200+, HLA-G- placental stem cells are also CD34-, CD38-, CD45-, CD73+ and CD105+. In another embodiment, said cell population produces one or more embryoid-like bodies when cultured under conditions that allow the formation of embryoid-like bodies. In another specific embodiment, said cell population is isolated away from placental cells that are not stem cells. In another specific embodiment, said isolated CD200+, HLA-G- placental stem cells are isolated away from placental cells that do not display these markers.

In another embodiment, the isolated placental stem cells useful in the methods and compositions described herein are CD73+, CD105+, and CD200+. In another specific embodiment, the isolated placental stem cells are HLA-G-. In another specific embodiment, the isolated placental stem cells are CD34-, CD38- or CD45-. In another specific embodiment, the isolated placental stem cells are CD34-, CD38- and CD45-. In another specific embodiment, the isolated placental stem cells are CD34-, CD38-, CD45-, and HLA-G-. In another specific embodiment, the isolated CD73+, CD105+, and CD200+ placental stem cells facilitate the formation of one or more embryoid-like bodies in a population of placental cells comprising the isolated placental stem cells, when the population is cultured under conditions that allow the formation of embryoid-like bodies. In another specific embodiment, the isolated placental stem cells are isolated away from placental cells that are not the isolated placental stem cells. In another specific embodiment, the isolated placental stem cells are isolated away from placental cells that do not display these markers.

In another embodiment, a cell population useful in the methods and compositions described herein is a population of cells comprising, e.g., that is enriched for, isolated CD73+, CD105+, CD200+ placental stem cells. In various embodiments, at least about 10%, at least about 20%, at least about 30%, at least about 40%, at least about 50%, or at least about 60% of cells in said cell population are isolated CD73+, CD105+, CD200+ placental stem cells. In another embodiment, at least about 70% of said cells in said population of cells are isolated CD73+, CD105+, CD200+ placental stem cells. In another embodiment, at least about 90%, 95% or 99% of cells in said population of cells are isolated CD73+, CD105+, CD200+ placental stem cells. In a specific embodiment of said populations, the isolated placental stem cells are HLA-G-. In another specific embodiment, the isolated placental stem cells are additionally CD34-, CD38- or CD45-. In another specific embodiment, the isolated placental stem cells are additionally CD34-, CD38- and CD45-. In another specific embodiment, the isolated placental stem cells are additionally CD34-, CD38-, CD45-, and HLA-G. In another specific embodiment, said population of cells comprising said placental stem cells produces one or more embryoid-like bodies when cultured under conditions that allow the formation of embryoid-like bodies. In another specific embodiment, said population of placental stem cells is isolated away from placental cells that are not stem cells. In another specific embodiment, said population of placental cells is isolated away from placental cells that do not display these characteristics.

In certain other embodiments, the isolated placental stem cells are one or more of CD10+, CD29+, CD34-, CD38-, CD44+, CD45-, CD54+, CD90+, SH2+, SH4+, SSEA3-, SSEA4-, OCT-4+, HLA-G- or ABC-p+. In a specific embodiment, the isolated placental stem cells are CD10+, CD29+. CD34-, CD38-, CD44+, CD45-, CD54+, CD90+, SH2+, SH3+, SH4+, SSEA3-, SSEA4-, and OCT-4+. In another specific embodiment, the isolated placental stem cells are CD10+, CD29+, CD34-, CD38-, CD45-, CD54+, SH2+, SH3+, and SH4+. In another specific embodiment, the isolated placental stem cells are CD10+, CD29+, CD34-, CD38-, CD45-, CD54+, SH2+, SH3+, SH4+ and OCT-4+. In another specific embodiment, the isolated placental stem cells are CD10+, CD29+, CD34-, CD38-, CD44+, CD45-, CD54+, CD90+, HLA-G-, SH2+, SH3+, and SH4+. In another specific embodiment, the isolated placental stem cells are OCT-4+ and ABC-p+. In another specific embodiment, the isolated placental stem cells are SH2+, SH3+, SH4+ and OCT-4+. In another embodiment, the isolated placental stem cells are OCT-4+, CD34-, SSEA3-, and SSEA4-. In a specific embodiment, said isolated OCT-4+, CD34-, SSEA3-, and SSEA4- placental cells are additionally CD10+, CD29+, CD34-, CD44+, CD45-, CD54+, CD90+, SH2+, SH3+, and SH4+. In another embodiment, the isolated placental stem cells are OCT-4+ and CD34-, and either SH3+ or SH4+. In another embodiment, the isolated placental stem cells are CD34- and either CD10+, CD29+, CD44+, CD54+, CD90+, or OCT-4+.

In another embodiment, the isolated placental stem cells useful in the methods and compositions described herein are CD200+ and OCT-4+. In a specific embodiment, the isolated placental stem cells are CD73+ and CD105+. In another specific embodiment, said isolated placental stem cells are HLA-G-. In another specific embodiment, said isolated CD200+, OCT-4+ placental stem cells are CD34-, CD38- or CD45-. In another specific embodiment, said isolated CD200+, OCT-4+ placental stem cells are CD34-, CD38- and CD45-. In another specific embodiment, said isolated CD200+, OCT-4+ placental stem cells are CD34-, CD38-, CD45-, CD73+, CD105+ and HLA-G-. In another specific embodiment, the isolated CD200+, OCT-4+ placental stem cells facilitate the production of one or more embryoid-like bodies by a population of placental cells that comprises the isolated cells, when the population is cultured under conditions that allow the formation of embryoid-like bodies. In another specific embodiment, said isolated CD200+, OCT-4+ placental stem cells are isolated away from placental cells that are not stem cells. In another specific embodiment, said isolated CD200+, OCT-4+ placental cells are isolated away from placental cells that do not display these characteristics.

In another embodiment, a cell population useful in the methods and compositions described herein is a population of cells comprising, e.g., that is enriched for, CD200+, OCT-4+ placental stem cells. In various embodiments, at least about 10%, at least about 20%, at least about 30%, at least about 40%, at least about 50%, or at least about 60% of cells in said cell population are isolated CD200+, OCT-4+ placental stem cells. In another embodiment, at least about 70% of said cells are said isolated CD200+, OCT-4+ placental stem cells. In another embodiment, at least about 80%, 90%, 95%, or 99% of cells in said cell population are said isolated CD200+, OCT-4+ placental stem cells. In a specific embodiment of the isolated populations, said isolated CD200+, OCT-4+ placental cells are additionally CD73+ and CD105+. In another specific embodiment, said isolated CD200+, OCT-4+ placental stem cells are additionally HLA-G-. In another specific embodiment, said isolated CD200+, OCT-4+ placental stem cells are additionally CD34-, CD38- and CD45-. In another specific embodiment, said isolated CD200+, OCT-4+ placental cells are additionally CD34-, CD38-, CD45-, CD73+, CD105+ and HLA-G-. In another specific embodiment, the cell population produces one or more embryoid-like bodies when cultured under conditions that allow the formation of embryoid-like bodies. In another specific embodiment, said cell population is isolated away from placental cells that are not isolated CD200+, OCT-4+ placental cells. In another specific embodiment, said cell population is isolated away from placental cells that do not display these markers.

In another embodiment, the isolated placental stem cells useful in the methods and compositions described herein are CD73+, CD105+ and HLA-G-. In another specific embodiment, the isolated CD73+, CD105+ and HLA-G-placental stem cells are additionally CD34-, CD38- or CD45-. In another specific embodiment, the isolated CD73+, CD105+, HLA-G- placental cells are additionally CD34-, CD38- and CD45-. In another specific embodiment, the isolated CD73+, CD105+, HLA-G- placental stem cells are additionally OCT-4+.

In another specific embodiment, the isolated CD73+, CD105+, HLA-G- placental stem cells are additionally CD200+. In another specific embodiment, the isolated CD73+, CD105+, HLA-G- placental stem cells are additionally CD34-, CD38-, CD45-, OCT-4+ and CD200+. In another specific embodiment, the isolated CD73+, CD105+, HLA-G-placental stem cells facilitate the formation of embryoid-like bodies in a population of placental cells comprising said cells, when the population is cultured under conditions that allow the formation of embryoid-like bodies. In another specific embodiment, said the isolated CD73+, CD105+, HLA-G- placental stem cells are isolated away from placental cells that are not the isolated CD73+, CD105+, HLA-G- placental stem cells. In another specific embodiment, said the isolated CD73+, CD105+, HLA-G- placental stem cells are isolated away from placental cells that do not display these markers.

In another embodiment, a cell population useful in the methods and compositions described herein is a population of cells comprising, e.g., that is enriched for, isolated CD73+, CD105 and HLA-G- placental stem cells. In various embodiments, at least about 10%, at least about 20%, at least about 30%, at least about 40%, at least about 50%, or at least about 60% of cells in said population of cells are isolated CD73+, CD105+, HLA-G- placental stem cells. In another embodiment, at least about 70% of cells in said population of cells are isolated CD73+, CD105+, HLA-G-placental stem cells. In another embodiment, at least about 90%, 95% or 99% of cells in said population of cells are isolated CD73+, CD105+, HLA-G- placental stem cells. In a specific embodiment of the above populations, said isolated CD73+, CD105+, HLA-G- placental stem cells are additionally CD34-, CD38- or CD45-. In another specific embodiment, said isolated CD73+, CD105+, HLA-G- placental stem cells are additionally CD34-, CD38- and CD45-. In another specific embodiment, said isolated CD73+, CD105+, HLA-G- placental stem cells are additionally OCT-4+. In another specific embodiment, said isolated CD73+, CD105+, HLA-G- placental stem cells are additionally CD200+. In another specific embodiment, said isolated CD73+, CD105+, HLA-G- placental stem cells are additionally CD34-, CD38-, CD45-, OCT-4+ and CD200+. In another specific embodiment, said cell population is isolated away from placental cells that are not said CD73+, CD105+, HLA-G- placental stem cells. In another specific embodiment, said cell population is isolated away from placental cells that do not display these markers.

In another embodiment, the isolated placental stem cells useful in the methods and compositions described herein are CD73+ and CD105+ and facilitate the formation of one or more embryoid-like bodies in a population of isolated placental cells comprising said CD73+, CD105+ placental stem cells when said population is cultured under conditions that allow formation of embryoid-like bodies. In another specific embodiment, said isolated CD73+, CD105+ placental stem cells are additionally CD34-, CD38- or CD45-. In another specific embodiment, said isolated CD73+, CD105+ placental stem cells are additionally CD34-, CD38-and CD45-. In another specific embodiment, said isolated CD73+, CD105+ placental stem cells are additionally OCT-4+. In another specific embodiment, said isolated CD73+, CD105+ placental stem cells are additionally OCT-4+, CD34-, CD38- and CD45-. In another specific embodiment, said isolated CD73+, CD105+ placental cells are isolated away from placental cells that are not said cells. In another specific embodiment, said isolated CD73+, CD105+ placental cells are isolated away from placental cells that do not display these characteristics.

In another embodiment, a cell population useful in the methods and compositions described herein is a population of cells comprising, e.g., that is enriched for, isolated placental stem cells that are CD73+, CD105+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated CD73+, CD105+ placental cells. In another embodiment, at least about 70% of cells in said population of cells are said isolated CD73+, CD105+ placental stem cells. In another embodiment, at least about 90%, 95% or 99% of cells in said population of cells are said isolated CD73+, CD105+ placental stem cells. In a specific embodiment of the above populations, said isolated CD73+, CD105+ placental stem cells are additionally CD34-, CD38- or CD45-. In another specific embodiment, said isolated CD73+, CD105+ placental stem cells are additionally CD34-, CD38- and CD45-. In another specific embodiment, said isolated CD73+, CD105+ placental stem cells are additionally OCT-4+. In another specific embodiment, said isolated CD73+, CD105+ placental stem cells are additionally CD200+. In another specific embodiment, said isolated CD73+, CD105+ placental stem cells are additionally CD34-, CD38-, CD45-, OCT-4+ and CD200+. In another specific embodiment, said cell population is isolated away from placental cells that are not said isolated CD73+, CD105+ placental stem cells. In another specific embodiment, said cell population is isolated away from placental cells that do not display these markers.

In another embodiment, the isolated placental stem cells useful in the methods and compositions described herein are OCT-4+ and facilitate formation of one or more embryoid-like bodies in a population of isolated placental cells comprising said cells when cultured under conditions that allow formation of embryoid-like bodies. In a specific embodiment, said isolated OCT-4+ placental stem cells are additionally CD73+ and CD105+. In another specific embodiment, said isolated OCT-4+ placental stem cells are additionally CD34-, CD38-, or CD45-. In another specific embodiment, said isolated OCT-4+ placental stem cells are additionally CD200+. In another specific embodiment, said isolated OCT-4+ placental stem cells are additionally CD73+, CD105+, CD200+, CD34-, CD38-, and CD45-. In another specific embodiment, said isolated OCT-4+ placental stem cells are isolated away from placental cells that are not OCT-4+ placental stem cells. In another specific embodiment, said isolated OCT-4+ placental cells are isolated away from placental cells that do not display these characteristics.

In another embodiment, a cell population useful in the methods and compositions described herein is a population of cells comprising, e.g., that is enriched for, isolated placental stem cells that are OCT-4+ and facilitate the formation of one or more embryoid-like bodies in a population of isolated placental cells comprising said cells when said population is cultured under conditions that allow formation of embryoid-like bodies. In various embodiments, at least about 10%, at least about 20%, at least about 30%, at least about 40%, at least about 50%, or at least about 60% of cells in said population of cells are said isolated OCT-4+ placental stem cells. In another embodiment, at least about 70% of cells in said population of cells are said isolated OCT-4+ placental cells. In another embodiment, at least about 80%, 90%, 95% or 99% of cells in said population of cells are said isolated OCT-4+ placental stem cells. In a specific embodiment of the above populations, said isolated OCT-4+ placental stem cells are additionally CD34-, CD38- or CD45-. In another specific embodiment, said isolated OCT-4+ placental stem cells are additionally CD34-, CD38- and CD45-. In another specific embodiment, said isolated OCT-4+ placental stem cells are additionally CD73+ and CD105+. In another specific embodiment, said isolated OCT-4+ placental stem cells are additionally CD200+. In another specific embodiment, said isolated OCT-4+ placental stem cells are additionally CD73+, CD105+, CD200+, CD34-, CD38-, and CD45-. In another specific embodiment, said cell population is isolated away from placental cells that are not said placental stem cells. In another specific embodiment, said cell population is isolated away from placental cells that do not display these markers.

In another embodiment, the isolated placental stem cells useful in the methods and compositions described herein are isolated HLA-A,B,C+, CD45-, CD133- and CD34-placental stem cells. In another embodiment, a cell population useful in the methods and compositions described herein is a population of cells comprising isolated placental stem cells, wherein at least about 70%, at least about 80%, at least about 90%, at least about 95% or at least about 99% of cells in said population of cells are isolated HLA-A,B,C+, CD45-, CD133- and CD34- placental stem cells. In a specific embodiment, said isolated placental stem cell or population of isolated placental stem cells is isolated away from placental cells that are not HLA-A,B,C+, CD45-, CD133- and CD34- placental stem cells. In another specific embodiment, said isolated placental stem cells are non-maternal in origin. In another specific embodiment, said population of isolated placental stem cells are substantially free of maternal components; e.g., at least about 40%, 45%, 5-0%, 55%, 60%, 65%, 70%, 75%, 90%, 85%, 90%, 95%, 98% or 99% of said cells in said population of isolated placental cells are non-maternal in origin.

In another embodiment, the isolated placental stem cells useful in the methods and compositions described herein are isolated CD10+, CD13+, CD33+, CD45-, CD117- and CD 133- placental stem cells. In another embodiment, a cell population useful in the methods and compositions described herein is a population of cells comprising isolated placental stem cells, wherein at least about 70%, at least about 80%, at least about 90%, at least about 95% or at least about 99% of cells in said population of cells are isolated CD10+, CD13+, CD33+, CD45-, CD117- and CD133- placental stem cells. In a specific embodiment, said isolated placental stem cells or population of isolated placental stem cells is isolated away from placental cells that are not said isolated placental stem cells. In another specific embodiment, said isolated CD10+, CD13+, CD33+, CD45-, CD117- and CD133- placental cells are non-maternal in origin, i.e., have the fetal genotype. In another specific embodiment, at least about 40%, 45%, 50%, 55%, 60%, 65%, 70%, 75%, 90%, 85%, 90%, 95%, 98% or 99% of said cells in said population of isolated placental stem cells, are non-maternal in origin. In another specific embodiment, said isolated placental stem cells or population of isolated placental stem cells are isolated away from placental cells that do not display these characteristics.

In another embodiment, the isolated placental stem cells useful in the methods and compositions described herein are isolated CD10+ CD33-, CD44+, CD45-, and CD117-placental stem cells. In another embodiment, a cell population useful for the in the methods and compositions described herein is a population of cells comprising, e.g., enriched for, isolated placental stem cells, wherein at least about 70%, at least about 80%, at least about 90%, at least about 95% or at least about 99% of cells in said population of cells are isolated CD10+ CD33-, CD44+, CD45-, and CD117- placental stem cells. In a specific embodiment, said isolated placental cell or population of isolated placental stem cells is isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these markers.

In another embodiment, the isolated placental stem cells useful in the methods and compositions described herein are isolated CD10+ CD13-, CD33-, CD45-, and CD117-placental stem cells. In another embodiment, a cell population useful in the methods and compositions described herein is a population of cells comprising, e.g., enriched for, isolated CD10+, CD13-, CD33-, CD45-, and CD117- placental stem cells, wherein at least about 70%, at least about 80%, at least about 90%, at least about 95% or at least about 99% of cells in said population are CD10+ CD13-, CD33-, CD45-, and CD117- placental cells. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cell or population of isolated placental stem cells is isolated away from placental cells that do not display these characteristics.

In another embodiment, the isolated placental stem cells useful in the methods and compositions described herein are HLA A,B,C+, CD45-, CD34-, and CD133-, and are additionally CD10+, CD13+, CD38+, CD44+, CD90+, CD105+, CD200+ and/or HLA-G-, and/or negative for CD117. In another embodiment, a cell population useful in the methods described herein is a population of cells comprising isolated placental stem cells, wherein at least about 20%, 25%, 30%, 35%, 40%, 45%, 50%, 55%, 60%, 65%, 70%, 75%, 80%, 85%, 90%, 95%, 98% or about 99% of the cells in said population are isolated placental stem cells that are HLA A,B,C+, CD45-, CD34-, CD133-, and that are additionally positive for CD10, CD13, CD38, CD44, CD90, CD105, CD200, and/or negative for CD117 and/or HLA-G. In a specific embodiment, said isolated placental stem cells or population of isolated placental stem cells are isolated away from placental cells that are not said cells. In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin. In another specific embodiment, said isolated placental stem cells or population of isolated placental stem cells are isolated away from placental cells that do not display these markers.

In another embodiment, the isolated placental stem cells useful in the methods and compositions described herein are isolated placental stem cells that are CD200+ and CD10+, as determined by antibody binding, and CD117-, as determined by either antibody binding or RT-PCR. In another embodiment, the isolated placental stem cells useful in the methods and compositions described herein are isolated placental stem cells, e.g., placental stem cells or placental multipotent cells, that are CD10+, CD29+, CD54+, CD200+, HLA-G-, MHC class I+ and $\beta$-2-microglobulin+. In another embodiment, isolated placental stem cells useful in the methods and compositions described herein are placental cells wherein the expression of at least one cellular marker is at least two-fold higher than for a mesenchymal stem cell (e.g., a bone marrow-derived mesenchymal stem cell). In another specific embodiment, said isolated placental stem cells are non-maternal in origin. In another specific embodiment, at least about 40%, 45%, 50%, 55%, 60%, 65%, 70%, 75%, 90%, 85%, 90%, 95%, 98% or 99% of said cells in said cell population are non-maternal in origin.

In another embodiment, the isolated placental stem cells useful in the methods and compositions described herein are isolated placental stem cells, e.g., placental stem cells or placental multipotent cells, that are one or more of CD10+, CD29+, CD44+, CD45-, CD54/ICAM+, CD62E-. CD62L-, CD62P-, CD80-, CD86-, CD103-, CD104-, CD105+, CD106/VCAM+, CD144/VE-cadherin$^{low}$, CD184/CXCR4-, $\beta$-microglobulin$^{low}$, MHC-I$^{low}$, MHC-II-, HLA-G$^{low}$, and/or PDL1$^{low}$. In a specific embodiment, the isolated placental stem cells are at least CD29+ and CD54+. In another specific embodiment, the isolated placental stem cells are at least CD44+ and CD106+. In another specific embodiment, the isolated placental stem cells are at least CD29+.

In another embodiment, a cell population useful in the methods and compositions described herein comprises isolated placental stem cells, wherein at least 50%, 60%, 70%, 80%, 90%, 95%, 98% or 99% of the cells in said cell population are isolated placental stem cells that are one or more of CD10+, CD29+, CD44+, CD45-, CD54/ICAM+, CD62-E-, CD62-L-, CD62-P-, CD80-, CD86-, CD103-, CD104-, CD105+, CD106/VCAM+, CD144/VE-cadherin$^{dim}$, CD184/CXCR4-, $\beta$-microglobulin$^{dim}$, HLA-I$^{dim}$, HLA-II-, HLA-G$^{dim}$, and/or PDL1$^{dim}$. In another specific embodiment, at least 50%, 60%, 70%, 80%, 90%, 95%, 98% or 99% of cells in said cell population are CD10+, CD29+, CD44+, CD45-, CD54/ICAM+, CD62-E-, CD62-L-, CD62-P-, CD80-, CD86-, CD103-, CD104-, CD105+, CD106/VCAM+, CD144NE-cadherin$^{dim}$, CD184/CXCR4-, $\beta$-microglobulin$^{dim}$, MHC-I$^{dim}$, MHC-II-, HLA-G$^{dim}$, and PDL1$^{dim}$. In certain embodiments, the placental cells express HLA-II markers when induced by interferon gamma (IFN-$\gamma$).

In another embodiment, the isolated placental stem cells useful in the methods and compositions described herein are isolated placental stem cells that are one or more, or all, of CD10+, CD29+, CD34-, CD38-, CD44+, CD45-, CD54+, CD90+, SH2+, SH3+, SH4+, SSEA3-, SSEA4-, OCT-4+, and ABC-p+, where ABC-p is a placenta-specific ABC transporter protein (also known as breast cancer resistance protein (BCRP) and as mitoxantrone resistance protein (MXR)), wherein said isolated placental stem cells are obtained, e.g., by perfusion of a mammalian, e.g., human, placenta that has been drained of cord blood and perfused to remove residual blood.

In another specific embodiment of any of the above characteristics, expression of the cellular marker (e.g., cluster of differentiation or immunogenic marker) is determined by flow cytometry; in another specific embodiment, expression of the marker is determined by RT-PCR.

Gene profiling confirms that isolated placental stem cells, and populations of isolated placental stem cells, are distinguishable from other cells, e.g., mesenchymal stem cells, e.g., bone marrow-derived mesenchymal stem cells. The isolated placental stem cells described herein can be distinguished from, e.g., mesenchymal stem cells on the basis of the expression of one or more genes, the expression of which is significantly higher in the isolated placental stem cells in comparison to bone marrow-derived mesenchymal stem cells. In particular, the isolated placental stem cells, useful in the methods of treatment provided herein, can be distinguished from mesenchymal stem cells on the basis of the expression of one or more genes, the expression of which is significantly higher (that is, at least twofold higher) in the isolated placental stem cells than in an equivalent number of bone marrow-derived mesenchymal stem cells, wherein the one or more genes are ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, ZC3H12A, or a combination of any of the foregoing, when the cells are grown under equivalent conditions. See, e.g., U.S. Patent Application Publication No. 2007/0275362. In certain specific embodiments, said expression of said one or more genes is determinable, e.g., by RT-PCR or microarray analysis, e.g., using a U133-A microarray (Affymetrix). In another specific embodiment, said isolated placental stem cells express said one or more genes when cultured for a number of population doublings, e.g., anywhere from about 3 to about 35 population doublings, in a medium comprising DMEM-LG (e.g., from Gibco); 2% fetal calf serum (e.g., from Hyclone Labs.); 1X insulin-transferrin-selenium (ITS); 1X linoleic acid-bovine serum albumin (LA-BSA); $10^{-9}$ M dexamethasone (e.g., from Sigma); $10^{-4}$ M ascorbic acid 2-phosphate (e.g., from Sigma); epidermal growth factor 10 ng/mL (e.g., from R&D Systems); and platelet-derived growth factor (PDGF-BB) 10 ng/mL (e.g., from R&D Systems). In another specific embodiment, the isolated placental cell-specific gene is CD200.

Specific sequences for these genes can be found in GenBank at accession nos. NM_001615 (ACTG2), BC065545 (ADARB1), (NM_181847 (AMIGO2), AY358590 (ARTS-1), BC074884 (B4GALT6), BC008396 (BCHE), BCO20196 (C11orf9), BCO31103 (CD200), NM_001845 (COL4A1), NM_001846 (COL4A2), BCO52289 (CPA4), BC094758 (DMD), AF293359 (DSC3), NM_001943 (DSG2), AF338241 (ELOVL2), AY336105 (F2RL1), NM_018215 (FLJ10781), AY416799 (GATA6), BC075798 (GPR126), NM_016235 (GPRC5B), AF340038 (ICAM1), BC000844 (IER3), BC066339 (IGFBP7), BC013142 (ILIA), BT019749 (IL6), BC007461 (IL18), (BC072017) KRT18, BC075839 (KRT8), BC060825 (LIPG), BC065240 (LRAP), BC010444 (MATN2), BC011908 (MEST), BC068455 (NFE2L3), NM_014840 (NUAK1), AB006755 (PCDH7), NM_014476 (PDLIM3), BC126199 (PKP-2), BC090862 (RTN1), BC002538 (SERPINB9), BCO23312 (ST3GAL6), BC001201 (ST6GALNAC5), BC126160 or BC065328 (SLC12A8), BCO25697 (TCF21), BC096235 (TGFB2), BC005046 (VTN), and BC005001 (ZC3H12A) as of March 2008.

In certain specific embodiments, said isolated placental stem cells express each of ACTG2, ADARB1, AMIGO2, ARTS-1, B4GALT6, BCHE, C11orf9, CD200, COL4A1, COL4A2, CPA4, DMD, DSC3, DSG2, ELOVL2, F2RL1, FLJ10781, GATA6, GPR126, GPRC5B, ICAM1, IER3, IGFBP7, ILIA, IL6, IL18, KRT18, KRT8, LIPG, LRAP, MATN2, MEST, NFE2L3, NUAK1, PCDH7, PDLIM3, PKP2, RTN1, SERPINB9, ST3GAL6, ST6GALNAC5, SLC12A8, TCF21, TGFB2, VTN, and ZC3H12A at a detectably higher level than an equivalent number of bone marrow-derived mesenchymal stem cells, when the cells are grown under equivalent conditions.

In specific embodiments, the placental cells express CD200 and ARTS 1 (aminopeptidase regulator of type 1 tumor necrosis factor); ARTS-1 and LRAP (leukocyte-derived arginine aminopeptidase); IL6 (interleukin-6) and TGFB2 (transforming growth factor, beta 2); IL6 and KRT18 (keratin 18); IER3 (immediate early response 3), MEST (mesoderm specific transcript homolog) and TGFB2; CD200 and IER3; CD200 and IL6; CD200 and KRT18; CD200 and LRAP; CD200 and MEST; CD200 and NFE2L3 (nuclear factor (erythroid-derived 2)-like 3); or CD200 and TGFB2 at a detectably higher level than an equivalent number of bone marrow-derived mesenchymal stem cells (BM-MSCs) wherein said bone marrow-derived mesenchymal stem cells have undergone a number of passages in culture equivalent to the number of passages said isolated placental stem cells have undergone. In other specific embodiments, the placental cells express ARTS-1, CD200, IL6 and LRAP; ARTS-1, IL6, TGFB2, IER3, KRT18 and MEST; CD200, IER3, IL6, KRT18, LRAP, MEST, NFE2L3, and TGFB2; ARTS-1, CD200, IER3, IL6, KRT18, LRAP, MEST, NFE2L3, and TGFB2; or IER3, MEST and TGFB2 at a detectably higher level than an equivalent number of bone marrow-derived mesenchymal stem cells BM-MSCs, wherein said bone marrow-derived mesenchymal stem cells have undergone a number of passages in culture equivalent to the number of passages said isolated placental stem cells have undergone.

Expression of the above-referenced genes can be assessed by standard techniques. For example, probes based on the sequence of the gene(s) can be individually selected and constructed by conventional techniques. Expression of the genes can be assessed, e.g., on a microarray comprising probes to one or more of the genes, e.g. an Affymetrix GENECHIP™ Human Genome U133A 2.0 array, or an Affymetrix GENECHIP™ Human Genome U133 Plus 2.0 (Santa Clara, Calif.). Expression of these genes can be assessed even if the sequence for a particular GenBank accession number is amended because probes specific for the amended sequence can readily be generated using well-known standard techniques.

The level of expression of these genes can be used to confirm the identity of a population of isolated placental stem cells, to identify a population of cells as comprising at least a plurality of isolated placental stem cells, or the like. Populations of isolated placental stem cells, the identity of which is confirmed, can be clonal, e.g., populations of isolated placental stem cells expanded from a single isolated placental cell, or a mixed population of stem cells, e.g., a population of cells comprising solely isolated placental stem cells that are expanded from multiple isolated placental stem cells, or a population of cells comprising isolated placental stem cells, as described herein, and at least one other type of cell.

The placental stem cells can be obtained by perfusion, e.g., produced according to a method comprising perfusing a mammalian placenta that has been drained of cord blood and perfused to remove residual blood; perfusing said placenta with a perfusion solution; and collecting said perfusion solution, wherein said perfusion solution after perfusion comprises a population of placental cells that comprises placental stem cells; and isolating a plurality of said placental stem cells from said population of cells. In a specific embodiment, the perfusion solution is passed through both the umbilical vein and umbilical arteries and collected after it exudes from the placenta. Populations of placental stem cells produced by this method typically comprise a mixture of fetal and maternal cells. In another specific embodiment, the perfusion solution is passed through the umbilical vein and collected from the umbilical arteries, or passed through the umbilical arteries and collected from the umbilical vein. Populations of placental stem cells produced by this method typically are substantially exclusively fetal in origin; that is, e.g., greater than 90%, 95%, 99%, or 99.5% of the placental stem cells in the population are fetal in origin.

In various embodiments, the placental stem cells, contained within a population of cells obtained from perfusion of a placenta, are at least 50%, 60%, 70%, 80%, 90%, 95%, 99% or at least 99.5% of said population of placental cells. In another specific embodiment, the placental stem cells collected by perfusion comprise fetal and maternal cells. In another specific embodiment, the placental stem cells collected by perfusion are at least 50%, 60%, 70%, 80%, 90%, 95%, 99% or at least 99.5% fetal cells.

Placental stem cells can also be isolated from a mammalian placenta by physical disruption, e.g., enzymatic digestion, of the organ or a portion thereof. For example, the placenta, or a portion thereof, may be, e.g., crushed, sheared, minced, diced, chopped, macerated or the like, and the tissue subsequently digested with one or more enzymes. The placenta, or a portion thereof, may also be physically disrupted and digested with one or more enzymes, and the resulting material then immersed in, or mixed into, e.g., medium or buffer. Any method of physical disruption can be used, provided that the method of disruption leaves a plurality, more preferably a majority, and more preferably at least 60%, 70%, 80%, 90%, 95%, 98%, or 99% of the cells in said organ viable, as determined by, e.g., trypan blue exclusion.

The placenta can be dissected into components prior to physical disruption and/or enzymatic digestion and stem cell recovery. For example, placental stem cells can be obtained from the amniotic membrane, chorion, umbilical cord, placental cotyledons, or any combination thereof. Typically, placental stem cells can be obtained by disruption of a small block of placental tissue, e.g., a block of placental tissue that is about 1, 2, 3, 4, 5, 6, 7, 8, 9, 10, 20, 30, 40, 50, 60, 70, 80, 90, 100, 200, 300, 400, 500, 600, 700, 800, 900 or about 1000 cubic millimeters in volume.

A preferred stem cell collection composition, useful in placental stem cells by perfusion or physical/enzymatic disruption of placental tissue, comprises one or more tissue-disruptive enzyme(s), e.g., collagenase, dispase, hyaluronidase, LIBERASE (Boehringer Mannheim Corp., Indianapolis, Ind.), papain, deoxyribonucleases, serine proteases, such as trypsin, chymotrypsin, or elastase, or the like. Any combination of tissue digestion enzymes can be used. Typical concentrations for tissue digestion enzymes include, e.g., 50-200 U/mL for collagenase I and collagenase IV, 1-10 U/mL for dispase, and 10-100 U/mL for elastase. Proteases can be used in combination, that is, two or more proteases in the same digestion reaction, or can be used sequentially in order to liberate placental stem cells. For example, in one embodiment, a placenta, or part thereof, is digested first with an appropriate amount of collagenase I at 2 mg/ml for 30 minutes, followed by digestion with trypsin, 0.25%, for 10 minutes, at 37°C Serine proteases are preferably used consecutively following use of other enzymes.

In another embodiment, the tissue can further be disrupted by the addition of a chelator, e.g., ethylene glycol bis(2-aminoethyl ether)-N,N,N'N'-tetraacetic acid (EGTA) or ethylenediaminetetraacetic acid (EDTA) to the stem cell collection composition comprising the stem cells, or to a solution in which the tissue is disrupted and/or digested prior to isolation of the stem cells with the stem cell collection composition.

Where an entire placenta, or portion of a placenta comprising both fetal and maternal cells (for example, where the portion of the placenta comprises the chorion or cotyledons), the placental stem cells collected will comprise a mix of placental stem cells derived from both fetal and maternal sources. Where a portion of the placenta that comprises no, or a negligible number of, maternal cells (for example, amnion), the placental stem cells collected will comprise almost exclusively fetal placental stem cells.

### 5.4.2. Isolation and Characterization of Placental Stem Cells

Stem cells from mammalian placenta, whether obtained by perfusion or enzymatic digestion, can initially be purified from (i.e., be isolated from) other cells by, e.g., Ficoll gradient centrifugation. Such centrifugation can follow any standard protocol for centrifugation speed, etc. In one embodiment, for example, cells collected from the placenta are recovered from perfusate by centrifugation at 5000 X g for 15 minutes at room temperature, which separates cells from, e.g., contaminating debris and platelets. In another embodiment, placental perfusate is concentrated to about 200 ml, gently layered over Ficoll, and centrifuged at about 1100 X g for 20 minutes at 22°C, and the low-density interface layer of cells is collected for further processing.

Cell pellets can be resuspended in fresh stem cell collection composition, or a medium suitable for stem cell maintenance, e.g., IMDM serum-free medium containing 2 U/ml heparin and 2 mM EDTA (GibcoBRL, NY). The total mononuclear cell fraction can be isolated, e.g., using Lymphoprep (Nycomed Pharma, Oslo, Norway) according to the manufacturer's recommended procedure.

Placental cells obtained by perfusion or digestion can, for example, be further, or initially, isolated by differential trypsinization using, e.g., a solution of 0.05% trypsin with 0.2% EDTA (Sigma, St. Louis, Missouri). Differential trypsinization is possible because placental stem cells typically detach from plastic surfaces within about five minutes whereas other adherent populations typically require more than 20-30 minutes incubation. The detached placental stem cells can be harvested following trypsinization and trypsin neutralization, using, e.g., Trypsin Neutralizer Solution (Cascade Biologics, Portland, Oregon). In one embodiment of isolation of adherent cells, aliquots of, for example, about 5-10 X $10^6$ cells are placed in each of several T-75 flasks, preferably fibronectin-coated T75 flasks. In such an embodiment, the cells can be cultured with, e.g., a commercially available mesenchymal stem cell culture medium such as MESENCULT® (Stemcell Technologies, Vancouver, BC, Canada), and placed in a tissue culture incubator (37°C, 5% $CO_2$). After 10 to 15 days, non-adherent cells are removed from the flasks by washing with PBS. The PBS is then replaced by MESENCULT® or similar medium. Flasks are preferably examined daily for the presence of various adherent cell types and in particular, for identification and expansion of clusters of fibroblastoid cells.

The number and type of cells collected from a mammalian placenta can be monitored, for example, by measuring changes in morphology and cell surface markers using standard cell detection techniques such as flow cytometry, cell sorting, immunocytochemistry (e.g., staining with tissue specific or cell-marker specific antibodies) fluorescence activated cell sorting (FACS), magnetic activated cell sorting (MACS), by examination of the morphology of cells using light or confocal microscopy, and/or by measuring changes in gene expression using techniques well known in the art, such as PCR and gene expression profiling. These techniques can be used, too, to identify cells that are positive for one or more particular markers. For example, using antibodies to CD34, one can determine, using the techniques above, whether a cell comprises a detectable amount of CD34; if so, the cell is CD34+. Likewise, if a cell produces enough OCT-4 RNA to be detectable by RT-PCR, or significantly more OCT-4 RNA than an adult cell, the cell is OCT-4+. Antibodies to cell surface markers (e.g., CD markers such as CD34) and the sequence of stem cell-specific genes, such as OCT-4, are well-known in the art.

Placental stem cells, particularly cells that have been isolated by Ficoll separation, differential adherence, or a combination of both, may be sorted using a fluorescence activated cell sorter (FACS). Fluorescence activated cell sorting (FACS) is a well-known method for separating particles, including cells, based on the fluorescent properties of the particles (see, e.g., Kamarch, 1987, Methods Enzymol, 151:150-165). Laser excitation of fluorescent moieties in the individual particles results in a small electrical charge allowing electromagnetic separation of positive and negative particles from a mixture. In one embodiment, cell surface marker-specific antibodies or ligands are labeled with distinct fluorescent labels. Cells are processed through the cell sorter, allowing separation of cells based on their ability to bind to the antibodies used. FACS sorted particles may be directly deposited into individual wells of 96-well or 384-well plates to facilitate separation and cloning. Antibodies linked to magnetic beads may also be used to sort cells.

In one sorting scheme, stem cells from placenta are sorted on the basis of expression of the markers CD34, CD38, CD44, CD45, CD73, CD105, OCT-4 and/or HLA-G. This can be accomplished in connection with procedures to select stem cells on the basis of their adherence properties in culture. For example, an adherence selection stem can be accomplished before or after sorting on the basis of marker expression. In one embodiment, for example, cells are sorted first on the basis of their expression of CD34; CD34- cells are retained, and cells that are CD200+HLA-G-, are separated from all other CD34- cells. In another embodiment, cells from placenta are based on their expression of CD200 and/or lack of expression of HLA-G; for example, cells displaying either of these markers are isolated for further use. Cells that express, e.g., CD200 and/or lack expression of HLA-G can, in a specific embodiment, be further sorted based on their expression of CD73 and/or CD105, or epitopes recognized by antibodies SH2, SH3 or SH4, or lack of expression of CD34, CD38 or CD45. For example, in one embodiment, placental cells are sorted by expression, or lack thereof, of CD200, HLA-G, CD73, CD105, CD34, CD38 and CD45, and placental cells that are CD200+, HLA-G-, CD73+, CD105+, CD34-, CD38- and CD45- are isolated from other placental cells for further use.

In another embodiment, magnetic beads can be used to separate cells. The cells may be sorted using a magnetic activated cell sorting (MACS) technique, a method for separating particles based on their ability to bind magnetic beads (0.5-100 μm diameter). A variety of useful modifications can be performed on the magnetic microspheres, including covalent addition of antibody that specifically recognizes a particular cell surface molecule or hapten. The beads are then mixed with the cells to allow binding. Cells are then passed through a magnetic field to separate out cells having the specific cell surface marker. In one embodiment, these cells can then isolated and re-mixed with magnetic beads coupled to an antibody against additional cell surface markers. The cells are again passed through a magnetic field, isolating cells that bound both the antibodies. Such cells can then be diluted into separate dishes, such as microtiter dishes for clonal isolation.

Placental stem cells can be assessed for viability, proliferation potential, and longevity using standard techniques known in the art, such as trypan blue exclusion assay, fluorescein diacetate uptake assay, propidium iodide uptake assay (to assess viability); and thymidine uptake assay, MTT cell proliferation assay (to assess proliferation). Longevity may be determined by methods well known in the art, such as by determining the maximum number of population doubling in an extended culture.

Placental stem cells can also be separated from other placental cells using other techniques known in the art, e.g., selective growth of desired cells (positive selection), selective destruction of unwanted cells (negative selection); separation based upon differential cell agglutinability in the mixed population as, for example, with soybean agglutinin; freeze-thaw procedures; filtration; conventional and zonal centrifugation; centrifugal elutriation (counter-streaming centrifugation); unit gravity separation; countercurrent distribution; electrophoresis; and the like.

### 5.4.3. Culture of Placental Stem Cells

Placental stem cells can be isolated as described above and immediately contacted with double-dried ECM. Placental stem cells can also be cultured, e.g., in cell culture, for a number of generations prior to contacting with double-dried ECM. For example, isolated placental stem cells, or placental stem cell population, or cells or placental tissue from which placental stem cells grow out, can be used to initiate, or seed, cell cultures. Cells are generally transferred to sterile tissue culture vessels either uncoated or coated with extracellular matrix or ligands such as laminin, collagen (e.g., native or denatured), gelatin, fibronectin, ornithine, vitronectin, and/or commercially-available extracellular membrane protein.

In certain embodiments, the placental stem cells are cultured on the double-dried ECM provided herein. In certain embodiments, the double-dried ECM comprises detectable amounts of fibronectin and laminin. In other embodiments, the EC double-dried ECM comprises no detectable amount of fibronectin or laminin. In other embodiments, the double-dried ECM comprises at least about 5%, or at least about 10%, elastin by dry weight. In another embodiment, the ECM comprises no more than about 5% elastin by dry weight.

In certain embodiments, placental stem cells are cultured for the production of specific cytokines that are collectable from the culture medium. In specific embodiments, the cytokine is IL-6, IL-8, and/or monocyte chemotactic protein-1 (MCP-1). In certain other embodiments, the placental stem cells are cultured for the production of fibronectin. In a specific embodiment, the placental stem cells are cultured on double-dried ECM which comprises less than about 5% fibronectin.

As noted above, double-dried ECM can be shaped into any shape that is useful, e.g., medically useful. These compositions, once shaped and dried, are typically stable in aqueous solution, e.g., tissue culture medium or buffer. Thus, stem cells, such as placental stem cells, can be cultured directly on the shaped compositions. Such culturing can be done in cell culture dishes or other liquid containers, e.g., flasks, suitable for cell culture.

Placental stem cells can be cultured in any medium, and under any conditions, recognized in the art as acceptable for the culture of stem cells. In certain embodiments, the culture medium comprises serum, e.g., human serum or bovine calf serum/fetal calf serum. In certain other embodiments, the culture medium is serum-free. Placental stem cells can be cultured in, for example, DMEM-LG (Dulbecco's Modified Essential Medium, low glucose)/MCDB 201 (chick fibroblast basal medium) containing ITS (insulin-transferrin-selenium), LA+BSA (linoleic acid-bovine serum albumin), dextrose, L-ascorbic acid, PDGF, EGF, IGF-1, and penicillin/streptomycin; DMEM-HG (high glucose) comprising 10% fetal bovine serum (FBS); DMEM-HG comprising 15% FBS; IMDM (Iscove's modified Dulbecco's medium) comprising 10% FBS, 10% horse serum, and hydrocortisone; M199 comprising 10% FBS, EGF, and heparin; {umlaut over (.gamma.)}-MEM (minimal essential medium) comprising 10% FBS, GLUTAMAX.TM. and gentamicin; DMEM comprising 10% FBS, GLUTAMAX.TM. and gentamicin, etc. In one embodiment, the medium is DMEM-LG/MCDB-201 comprising 2% FBS, ITS, LA+BSA, dextrose, L-ascorbic acid, PDGF, EGF, and penicillin/streptomycin.

Other media in that can be used to culture placental stem cells include DMEM (high or low glucose), Eagle's basal medium, Ham's F10 medium (F10), Ham's F-12 medium (F12), Iscove's modified Dulbecco's medium, Mesenchymal Stem Cell Growth Medium (MSCGM), Liebovitz's L-15 medium, MCDB, DMEM/F12, RPMI 1640, advanced DMEM (Gibco), DMEM/MCDB201 (Sigma), and CELL-GRO FREE.

The culture medium can be supplemented with one or more components including, for example, serum (e.g., fetal bovine serum (FBS), preferably about 2-15% (v/v); equine (horse) serum (ES); human serum (HS)); beta-mercaptoethanol (BME), preferably about 0.001% (v/v); one or more growth factors, for example, platelet-derived growth factor (PDGF), epidermal growth factor (EGF), basic fibroblast growth factor (bFGF), insulin-like growth factor-1 (IGF-1), leukemia inhibitory factor (LIF), vascular endothelial growth factor (VEGF), and erythropoietin (EPO); amino acids, including L-valine; and one or more antibiotic and/or antimycotic agents to control microbial contamination, such as, for example, penicillin G, streptomycin sulfate, amphotericin B, gentamicin, and nystatin, either alone or in combination.

Placental stem cells can be cultured in standard tissue culture conditions, e.g., in tissue culture dishes or multiwell plates. Placental stem cells can also be cultured using a hanging drop method. In this method, placental stem cells are suspended at about $1 \times 10^4$ cells per mL in about 5 mL of medium, and one or more drops of the medium are placed on the inside of the lid of a tissue culture container, e.g., a 100 mL Petri dish. The drops can be, e.g., single drops, or multiple drops from, e.g., a multichannel pipetter. The lid is carefully inverted and placed on top of the bottom of the dish, which contains a volume of liquid, e.g., sterile PBS sufficient to maintain the moisture content in the dish atmosphere, and the stem cells are cultured.

Once an isolated placental stem cell, or isolated population of stem cells comprising placental stem cells (e.g., a stem cell or population of stem cells separated from at least 50% of the placental cells with which the stem cell or population of stem cells is normally associated in vivo) is obtained, the placental stem cells or population of cells can be proliferated and expanded in vitro. For example, placental stem cells can be cultured in tissue culture containers, e.g., dishes, flasks, multiwell plates, or the like, for a sufficient time for the stem cells to proliferate to 70-90% confluence, that is, until the stem cells and their progeny occupy 70-90% of the culturing surface area of the tissue culture container.

Placental stem cells can be seeded in culture vessels at a density that allows cell growth. For example, the cells may be seeded at low density (e.g., about 1,000 to about 5,000 cells/cm$^2$) to high density (e.g., about 50,000 or more cells/cm$^2$). In a preferred embodiment, the cells are cultured at about 0 to about 5 percent by volume $CO_2$ in air. In some preferred embodiments, the cells are cultured at about 2 to about 25 percent $O_2$ in air, preferably about 5 to about 20 percent $O_2$ in air. The cells preferably are cultured at about 25°C to about 40°C, preferably 37°C. The cells are preferably cultured in an incubator. The culture medium can be static or agitated, for example, using a bioreactor. Placental stem cells may be grown under low oxidative stress (e.g., with addition of glutathione, ascorbic acid, catalase, tocopherol, N-acetylcysteine, or the like).

Once 70%-90% confluence is obtained, the cells may be passaged. For example, the cells can be enzymatically treated, e.g., trypsinized, using techniques well-known in the art, to separate them from the tissue culture surface. After removing the cells by pipetting and counting the cells, about 20,000-100,000 stem cells, preferably about 50,000 stem cells, are passaged to a new culture container containing fresh culture medium. Typically, the new medium is the same type of medium from which the stem cells were removed. Placental stem cells that have been passaged at least 1, 2, 3, 4, 5, 6, 7, 8, 9, 10, 12, 14, 16, 18, or 20 times, or more, can be used in combination with the double-dried ECM.

## 5.5. Non-Stem Cells

The double-dried ECM can, in certain embodiments, comprise one or more types of non-stem cells. As used herein, "non-stem cell" indicates a terminally-differentiated cell. For example, in one embodiment, the double-dried ECM comprises a plurality of fibroblasts. Non-stem cells that can be co0mbined with the double-dried ECM include, without limitation, fibroblasts or fibroblast-like cells, dermal cells, endothelial cells, epithelial cells, muscle cells, cardiac cells, pancreatic cells, and the like. In certain other embodiments, the composition comprises at least two types of stem cells and at least two types of non-stem cells.

For any of the above embodiments in which double-dried ECM is combined with stem cells or non-stem cells, the cells and the double-dried ECM can be administered to an individual together, e.g., as a unitary composition. For example, in one embodiment, the composition can comprise stem cells that have been contacted with the composition immediately prior (e.g., within 10-20 minutes) of administering the composition to the individual. In another embodiment, the stem cells can be contacted with the composition at a time prior to administration sufficient to allow the stem cells to attach to the composition, typically at least 1 hour prior to administration. In a more specific embodiment, the time prior to administration is a time sufficient for the stem cells to attach and proliferate, typically at least 24 hours to 48 hours, or more, prior to administration. In another more specific embodiment, the time is a time sufficient for the stem cells to attach to, and proliferate on, double-dried ECM, and to deposit a detectable amount of an extracellular matrix protein, e.g., fibronectin.

The double-dried ECM, and stem cells or non-stem cells, can be administered to the individual separately, as well. For instance, in one embodiment, the composition can be administered to an individual, e.g., at the site of a wound or tissue needing repair, and the stem cells can be subsequently administered. In another embodiment, the stem cells are contacted with the site of an oral lesion, and the wound or tissue needing repair is subsequently contacted with double-dried ECM.

In one embodiment, therefore, provided herein is a method of promoting the healing of an oral lesion, comprising contacting the lesion with double-dried ECM comprising stem cells, e.g., placental stem cells, wherein the stem cells secrete IL-6, IL-8 or MCP-1, or a any combination thereof, or secrete fibronectin, into at least a portion of the lesion. Where the stem cells are to secrete fibronectin, it is preferred that double-dried ECM comprise an undetectable amount of fibronectin. In a specific embodiment, the double-dried ECM is shaped or formed approximately to the shape of the oral lesion.

### 5.6. Kits Comprising the double-dried ECM

In another aspect provided herein are kits comprising double-dried ECM, and additional components, to facilitate treatment of any of the indications described herein, e.g., an oral lesion. In certain embodiments, the kit comprises one or more packages of double-dried ECM for distribution to a practitioner of skill in the art. The kits can comprise a label or labeling with instructions on using the double-dried ECM in the treatment of the indication. In certain embodiments, the kits can comprise components useful for carrying out the methods such as means for administering the double-dried ECM such as one or more spray bottles, tweezers, spatula (for applying paste), cannulas, catheters, etc. In certain embodiments, the kit can comprise one or more components useful for the safe disposal of means for administering the double-dried ECM (e.g. a 'sharps' container). In certain embodiments, the kits can comprise double-dried ECM in pre-filled syringes, unit-dose or unit-of-use packages.

In certain other embodiments, the kit comprises double-dried ECM and one or more other components for the culture of a population of stem cells or non-stem cells. For example, the kit can comprise double-dried ECM in one or more configurations suitable for the culture of stem cells, e.g., placental stem cells, e.g., double-dried ECM in the form of a sheet, tube, mesh, and the like. The kit can comprise one or more items to be used for the culture of stem cells, e.g., culture dishes that are able to contain double-dried ECM during cell culture; plasticware, syringes, pipet tips, cell culture media, one or more cytokines or growth factors, disposables, and the like.

In other embodiments, the kit can comprise one or more components that facilitate the collection of stem cells from placental tissue. In various specific embodiments, the kit comprises components that facilitate perfusion of a placenta to collect stem cells, e.g., perfusion solution; one or more trays large enough to contain a placenta, glassware or plasticware for collection of perfusion solution; one or more bags for collection of perfusion solution, needs and/or canulae for canalizing umbilical vessels; proteases suitable for tissue digestion, implements for macerating or otherwise rendering placental tissue, and the like. In other specific embodiments, the kit comprises one or more components that facilitate enzymatic digestion of placental tissue to isolate placental stem cells, e.g., one or more tissue-digesting enzymes (e.g., trypsin, chymotrypsin, or the like); plasticware suitable for cell culture (e.g., culture dishes, multiwell culture plates, and the like).

### 5.7. Methods of Treatment Using the Double-Dried ECM

The double-dried ECMs have a broad array of potential uses, including, but not limited to, manufacture of engineered tissue and organs, including structures such as patches or plugs of tissues or matrix material, prosthetics, and other implants, tissue scaffolding, repair or dressing of wounds, hemostatic devices, devices for use in tissue repair and support such as sutures, surgical and orthopedic screws, and surgical and orthopedic plates, natural coatings or components for synthetic implants, cosmetic implants and supports, repair or structural support for organs or tissues, substance delivery, bioengineering platforms, platforms for testing the effect of substances upon cells, cell culture, and numerous other uses. This discussion of possible uses is not intended to be exhaustive and many other embodiments exist. Furthermore, although many specific examples are provided below regarding combination of collagen with other materials and/or specific substances, many other combinations of materials and substances may be used.

In certain embodiments, the double-dried ECM is administered to a subject. The term "subject" refers to animals such as mammals, including, but not limited to, primates (e.g., humans), cows, sheep, goats, horses, dogs, cats, rabbits, rats, mice and the like. In certain embodiments, the subject is a human.

In applications in which the double-dried ECM is to be used for the treatment or filling of a wound, it may be advantageous for the composition to stimulate the production of fibronectin by stem cells in surrounding tissues. In such an embodiment, the wound can be contacted with a double-dried ECM that comprises little or no detectable amount of fibronectin.

The ability to combine cells in a double-dried ECM provides the ability to use the double-dried ECM to build tissue, organs, or organ-like tissue. Cells included in such tissues or organs can include cells that serve a function of delivering a substance, seeded cells that will provide the beginnings of replacement tissue, or both. Many types of cells can be used to create tissue or organs. Stem cells, committed stem cells, and/or differentiated cells are used in various embodiments. Examples of stem cells used in these embodiments include, but are not limited to, embryonic stem cells, bone marrow stem cells and umbilical cord stem cells used to make organs or organ-like tissue such as livers or kidneys. In some embodiments the shape of the composition helps send signals to the cells to grow and reproduce in a specific type of desired way. Other substances, for example differentiation inducers, can be added to the matrix to promote specific types of cell growth. Further, different mixtures of cell types are incorporated into the composition in some embodiments. The ability to use collagen materials and matrices to bioengineer tissue or organs creates a wide variety of bioengineered tissue replacement applications. Examples of bioengineered components include, but are not limited to, bone, dental structures, joints, cartilage, skeletal muscle, smooth muscle, cardiac muscle, tendons, menisci, ligaments, blood vessels, stents, heart valves, corneas, ear drums, nerve guides, tissue or organ patches or sealants, a filler for missing tissues, sheets for cosmetic repairs, skin (sheets with cells added to make a skin equivalent), soft tissue structures of the throat such as trachea, epiglottis, and vocal cords, other cartilaginous structures such as nasal cartilage, tarsal plates, tracheal rings, thyroid cartilage, and arytenoid cartilage, connective tissue, vascular grafts and components thereof, and sheets for topical applications, and repair to or replacement of organs such as livers, kidneys, and pancreas. In some embodiments, such matrices are combined with drug and substance delivery matrices provided herein in ways that will improve the function of the implant. For example, antibiotics, anti-inflammatory agents, local anesthetics or combinations thereof, can be added to the matrix of a bioengineered organ to speed the healing process and reduce discomfort.

### 5.7.1. Methods of Treating Oral Lesions Using ECM

The double-dried ECM provided herein is, in one aspect, used to treat an oral lesion, wherein said lesion is not caused by a dental procedure or by oral surgery. In certain embodiments, provided herein is a method of treating an individual who has an oral lesion comprising administering to the individual, e.g., administering to the oral lesion, a therapeutically-effective amount of double-dried ECM. In this context, "therapeutically effective amount" means an amount of double-dried ECM that acts to reduce or eliminate at least one symptom or aspect of the oral lesion. For example, the double-dried ECM can be administered in order to repair the lesion, or can be administered as a palliative, e.g., to reduce pain or inflammation caused by or associated with the oral lesion.

In certain embodiments, the double-dried ECM is administered directly into said oral lesion. In other embodiments, the double-dried ECM is administered adjacent to or at the periphery of at least a part of the oral lesion. Such administration can be, for example, by placement of a sheet of the double-dried ECM over at least a portion, or the whole of, the oral lesion. In certain embodiments, the double-dried ECM is administered to the lesion as a paste. In certain other embodiments, the double-dried ECM is administered to the lesion in the form of a spray or aerosol. In certain other embodiments, the double-dried ECM is administered to the lesion in the form of a solution, e.g., in a mouthwash. In certain other embodiments, the double-dried ECM is administered as a sheet or patch.

In another embodiment, the oral lesion is, results from, or is associated with desquamation, e.g., is a desquamating oral disorder. In another specific embodiment, the oral lesion is an aphthous ulcer. In a specific embodiment, the aphthous ulcer is caused by, or is a part of, Behçet's disease. In another specific embodiment, the aphthous ulcer is idiopathic.

In certain embodiments, said individual having said oral lesion is undergoing, or has undergone, hematopoietic stem cell therapy. In another specific embodiment, said individual is receiving, or has received, a bone marrow transplant. In certain embodiments, the oral lesion is caused by or is associated with graft-versus-host disease. In another specific embodiment, the oral lesion is caused by or associated with use of melphalan by the individual having the oral lesion. In a more specific embodiments, said hematopoietic stem cell therapy comprises partial or complete hematopoietic ablation. In a specific embodiment, said ablation comprises partial or full-body radiation.

In another embodiment, the individual having the oral lesion is undergoing, or has undergone, radiotherapy to the head or neck.

In another embodiment, the oral lesion is caused by or associated with chemotherapy, e.g., chemotherapy that has been administered to the individual to treat a tumor, blood cancer, or other type of cancer. In a specific embodiment, the oral lesion is caused by post-chemotherapy oral mucositis or chemotherapy-induced oral mucositis. In another specific embodiment, the oral lesion is, or is diagnosed as, aphthous stomatitis, *e.g.*, idiopathic aphthous stomatitis. In a specific embodiment, the oral lesion is caused by or associated with use of an mTOR (mammalian target of rapamycin) inhibitor by the individual having the oral lesion. In another specific embodiment, the oral lesion is caused by or associated with use of 5-fluorouracil by the individual having the oral lesion. In specific embodiments, in which the oral lesion is caused by or associated with chemotherapy, e.g., is caused by or associated with use of a chemotherapeutic agent, the chemotherapeutic agent is, e.g., an alkylating agent (e.g., busulfan, cisplatin, carboplatin, cyclophosphamide, dacarbazine, ifosfamide, mechlorethamine or melphalan); an anti-metabolite (e.g., 5-fluorouracil, methotrexate, gemcitabine, cytarabine, or fludarabine); antibiotics having an antitumor effect (e.g., bleomycin, dactinomycin, daunorubicin, doxorubicin, or idarubicin); or mitotic inhibitors (e.g., paclitaxel, docetaxel, etoposide, vinblastine, vincristine or vinorelbine). In another specific embodiment, development of said oral lesion in said individual, wherein said individual is receiving or has received a course of therapy, e.g., chemotherapy, has caused, or is expected to cause, a premature termination of said course of therapy. In this context, "premature termination" means termination of the course of therapy prior to what has been prescribed for said individual, partially or wholly as a result of said oral lesion.

In another embodiment, the oral lesion is caused by or associated with administration of an antibody to said individual. In certain specific embodiments, the antibody is an anti-CD20 antibody. In a more specific embodiment, the antibody is rituximab (e.g., RITUXAN®), ofatumumab (e.g., ARZERRA®), veltuzumab or ocrelizumab. In another specific embodiment, the antibody is an anti-tumor necrosis factor antibody. In more specific embodiments, the antibody is adalimumab (e.g., HUMIRA®), etanercept (e.g., ENBREL®), infliximab (e.g., REMICADE®), certolizumab pegol (e.g.,

CIMZIA®), natalizumab (e.g., TYSABRI®) or golimumab (e.g., SIMPONI®). In another specific embodiment, development of said oral lesion in said individual, wherein said individual is receiving or has received a course antibody therapy has caused a premature termination of said course of antibody therapy. In this context, "premature termination" means termination of the course of antibody therapy prior to what has been prescribed for said individual, partially or wholly as a result of said oral lesion.

In one embodiment, the individual having an oral lesion is diagnosed or evaluated using the World Health Organization Oral Toxicity (WHO-OT) score. In specific embodiments, administration of said double-dried ECM to said individual results in a reduction in the WHO-OT score from Grade 4 to Grade 3, from Grade 3 to Grade 2, from Grade 2 to Grade 1, from Grade 4 to Grade 2, from Grade 4 to Grade 1, or from Grade 3 to Grade 1, *e.g.*, within 1, 2, 3, 4, 5, 6, or 7 days post-administration.

In another embodiment, the individual having an oral lesion is diagnosed or evaluated using the National Cancer Institute Common Toxicity Criteria (NCI-CTC) for Oral Mucositis score. In specific embodiments, administration of said double-dried ECM to said individual results in a reduction in the NCI-CTC score (for stomatitis/pharyngitis [oral/pharyngeal mucositis]) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, e.g., within 1, 2, 3, 4, 5, 6, or 7 days post-administration.

In another embodiment, the individual having an oral lesion is diagnosed or evaluated using the Oral Mucositis Assessment Scale (OMAS), wherein said OMAS comprises subscores: a mean mucositis score, a weighted mean mucositis score, an extent of mucositis score, and a worst site score. In specific embodiments, administration of said double-dried ECM to said individual results in a reduction of one or more of the mean mucositis score, the weighted mean mucositis score, the extent of mucositis score, or the worst site score of at least 1, at least 2, at least 3, at least 4, or at least 5 points, *e.g.*, within 1, 2, 3, 4, 5, 6, or 7 days post-administration. *See* Sonis et al., Cancer 85(10):2103-2113 (1999).

In another embodiment, the individual having an oral lesion is diagnosed or evaluated using the Western Consortium for Cancer Nursing Research (WCCNR) score. In specific embodiments, administration of said double-dried ECM to said individual results in a reduction in the ACCRN score from Stage 3 to Stage 2, from Stage 3 to Stage 1, from Stage 3 to Stage 0, from stage 2 to Stage 1, from Stage 2 to Stage 0, or from stage 1 to Stage 0, *e.g.*, within 1, 2, 3, 4, 5,6, or 7 days post-administration.

In another embodiment, the individual having an oral lesion is diagnosed or evaluated using the Radiation Therapy Oncology Group (RTOG) score (for mucous membranes). In specific embodiments, administration of said double-dried ECM to said individual results in a reduction in the RTOG score (for mucositis) from Grade 4 to Grade 3, from Grade 3 to Grade 2, from Grade 2 to Grade 1, from grade 1 to Grade 0, from Grade 4 to Grade 2, from Grade 4 to Grade 1, from Grade 4 to Grade 0, from Grade 3 to Grade 1, from Grade 3 to Grade 0, or from Grade 2 to Grade 0, *e.g.*, within 1, 2, 3, 4, 5, 6, or 7 days post-administration.

In another embodiment, the oral lesion is caused by, or is associated with, osteonecrosis of the jaw in said individual. In certain specific embodiments, said individual is receiving, or has received, bisphosphonate therapy.

### 5.7.2. Cosmetic Applications

Human skin is a composite material of the epidermis and the dermis. The outermost layer of the epidermal layer of the skin is the stratum corneum. Beneath the stratum corneum layer is the epidermis. Below the epidermis, is the outermost layer of the dermis called the papillary dermis, followed by the reticular dermis and the subcutaneous layer.

The double-dried ECM may in certain embodiments be used for skin augmentation in a patient. In one embodiment, a method for skin augmentation in a patient comprises injecting or otherwise administering a double-dried ECM to an area of the face or body of a patient in need of augmenting, wherein the area of the face or body of the patient is augmented as compared to the area prior to administration of the collagen. "Skin augmentation" as used herein refers to any change of the natural state of a patient's (e.g., a human's) skin and related areas due to external acts or effects. Non-limiting areas of the skin that may be changed by skin augmentation include the epidermis, dermis, subcutaneous layer, fat, arrector pill muscle, hair shaft, sweat pore, sebaceous gland, or a combination thereof.

In some embodiments, methods provided herein comprise injecting or otherwise administrating double-dried ECM provided herein to a patient for the treatment of crow's feet, nasolabial folds ("smile lines"), marionette lines, glabellar folds ("frown lines"), or a combination thereof. The double-dried ECM can help fill in lines, creases, and other wrinkles and restore a smoother, more youthful-looking appearance. The double-dried ECM can be used alone or in conjunction with one or more additional injectable compositions, a resurfacing procedure, such as a laser treatment, or a recontouring procedure, such as a facelift.

In one embodiment, the double-dried ECM provided herein may also be used to augment creased or sunken areas of the face and/or to add or increase the fullness to areas of the face and body of a patient. The areas of the face and/or body requiring augmentation may be the result of, e.g., aging, trauma, disease, sickness, environmental factors, weight loss, child birth or a combination thereof. Non-limiting examples of an area of the face or body of a patient where the double-dried ECM may be injected or otherwise administered include the undereye, temple, upper malar, sub malar, chin, lip, jawline, forehead, glabella, outer brow, cheek, area between upper lip and nose, nose (such as the bridge of the nose), neck, buttocks, hips, sternum, or any other part of the face or body, or a combination thereof.

The double-dried ECM may be used to treat skin deficiencies including, but not limited to, wrinkles, depressions or other creases (e.g., frown lines, worry lines, crow's feet, marionette lines), stretch marks, internal and external scars (such as scars resulting from injury, wounds, accidents, bites, or surgery), or combinations thereof. In some embodiments, the double-dried ECM may be used for the correction of, for example, "hollow" eyes, visible vessels resulting in dark circles, as well as visible tear troughs. The double-dried ECM may also be used, for example, for correction of the undereye after aggressive removal of undereye fat pads from lower blepharoplasty or correction of the lower cheek after aggressive buccal fat extraction or natural loss. In one embodiment, the double-dried ECM may be used to correct the results of rhinoplasty, skin graft or other surgically-induced irregularities, such as indentations resulting from liposuction. In other embodiments, the double-dried ECM may be used for the correction of facial or body scars (e.g., wound, chicken pox, or acne scars). In some embodiments, the double-dried ECM is injected or otherwise administered into a patient for facial reshaping. Facial reshaping may be completed in a patient with neck laxity, or having a gaunt face, long face, bottom-heavy face, asymmetrical face, a chubby face, or having a face with localized fat atrophy, a midface retrusion, sunken eyes, and/or any combinations thereof.

In one embodiment, the double-dried ECM can be injected or otherwise administered to a patient for the treatment a skin deficiency, such as skin deficiency caused by a disease or illness, such as cancer or acne. The deficiency can be the direct or indirect result of the disease or illness. For example, a skin deficiency can by caused by a disease or illness or can be caused by a treatment of a disease or illness.

### 5.7.3. Non-Cosmetic Applications

### 5.7.3.1. Void Filling

The double-dried ECM may also be sued for sealing, filling and/or otherwise treating a void within the body of a patient. In some embodiments, the double-dried ECM injected or otherwise administered to a patient to fill a void within the body of the patient. For example, the double-dried ECM can be administered to the patient in the area where the void is located. The term "void" is intended to encompass any undesirable hollow space created by aging, disease, surgery, congenital abnormalities, or a combination thereof. For example, a void may be created following the surgical removal of a tumor or other mass from the body of a patient. Non-limiting examples of voids which may be filled with the double-dried ECM include a fissure, fistula, divercula, aneurysm, cyst, lesion, or any other undesirable hollow space in any organ or tissue of the patient's body.

In some embodiments, the double-dried ECM may be used to fill, seal and/or otherwise treat, in whole or in part, a crevice, fissure, or fistula within a tissue, organ, or other structure of the body (e.g., a blood vessel), or junctures between adjacent tissues, organs or structures, to prevent the leakage of biological fluids, such as blood, urine, or other biological fluids. For example, the double-dried ECM can be injected, implanted, threaded into, or otherwise administered into fistula between viscera, or into the opening or orifice from a viscus to the exterior of the patient's body. The double-dried ECM can be used to fill a void or other defect formed by these pathological states and stimulate fibroblast infiltration, healing, and ingrowth of tissue.

In one embodiment, a method used to fill, seal, and/or otherwise treat a fistula in a patient in need of treatment, said method comprising injecting or otherwise administering to the patient the double-dried ECM. The double-dried ECM can be administered to the patient by injection through a needle into one of the fistular orifices and filling most or all of the branches of the orifice. Alternatively, strings or rods of the collagens can be threaded into the fistulae lesions through an orifice, or the collagen can be introduced into the patient with a catheter. Various types of fistulae can be filled, sealed and/or otherwise treated using the double-dried ECM, such as anal, arteriovenous, bladder, carotid-cavernous, external, gastric, intestinal, parietal, salivary, vaginal, and anorectal fistulae, or a combination thereof.

In one embodiment, the double-dried ECM is used to fill, seal and/or otherwise treat a diverticulum in a patient in need of treatment, said method comprising injecting or otherwise administering to the patient the double-dried ECM. Diverticulae are abnormal physiological structures that are pouches or sac openings from a tubular or saccular organ, such as the intestine, the bladder, and the like, and can be filled or augmented using the double-dried ECM.

In another embodiment, the double-dried ECM is used to fill, seal and/or otherwise treat a cyst in a patient in need of treatment, by injecting or otherwise administering to the patient the double-dried ECM. In some embodiments, the cyst is a pseudocyst, which has an accumulation of, e.g., fluid but does not comprise an epithelial or other membranous lining. Additional non-limiting examples of cysts that can be filled, sealed and/or otherwise treated include sebaceous, dermoid, bone, or serous cysts, or a combination thereof.

In another embodiment, the double-dried ECM may be injected or otherwise administered to fill in whole, or in part, any void created as a result of surgical, chemical or biological removal of unnecessary or undesirable growths, fluids, cells, or tissues from a patient. The double-dried ECM can be locally injected or otherwise administered at the site of the void so as to augment the remaining and surrounding tissue, aid in the healing process, and minimize the risk of infection. This augmentation is especially useful for void sites created after tumor excision, such as after breast cancer surgery, surgery for removal of tumorous connective tissue, bone tissues or cartilage tissue, and the like.

The double-dried ECM may also be injected or otherwise administered not directly into the body, but extracorporeally into organs, components of organs, or tissues prior to the inclusion of said tissues, organs or components of organs into the body.

## 5.7.4. Tissue Bulking

In another embodiment, the double-dried ECM may be used for tissue bulking. "Tissue bulking" as used herein refers to any change of the natural state of a patient's (e.g., a human's) non-dermal soft tissues due to external acts or effects. The tissues encompassed herein include, but not limited to, muscle tissues, connective tissues, fats, and, nerve tissues. The tissues may be part of many organs or body parts including, but not limited to, the sphincter, the bladder sphincter and urethra.

### 5.7.4.1. Urinary Incontinence

Urinary incontinence (including stress urinary incontinence) is the sudden leakage of urine that occurs with activities that result in an increase in intra-abdominal pressure, such as coughing, sneezing, laughing or exercise. During these activities, intra-abdominal pressure rises transiently above urethral resistance, thus resulting in a sudden, usually small, amount of urinary leakage. Stress incontinence is generally a bladder storage problem in which the strength of the urethral sphincter is diminished, and the sphincter is not able to prevent urine flow when there is increased pressure from the abdomen. Urinary incontinence may occur as a result of weakened pelvic muscles that support the bladder and urethra, or because of malfunction of the urethral sphincter. For example, prior trauma to the urethral area, neurological injury, and some medications may weaken the urethra. Urinary incontinence is most commonly seen in women after menopause, pelvic surgery, or childbearing, e.g., after multiple pregnancies and vaginal childbirths, or who have pelvic prolapse (protrusion of the bladder, urethra, or rectal wall into the vaginal space), with cystocele, cystourethrocele, or rectocele), and is usually related to a loss of anterior vaginal support. In men, urinary incontinence may be observed after prostatic surgery, most commonly radical prostatectomy, in which there may be injury to the external urethral sphincter.

The double-dried ECM may be used for managing or treating urinary incontinence, or a symptom or condition resulting therefrom, by injecting or otherwise administering the double-dried ECM to a patient in need thereof, wherein, e.g., the patient's sphincter tissue is augmented and continence is improved or restored in the patient. The double-dried ECM can be injected or otherwise administered periurethrally to increase tissue bulk around the urethra for the management and/or treatment of urinary incontinence. Improvement in stress incontinence can achieved by increasing the tissue bulk and thereby increasing resistance to the outflow of urine.

In some embodiments, the double-dried ECM is injected or otherwise administered to a patient in the area around the urethra, for example, to close a hole in the urethra through which urine leaks out or to build up the thickness of the wall of the urethra so it seals tightly when urine is being held back.

In another embodiment, the double-dried ECM is injected or otherwise administered to a patient around the urethra just outside the muscle of the urethra at the bladder outlet. Injecting the bulking material can be done through the skin, through the urethra, or, in women, through the vagina.

When needles are used for injection of the double-dried ECM, needle placement can be guided by the use of a cystoscope inserted into the urethra. Urethral bulking procedures can be performed under local anesthesia, but some patients may require a general, regional or spinal anesthesia. A local anesthetic can be used so the patient can stand up after an injection, and it can be determined whether continence has been achieved. If continence has not been restored, one or more subsequent injection(s) can be administered to the patient. The procedure may need to be repeated after a few months to achieve bladder control. The collagen injection helps control the urine leakage by bulking up the area around the urethra, thus compressing the sphincter.

### 5.7.4.2. Vesicoureteral Reflux

Vesicoureteral reflux (VUR) (or urinary reflux) is characterized by the retrograde flow of urine from the bladder to the kidneys. Untreated VUR may cause devastating long-term effects on renal function and overall patient health. A patient with VUR has an increased risk of developing a urinary tract infection, renal scarring, pyelonephritis, hypertension, and progressive renal failure.

The double-dried ECM may, in certain embodiments, be injected or otherwise administered to a patient in need thereof double-dried ECM, wherein the ureteral wall of the patient is augmented, and the symptoms of VUR are reduced or eliminated. The composition can be injected (e.g., a subtrigonal injection) or otherwise administered, such as under endoscopic guidance, into the detrusor backing under the ureteral orifice using any method known to those in the art.

### 5.7.4.3. Gastroesophageal Reflux Disease

Gastroesophageal reflux disease (GERD) is a disorder that usually occurs because the lower esophageal sphincter (LES)--the muscular valve where the esophagus joins the stomach--does not close properly, relaxes or weakens, and stomach contents leak back, or reflux, into the esophagus. When the stomach acid, or occasionally bile salts, comes into contact with the esophagus it causes the burning sensation of heartburn that most of us occasionally feel. When refluxed stomach acid touches the lining of the esophagus, it causes a burning sensation in the chest or throat (heartburn), and the fluid may be tasted in the back of the mouth (acid indigestion). Over time, the reflux of stomach acid damages the tissue lining the esophagus, causing inflammation and pain. In adults, long-lasting, untreated GERD can lead to permanent damage of the esophagus and sometimes even cancer. Anyone, including infants, children, and pregnant women, can have GERD.

The double-dried ECM may be used in the management or treatment of GERD, or a symptom or condition resulting therefrom, injecting or other administering to a patient in need thereof double-dried ECM, wherein the LES of the patient is augmented, and the symptoms of GERD are reduced or eliminated. In some embodiments, the double-dried ECM is administered under endoscopic guidance into the esophageal wall at the level of the esophagogastric junction. Intended to impede reflux, the bulking effect results from a combination of the retained material and consequent tissue response. The double-dried ECM can be injected through standard or large-bore (e.g., large gauge) injection needles.

### 5.7.4.4. Vocal Cords and Larynx

The double-dried ECM may be used in the management or treatment of a disease, disorder (such as a neurological disorder), or other abnormality that affects the one or both vocal cords (folds) and/or the larynx (voice box). Non-limiting examples of such diseases, disorders or other abnormalities of the larynx an vocal cords are glottic incompetence, unilateral vocal cord paralysis, bilateral vocal cord paralysis, paralytic dysphonia, nonparalytic dysphonia, spasmodic dysphonia or a combination thereof. In other embodiments, the double-dried ECM may also be used to manage or treat diseases, disorders or other abnormalities that result in the vocal cords closing improperly, such as an incomplete paralysis of the vocal cord ("paresis"), generally weakened vocal cords, for instance, with old age ("presbylaryngis"), and/or scarring of the vocal cords (e.g., from previous surgery or radiotherapy).

The double-dried ECM can be used to provide support or bulk to a vocal fold in a patient that lacks the bulk (such as in vocal fold bowing or atrophy) or the mobility (such as in paralysis) the vocal cord once had. In some embodiments, the vocal cords and/or other soft tissues of the larynx can be augmented with the double-dried ECM, either alone or in combination with other treatments or medications. In one embodiment, the double-dried ECM augments or adds bulk to one (or both) vocal folds so that it can make contact with the other vocal fold.

Any one of a number of procedures well known to those in the art may be used for administration of the double-dried ECM to a vocal cord(s) or larynx of a patient. In some embodiments, a curved needle is used to inject double-dried ECM through the mouth of the patient. In other embodiments, a needle (such as a higher gauge, short needle) may be used to inject the double-dried ECM directly through the skin and the Adam's apple of the patient. The double-dried ECM can be administered to a patient while monitoring the vocal folds of the patient with a laryngoscope on a video monitor.

### 5.7.4.5. Glottic Incompetence

In one embodiment, the double-dried ECM can be used for the management or treatment of glottic incompetence. Percutaneous laryngeal collagen augmentation can occur by injection the double-dried ECM using a needle into the vocal cords of a patient using methods known in the art. In some cases, the patient has hypophonia and/or glottic incompetence that affects the voice function of the larynx, increased muscle rigidity, and decreased ability for movement of the thyroarytenoid muscle. In another embodiment, the hypophonia is a result of Parkinson's Disease. In one embodiment, the double-dried ECM can be used for the management or treatment of glottic incompetence in a patient in need thereof by injecting or otherwise administering double-dried ECM to the vocal cords of a patient, wherein the injection augments the vocal cord and improves glottic closure, such that glottic incompetence is reduced or eliminated in the patient. The patient may or may not have mobile vocal cords prior to administration of double-dried ECM.

### 5.7.4.6. Dysphonia

Dysphonia is any impairment of the voice or difficulty speaking. Dysphonia may or may not be associated with laryngeal or vocal cord paralysis. Provided herein are methods for the management or treatment of dysphonia, such as paralytic dysphonia, non-paralytic dysphonia or spasmodic dysphonia. In one embodiment, a method for managing or treating dysphonia in a patient comprises injecting or administering double-dried ECM to the patient in need thereof, wherein dysphonia is improved in patient as compared to prior to administration of the collagen composition. In some cases, laryngeal collagen injection permits further medialization of one or both vocal folds by small increments to improve phonation in conjunction with or after medialization thyroplasty.

### 5.7.4.7. Vocal Cord Paralysis

The vocal cord is essentially a muscle covered with a mucous membrane. When the muscle is no longer connected to a nerve, the muscle atrophies. Therefore, typical paralyzed vocal cords are be small in size and bowed. Additionally, depending on the type of paralysis, the vocal cord may or may not be moving close enough to the middle for the other vocal cord to come touch it. When vocal cords are incapable of meeting, it is difficult for the patient to make a sound (or at least a loud sound). Thus, provided herein are methods to augment or bulk an atrophied vocal cord in a patient with vocal cord paralysis, wherein the ability of the vocal cords to come together is improved.

Unilateral vocal fold paralysis is immobility of one vocal fold, typically because of nerve dysfunction, and often the larynx is unable to completely close. The recurrent laryngeal nerve is the main nerve that accounts for most of the movement of each vocal fold, and can be damaged, e.g., by various diseases, certain surgeries or viral infection. In some embodiments, vocal cord paralysis in a patient is a symptom or result of thyroid cancer, lung cancer, tuberculosis or sarcoid (or anything that causes lymph nodes to enlarge in the chest), stroke, a neurologic diseases (e.g., Charcot-Marie-Tooth, Shy-Drager, and multisystem atrophy).

Bilateral vocal cord paralysis is the immobility (usually close to the midline) of both vocal folds. In some embodiments, bilateral vocal fold paralysis in a patient is a symptom or result of, e.g., stroke or other neurologic condition (such as Arnold-Chiari malformation), thyroid cancer, surgery (such as major brain surgery) or thyroidectomy.

The double-dried ECM may be used in the management or treatment of vocal cord paralysis. In one embodiment, the double-dried ECM is used to manage or treat unilateral or bilateral vocal cord paralysis, or a symptom related thereto in a patient, by injecting or otherwise administering the double-dried ECM to the patient, wherein vocal fold closure is improved in the patient. In one embodiment, the double-dried ECM augments or adds bulk to one (or both) paralyzed vocal fold so that it can make contact with the other vocal fold. The injection of double-dried ECM to the patient in need thereof can be through the patient's mouth or directly through the skin and Adam's apple.

### 5.7.4.8. Drug Delivery

The double-dried ECM can be used as a drug delivery vehicle for controlled delivery of a drug, e.g., a therapeutic agent. In some embodiments the collagen composition delivers the one or more therapeutic agents to a subject, e.g. a human. The therapeutic agents encompassed within the scope of the disclosure are proteins, peptides, polysaccharides, polysaccharide conjugates, genetic based vaccines, live attenuated vaccines, whole cells. A non-limiting example of drugs is antibiotics, anti-cancer agents, anti-bacterial agents, anti-viral agents; vaccines; anesthetics; analgesics; anti-asthmatic agents; anti-inflammatory agents; antidepressants; anti-arthritic agents; anti-diabetic agents; anti-psychotics; central nervous system stimulants; hormones; immunosuppressants; muscle relaxants; prostaglandins.

The double-dried ECM may be used as a delivery vehicle for controlled delivery of one or more small molecules to a subject, e.g. a human. In some embodiments the collagen composition delivers the one or more small molecules to a subject, e.g. a human. As used herein, the term "small molecule," and analogous terms, include, but are not limited to, peptides, peptidomimetics, amino acids, amino acid analogs, polynucleotides, polynucleotide analogs, nucleotides, nucleotide analogs, organic or inorganic compounds (i.e., including heteroorganic and organometallic compounds) having a molecular weight less than about 10,000 grams per mole, organic or inorganic compounds having a molecular weight less than about 5,000 grams per mole, organic or inorganic compounds having a molecular weight less than about 1,000 grams per mole, organic or inorganic compounds having a molecular weight less than about 500 grams per mole, organic or inorganic compounds having a molecular weight less than about 100 grams per mole, and salts, esters, and other pharmaceutically acceptable forms of such compounds. Salts, esters, and other pharmaceutically acceptable forms of such compounds are also encompassed.

In certain embodiments, the double-dried ECM as a vehicle for drug delivery results in enhanced absorption of the drug; improved pharmacokinetic profile, and systemic distribution of the drug relative to the other drug delivery systems known in the art. By improved pharmacokinetics it is meant that an enhancement of pharmacokinetic profile is achieved as measured, for example, by standard pharmacokinetic parameters such as time to achieve maximal plasma concentration (Tmax); magnitude of maximal plasma concentration (Cmax); time to elicit a detectable blood or plasma concentration (Tlag). By enhanced absorption it is meant that absorption of the drug is improved as measured by such parameters. The measurement of pharmacokinetic parameters are routinely performed in the art.

In some embodiments, the double-dried ECM further comprises one or more biomolecules, e.g., therapeutic agents, including but not limited to, antibiotics, hormones, growth factors, anti-tumor agents, anti-fungal agents, anti-viral agents, pain medications, anti-histamines, anti-inflammatory agents, anti-infectives, wound healing agents, wound sealants, cellular attractants and scaffolding reagents, enzymes, receptor antagonists or agonists, hormones, growth factors, autogenous bone marrow or other cell types, antibiotics, antimicrobial agents, and antibodies, and the like, or combinations thereof. In a specific example, the double-dried ECM may be impregnated with one or more growth factors, for example, fibroblast growth factor, epithelial growth factor, etc. The double-dried ECM may also be impregnated with one or more small molecules, including but not limited to small organic molecules such as specific inhibitors of particular biochemical processes e.g., membrane receptor inhibitors, hormones, kinase inhibitors, growth inhibitors, anti-cancer drugs, antibiotics, etc.

In some embodiments, the double-dried ECM is impregnated with a biomolecule, during production or prior to injection depending on its intended use. In some embodiments, the double-dried ECM comprises a one or more interferons (.alpha.-IFN, .beta.-IFN, .gamma.-IFN), colony stimulating factors (CSF), granulocyte colony stimulating factors (GCSF), granulocyte-macrophage colony stimulating factors (GM-CSF), tumor necrosis factors (TNF), nerve growth factors (NGF), platelet derived growth factors (PDGF), lymphotoxins, epidermal growth factors (EGF), fibroblast growth factors (FGF), vascular endothelial cell growth factors, erythropoietin, transforming growth factors (TGF), oncostatin M, interleukins (IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, etc.), members of the families thereof, or combinations thereof. In some embodiments, the double-dried ECM comprises biologically active analogs, fragments, or derivatives of such growth factor or other biomolecule.

Particular active agents for use in methods provided herein include growth factors, such as transforming growth factors (TGFs), fibroblast growth factors (FGFs), platelet derived growth factors (PDGFs), epidermal growth factors (EGFs), connective tissue activated peptides (CTAPs), osteogenic factors, and biologically active analogs, fragments, and derivatives of such growth factors. Members of the transforming growth factor (TGF) supergene family, which are multifunctional regulatory proteins, are useful. Members of the TGF supergene family include the beta transforming growth factors (for example, TGF-$\beta$1, TGF-$\beta$2, TGF-$\beta$3); bone morphogenetic proteins (for example, BMP-1, BMP-2, BMP-3, BMP-4, BMP-5, BMP-6, BMP-7, BMP-8, BMP-9); heparin-binding growth factors (for example, fibroblast growth factor (FGF), epidermal growth factor (EGF), platelet-derived growth factor (PDGF), insulin-like growth factor (IGF)); inhibins (for example, inhibin A, inhibin B); growth differentiating factors (for example, GDF-1); and activins (for example, activin A, activin B, activin AB).

### 5.7.5. Wounds and Burns

The double-dried ECM is has enhanced clinical utility as a wound dressing, for augmenting or replacing hard and/or soft tissue repair, as compared to other biomaterials known in the art, e.g., those described in U.S. Pat. Nos. 3,157,524; 4,320,201; 3,800,792; 4,837,285; 5,116,620, due in part to its physical properties. The double-dried ECM because it retains collagen's native quaternary structure provides improved tissue in-growth through cell migration into the interstices of the collagen matrix. The double-dried ECM allows cells to attach and grow into the collagen matrix, and to synthesize their own macromolecules. The cells thereby produce a new matrix which allows for the growth of new tissue. Such cell development is not observed on other known forms of collagen such as fibers, fleeces and soluble collagen.

In some embodiments, the double-dried ECM may be used to treat a wound by placing the composition directly over the skin of the subject, i.e., on the stratum corneum, on the site of the wound, so that the wound is covered, for example, using an adhesive tape. In other embodiments, the method encompasses treating a wound using the composition as an implant, e.g., as a subcutaneous implant.

The rate of wound healing can be enhanced by the addition of a macromolecule capable of promoting tissue ingrowth to the double-dried ECM. Such macromolecules include but are not limited to hyaluronic acid, fibronectin, laminin, and proteoglycans (See, e.g., Doillon et al. (1987) Biomaterials 8:195 200; and Doillon and Silver (1986) Biomaterials 7:3 8).

In some embodiments, the double-dried ECM is used for the management of wounds including but not limited to partial and full-thickness wounds, pressure ulcers, pressure ulcers, venous ulcers, diabetic ulcers, chronic vascular ulcers, tunneled/undermined wounds, surgical wounds (e.g., donor sites/grafts, post-Moh-s surgery, post-laser surgery, podiatric, wound dehiscence), trauma wounds (e.g., abrasions, lacerations, second degree burns, and skin tears) and draining wounds. In certain embodiments, the double-dried ECM is intended for one-time use.

The double-dried ECM may incorporate pharmacologically active agents including but not limited to platelet-derived growth factor, insulin-like growth factor, epidermal growth factor, transforming growth factor beta, angiogenesis factor, antibiotics, antifungal agents, spermicidal agents, hormones, enzymes, enzyme inhibitors in the double-dried ECM as described above for delivery to the skin, and any biomolecule described above. In certain embodiments, the pharmacologically active agents are provided in a physiologically effective amount.

The double-dried ECM is particularly useful for the treatment of wound infections, e.g., wound infections followed by a breakdown of surgical or traumatic wounds. In a particular embodiment, the collagen composition is impregnated with a therapeutically effective amount of an agent useful in the treatment of a wound infection, including but not limited to, an antibiotic, anti-microbial agent, and an anti-bacterial agent. The double-dried ECM has clinical and therapeutic utility in the treatment of wound infections from any microorganism known in the art, e.g., microorganisms that infect wounds originating from within the human body, which is a known reservoir for pathogenic organisms, or from environmental origin. A non-limiting example of the microorganisms, the growth of which in wounds may be reduced or prevented by the methods and compositions provided herein are S. aureus, St. epidermis, beta haemolytic Streptococci, E. coli, Klebsiella and Pseudomonas species, and among the anaerobic bacteria, the Clostridium welchii or tartium, which are the cause of gas gangrene, mainly in deep traumatic wounds.

In other embodiments, the double-dried ECM can be used for wound treatment, including but not limited to epidermal wounds, skin wounds, chronic wounds, acute wounds, external wounds, internal wounds (e.g., the collagen composition may be wrapped around an anastosmosis site during surgery to prevent leakage of blood from suture lines, and to prevent the body from forming adhesions to the suture material), congenital wounds (e.g., dystrophic epidermolysis bullosa). In particular, the double-dried ECM has enhanced utility in the treatment of pressure ulcers (e.g., decubitus ulcers). Pressure ulcers occur frequently with patients subject to prolonged bedrest, e.g., quadriplegics and paraplegics who suffer skin loss due to the effects of localized pressure. The resulting pressure sores exhibit dermal erosion and loss of the epidermis and skin appendages. In yet other more specific embodiments, the double-dried ECM is used for the management of wounds including but not limited to partial and full-thickness wounds, pressure ulcers, venous ulcers, diabetic ulcers, chronic vascular ulcers, tunneled/undermined wounds, surgical wounds (e.g., donor sites/grafts, post-Moh's surgery, post-laser surgery, podiatric, wound dehiscence), trauma wound (e.g., abrasions, lacerations, second-degree burns, and skin tears) and draining wounds.

The double-dried ECM may also be used in the treatment of burns, including but not limited to first-degree burns, second-degree burns (partial thickness burns), third degree burns (full thickness burns), infection of burn wounds, infection of excised and unexcised burn wounds, infection of grafted wound, infection of donor site, loss of epithelium from a previously grafted or healed burn wound or skin graft donor site, and burn wound impetigo.

### 5.7.6. Dental

The double-dried ECM has utility in dentistry, e.g., periodontal surgery, guided tissue regeneration for regeneration of periodontal tissue, guided bone regeneration, and root coverage. The methods encompass the use of the double-dried ECM to promote regeneration of periodontal intrabony defects, including but not limited to matched bilateral periodontol defects, interdental intrabony defects, deep 3-wall intrabony defects, 2-wall intrabony defects, and intrabony defects 2 and 3. The double-dried ECM is expected to have an enhanced therapeutic utility and enhanced clinical parameters for the treatment of periodontal intrabony defects relative to other techniques known in the art, e.g., use of cross-linked collagen membranes such as those disclosed in Quteish et al., 1992, J. Clin. Periodontol. 19(7): 476-84; Chung et al., 1990, J. Periodontol. 61(12): 732-6; Mattson et al., 1995, J. Periodontol. 66(7): 635-45; Benque et al., 1997, J. Clin. Periodontol. 24(8): 544-9; Mattson et al., 1999, J. Periodontol. 70(5): 510-7). Examples of clinical parameters that are improved using the double-dried ECM include but are not limited to plaque and gingival index scorings, probing pocket depth, probing attachment depth, and classification of furcation involvement and bony defect, which are known to one skilled in the art.

Also provided herein is the use of the double-dried ECM in treating class II furcation defects including but not limited to bilateral defects, paired buccal Class II mandibular molar furcation defects, and bilateral mandibular furcation defect. The double-dried ECM is expected to have an enhanced therapeutic and clinical utility relative to the collagen membranes used in the art for the treatment of class II furcation defects, such as those disclosed in Paul et al., 1992, Int. J. Periodontics Restorative Dent. 12: 123-31; Wang et al., 1994, J. Periodontol. 65: 1029-36; Blumenthal, 1993, J. Periodontol. 64: 925-33; Black et al., 1994, J. Periodontol. 54: 598-604; Yukna et al., 1995, J. Periodontol. 67: 650-7).

The double-dried ECM may further be used in root coverage procedures. The composition is expected to have an enhanced clinical utility in root coverage as compared to collagen membranes in the art traditionally used for root coverage such as those disclosed in Shieh et al., 1997 J. Periodontol., 68: 770-8; Zahedi et al., 1998 J. Periodontol. 69: 975-81; Ozcan et al., 1997 J. Marmara Univ. Dent. Fa. 2: 588-98; Wang et al., 1997 J. Dent. Res. 78 (Spec Issue): 119 (Abstr. 106).

Further provided herein is use of the double-dried ECM in a subject with a periodontal disease including but not limited to, periodontitis and gingivitis. The composition also has clinical utility as an adjunct to scaling and root planing procedures. The double-dried ECM may be used to treat a subject with a periodontal disease by, e.g., inserting the composition, which can be impregnated with an antibiotic such as chlorhexidine gluconate, into one or more periodontal pockets in the subject, e.g., greater than or equal to 5 mm.

The double-dried ECM for use in dentistry may be impregnated with one or more biomolecules depending on the type of dental disorder being treated. Any biomolecule known in the art for the treatment of dental disorders is encompassed in the methods and compositions provided herein. In a specific embodiment, the collagen composition used in the treatment of a dental disorder associated with an infection may be impregnated with one or more antibiotics, including but not limited to doxocyclin, tetracycline, chlorhexidine gluconate, and minocycline.

### 5.7.7. Other Uses

The double-dried ECM may also be used as a post-operative adhesion barrier in the ovaries or uterine horns. The composition may also be used as an adhesion barrier in the brain (e.g., in the prevention of meningio-cerebral adhesion), or as a dural replacement, e.g., as a flat sheet to replace part of all of the dura after, e.g., brain surgery, injury or the like. The double-dried ECM may also be used for restoring the subdural space that separates the pachymeninx and leptomeninx. Generally, the double-dried ECM may be used as a wrapping on injured internal organs, for example, the spleen, or as a sheet adhered to the lung to control post-operative leakage. The double-dried ECM may also be used to support surgical treatment of tympanic membrane grafts (in tympanic perforations), or as a lining in mastoid cavities. The composition may also be used as a lining tissue in neovaginoplasty. In cardiovascular surgery, the double-dried ECM may be used as a pericardial closure material. The double-dried ECM may also be used in the completion of anastomosis in vasovasostomy.

## 6. EXAMPLES

### 6.1. Example 1: Generation of Double-Dried ECM

This example illustrates generation of double-dried ECM from placentas.

### 6.1.1. Initial preparation and washing

Placentas were obtained from a normal, full-term delivery and quarantined frozen until the completion of all donor screening tests. Upon being released for processing, the placenta was thawed at room temperature (22°C) for about 24 hrs. The placenta was removed from the container, placed on a sterile tray followed by removal of the amnion, chorionic skirt and umbilical cord. The placenta was cleaned to remove excess blood and blood clots and then cut into approximately 2 X 2 cm segments. The cut placental material was transferred into containers with 1M sterile sodium chloride (1.5L total volume per container). The suspended segments were then homogenized for about 120 seconds using an Omni Mixer homogenizer.

The homogenized tissue was transferred into processing bags and additional 1M sodium chloride sterile solution was added into the bags to make a total volume of 9.2L per single placenta (one batch). The tissue was washed 3 times with a 1 M NaCl sterile solution as follows: the bag with suspended tissue was agitated on a shaker for 3-5 minutes, and the tissue was allowed to settle. Blood and debris were separated from the tissue by removing 6.2L of supernatant via gravity drainage. The washed tissue was allowed to mix in 1M NaCl within the processing bags on an orbital shaker overnight (18-26 hours) at room temperature (22°C). At the end of the overnight period, the tissue was washed 4 times with 6.2 L of sterile water in the manner described above. The washed tissue was allowed to mix in water within the processing bags on an orbital shaker overnight (18-26 hours) at room temperature (22°C).

At the end of the second overnight period, the tissue was washed once with 6.2 L of sterile water in the manner described above. 6.2 L of sterile 3% sodium deoxycholate solution was then added into the bag to make a 2% final concentration of sodium deoxycholate. The tissue was allowed to mix in the sodium deoxycholate solution within the processing bags on an orbital shaker for approximately 72 hrs at room temperature (22°C). At the end of the 72 hr sodium dioxycholate treatment, the tissue was washed five times with 6.2 L of sterile water in the manner described above. After the last fill with sterile water, the pH inside the bag was adjusted to approximately pH 12 with NaOH, and the tissue was allowed to mix in water at this pH on an orbital shaker for about 30 minutes at room temperature (22°C). At the end of the high pH exposure, pH within the bags was adjusted to between pH 5.0 and pH 7.5. Supernatant (6.2L) was removed from the bags and the resulting extracellular matrix (ECM) suspension (approximately 3.0L) was collected into multiple sterile centrifugation bottles. The ECM was then pelleted by centrifugation, followed by decantation of the supernatant. The ECM was washed once with sterile water within the same bottles, centrifuged and the supernatant was again decanted, leaving a washed ECM paste.

### 6.1.2. Drying steps

The ECM paste obtained in Section 6.1.1 was re-suspended in sterile buffer and then transferred into molds. Molds with the ECM suspension were loaded into a controlled rate freezer, and the ECM was frozen at -80°C. Frozen ECM was then stored at -80°C for up to 60 days.

Molds with frozen ECM were loaded into a lyophilizer, where they were freeze-dried using established parameters for up to 72 hours to produce dehydrated ECM (DHCM).

The lyophilized DHCM product (in this example, a 3.5 X 3.5 inch piece) was removed from the molds, packaged, and labeled. Packaged, lyophilized DHCM was optionally stored at room temperature for up to 60 days in unsterilized form (packaged, sterilized DHCM is expected to last up to 5 years).

Non-sterile or sterilized DHCM 3.5"x3.5" wafers were subsequently removed from the pouches and positioned on a sterile Tyvek sheet on a heated vacuum drier platform. The DHCM was completely re-hydrated with buffer, covered with a protective layer of sterile Tyvek, and vacuum-heat-dried at temperatures between 40°C and 80°C for 4-72 hrs.

Upon completion of the drying cycle, DHCM 3.5"x3.5" sheets were removed from the dryer, packaged and labeled.

DHCM 3.5"x3.5" sheets were optionally sterilized by radiation.

### 6.2. Example 2: Culture of Cells on Double-Dried ECM

This Example demonstrates that double-dried ECM provides a suitable substrate for cell attachment and proliferation.

### 6.2.1 Material and Methods

**Human placental ECM (hpECM):** Frozen placenta was thawed, then washed and ground, then submitted to osmotic shock by washes with sterile water, followed by a mild detergent treatment (2% deoxycholic acid) which allowed for complete decellularization. After multiple washes with sterile water, the resulting ECM particles were blended into phosphate buffer to obtain an ECM paste.

**Cell Culture:** Amnion Derived Adherent Cells (AMDACs), as described in U.S. Patent No. 8,367,409, were isolated from human amnion, following the birth of normal full term infants and expended in medium containing 2% fetal bovine serum (FBS) and a battery of growth factors, see U.S. Patent No. 8,367,409.

hpECM sheets were cut into an appropriate size and shape for culture, and pre-hydrated in AMDAC culture medium for 1 hour at 37 °C prior to cell seeding. AMDACs were seeded (12,500 cells/cm$^2$) onto the pre-hydrated hpECM sheets and incubated at 37°C, 5% $CO_2$ for up to 10 days. Culture medium was changed every 2 days, and cultures were analyzed at day 1, 3 and 6 of culture.

**Cell Adhesion and proliferation:** AMDAC cultures on hpECM were stained with Calcein AM, and cell adhesion on the hpECM and cell morphology were monitored microscopically. Cell proliferation was evaluated by an MTS metabolic assay (Promega, Madison, WI).

**Cell Migration Assay:** AMDAC cultures were established for 24h in AMDAC medium in the lower compartment of 24-well transwells on hpECM or directly on the plate. Other wells contained hpECM or medium only controls. Keratinocytes were cultured (30,000 cells/transwell) in the upper compartment of each transwell (placed in clean wells) in keratinocyte medium. The cells were allowed to attach for 2h, after which all culture media were removed and the cells gently rinsed with PBS. Transwells containing keratinocytes were placed on wells containing either AMDAC cultures or the controls. Serum free keratinocyte medium supplemented with 0.1% BSA was added to both compartments of each well. After 5h of migration time, keratinocytes were carefully removed from the upper side of the transwell, and cells that had migrated to the lower side were fixed with PFA 4% n/o, washed with PBS and stained with DAPI. Nuclei were observed under fluorescence microscope and the number of migrated keratinocytes was recorded.

**Cytokine Secretion Analysis:** Cultures were serum deprived and supplemented with 1% BSA 24h prior to media collection. Media samples from the hpECM-AMDACs cultures as well as AMDACs alone and hpECM alone were collected on ice. Samples were centrifuged at 4°C for 10 minutes at 14,000rpm to eliminate cell debris, and supernatants were harvested at -80°C until use. Supernatants were analyzed for the presence of 15 cytokines relevant in wound healing (b-FGF, VEGF, PDGF, KGF, TGF-b, EGF, GM-SCF, MIP-1a, MIP-1b, MCP-1, TNF-a, IL-1, IL-6, IL-8, IL-10) using simplex ELISA kits (R&D Systems, Minneapolis, MN; eBioscience, San Diego, CA) and multiplex cytokine antibody array (Biosource, Camarillo, CA).

**Soluble Fibronectin Analysis Using ELISA method:** Fibronectin concentrations were determined in AMDAC-hpECM culture media harvested at different time points using a sandwich ELISA (eBioscience, San Diego, CA).

**Immunohistochemical Analysis:** hpECM alone and hpECM-AMDACs cultures were fixed in 4% paraformaldehyde (PFA) overnight. After PBS washes, fixed samples were infiltrated, paraffin embedded and sectioned. Sections were immunostained using rabbit antihuman fibronectin and laminin polyclonal antibodies (Abcam, Cambridge, MA). Anti-rabbit RFP-conjugated secondary antibody (Abcam) was used to detect fibronection and anti-rabbit FITC-conjugated secondary antibody was used to detect laminin. Sections were counter stained with DAPI. Fluorescence microscopic images were recorded and analyzed on EVOS digital microscope (AMG, Bothell, WA).

<u>AMDAC - HUVEC Co-Culture:</u> AMDAC and human umbilical vein endothelial cells (HUVEC) were labeled by transduction with GFP and RFP respectively using Smart Vector-2 Lentivirus particles (Dharmacon). AMDAC-GFP cultures were pre-established in AMDAC medium on hpECM at 12,500 cells/cm$^2$ 24h prior to HUVEC-RFP seeding at 8,000 cells/cm$^2$. Co-cultures were allowed to further grow in commercially available HUVEC medium (Invitrogen) and observed under the microscope (AMG) 3 days later.

### 6.2.2 Results

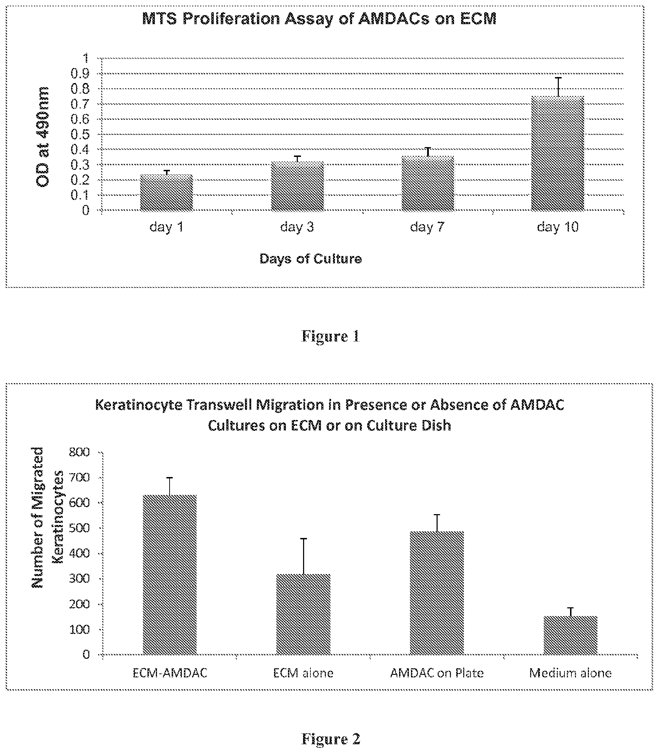

<u>Cell Adhesion and Proliferation:</u> AMDACs exhibited quick and homogenous attachment on hpECM surface and adopted stretched morphologies; cell appeared elongated and polarized. Microscopic observations of cultures at different time points clearly indicated that cells were proliferating on hpECM. hpECM cultures were over-confluent by day 10. MTS assay performed at different time points confirmed active and steady proliferation of AMDACs on hpECM. Metabolic activity, relative to cell number, increased 3 fold from day 1 to day 10 of culture (Figure 1).

<u>Cell Migration:</u> The number of migrated keratinocytes was the highest in the presence of AMDAC-hpECM cultures vs. AMDAC cultured on the plate (Figure 2). Also, the results showed that presence of hpECM alone tends to stimulate keratinocyte migration as compared to medium alone.

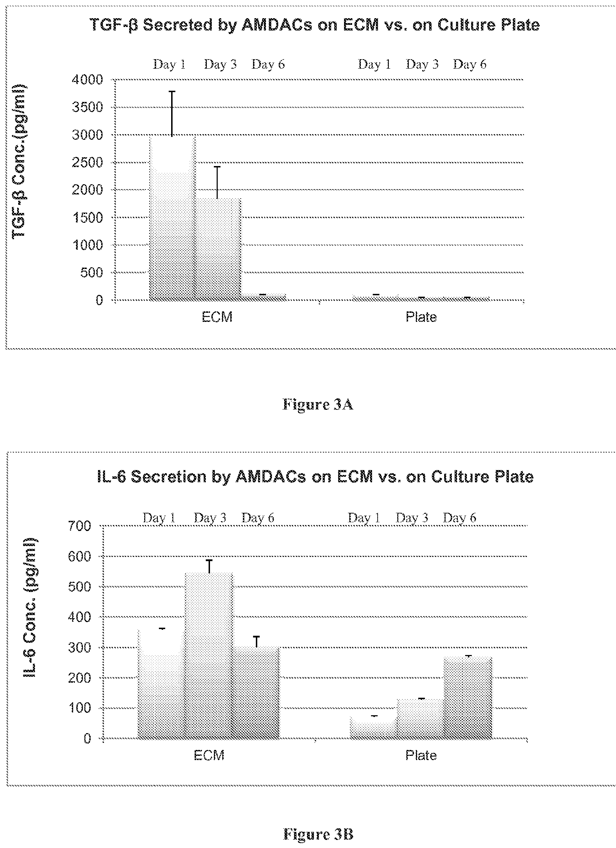

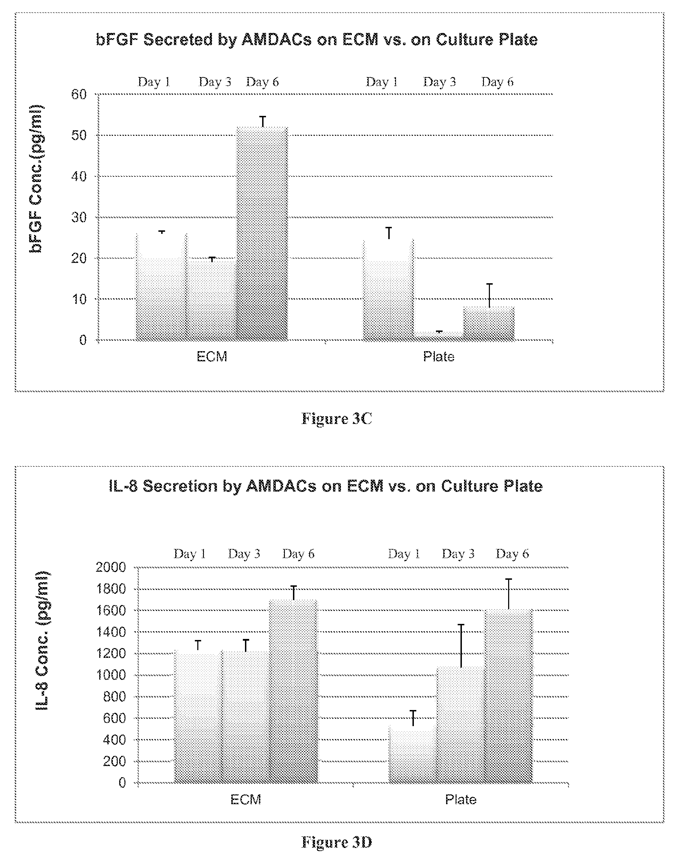

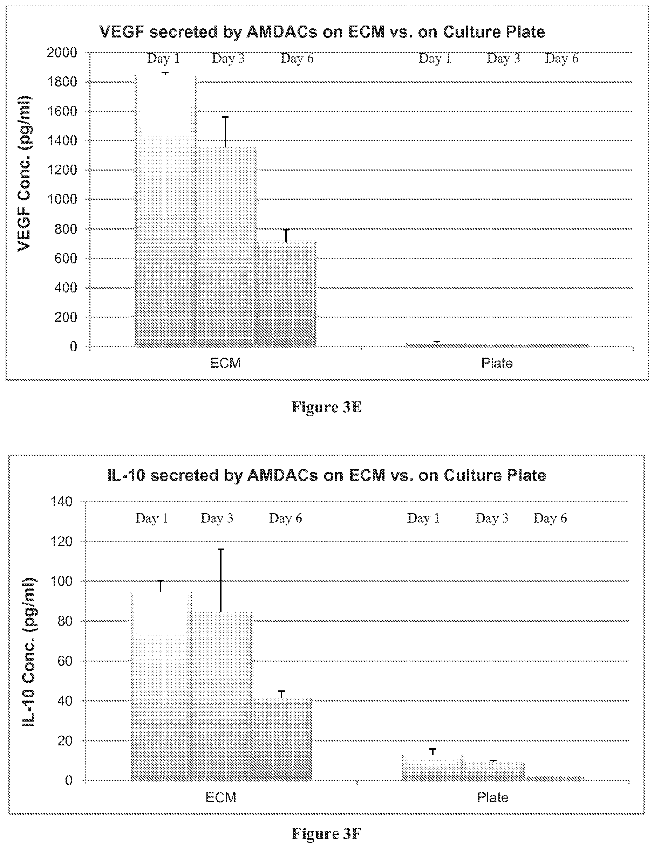

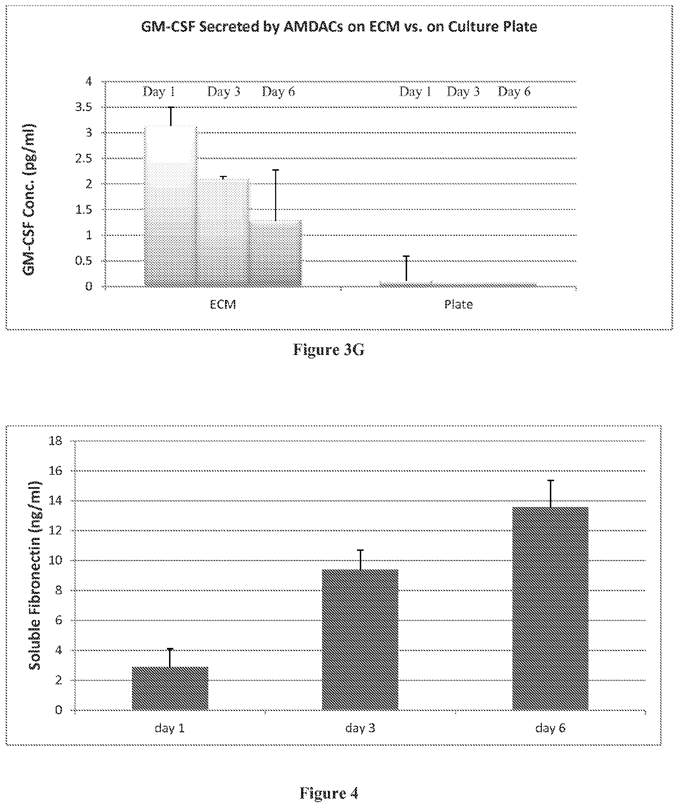

<u>Cytokine Secretion Profile:</u> 7 out of 15 cytokines tested in AMDAC culture media showed an up-regulation in secretion on hpECM vs. on culture plate. These cytokines included IL-6, IL-8, IL-10, bFGF, TGF-β, VEGF, and GM-SCF (Figures 3A-3G). IL-8 and IL-6, generally considered to be pro-inflammatory cytokines, showed significantly increased expression on hpECM vs. on plate at the early time point, while the levels were similar at later time points. bFGF displayed the opposite trend, with significantly higher expression levels at later time points. As for TGF-b and VEGF, significant expression levels were observed in AMDAC cultures on hpECM while these markers were barely detectable in cultures on plate. IL-10, displayed at least 6 fold higher expression levels at all time points in cultures on hpECM vs. on plate.

<u>Soluble Fibronectin Analysis:</u> AMDACs-hpECM cultures showed increasing secretion of soluble fibronectin from day 1 to day 6 of culture (Figure 4).

<u>Immunohistochemical Analysis:</u> Unlike staining performed on hpECM alone, AMDACs-hpECM staining (as shown by DAPI) showed positive signals in RFP and FITC, respectively associated with fibronectin and laminin proteins. These signals were further co-localized as shown by the yellow signal in merged images. These results demonstrate that AMDACs cultured on hpECM express and assemble abundant amounts of fibronectin and laminin.

<u>AMDAC - HUVEC Co-Culture:</u> AMDAC - HUVEC co-culture on hpECM showed that HUVEC-RFP cells seeded, after pre-establishing the AMDAC-GFP cultures for 24h, preferably grow in close proximity of AMDAC-GFP cells, which are most probably areas rich in cell assembled fibronectin and laminin.

In separate experiments, attempts to grow cells on placental ECM that had been lyophilized but not rehydrated and heat-dried, or that had been heat-dried only, but such attempts were inferior to results obtained using ECM as prepared in Example 1, e.g., there was significantly less cell proliferation than in the ECM prepared as described in Example 1.

### 6.2.3 Conclusions

Double-dried ECM generated herein is stable and supports cell adherence, proliferation and relatively long-term cell culture. Double-dried ECM sheets, e.g., act not only as a structural support to cells, but also as an inducer of matrix-mediated cellular signaling, critical to the normal wound healing processes. The amnion derived adherent cells (AMDACs) on the double-dried ECM sheets were stimulated to create a fibronectin and laminin matrix that can guide endogenous cell migration, proliferation and differentiation. In addition, AMDACs cultured on double-dried ECM deliver key "wound-healing" growth factors and cytokines to the wound, including TGF-β, VEGF, bFGF, IL-6, IL-8, IL-10 and GM-CSF

. Thus, double-dried ECM, e.g., double-dried placental ECM, e.g., sheets can serve as engineered wound covers not only providing support for endogenous cell migration, proliferation and differentiation, but also delivering AMDACs, and hence important wound healing cytokines and chemoknies to the wound site. Such compositions, therefore, can find use in treating burns and particularly in teating non-healing wounds characterized by a dysfunctional ECM that does not support normal wound healing process (Bennett & Schultz., 1993).

**Claims**

1. A method of producing an extracellular matrix (ECM) composition, comprising in order:

   decellularizing a tissue to produce decellularized ECM; freezing the ECM;
   lyophilizing the ECM; rehydrating the ECM; and
   drying the ECM at a temperature of 40°C to 70°C to produce said ECM composition,
   wherein said method does not comprise modification of the ECM with a protease.

2. The method of claim 1, wherein said drying of the ECM is performed at between 50°C to 70°C.

3. The method of clam 1 or claim 2, wherein said ECM is decellularized by

   (i) osmotic shock;
   (ii) using a detergent; or
   (iii) using a combination of osmotic shock and detergent.

4. The method of claim 3 item (iii), wherein said ECM is additionally decellularized using a base.

5. The method of claim 3 item (ii) or item (iii), or claim 4, wherein said detergent is deoxycholic acid.

6. The method of claim 4, wherein said base is ammonium hydroxide, potassium hydroxide, or sodium hydroxide, preferably wherein said base is used at a concentration of less than 0.5M.

7. The method of claim 1, wherein said extracellular matrix comprises collagen and one or more of laminin, fibronectin, elastin, and glycosaminoglycan.

8. An extracellular matrix (ECM) for use in a method of treating a lesion in an individual, wherein the method comprises administering said ECM to the individual, or contacting the individual with said ECM, wherein the ECM is prepared by the method of any of claims 1-7.

9. The ECM for use of claim 8, wherein the lesion is an oral lesion, wherein preferably

   (i) said oral lesion is caused by a dental procedure; or
   (ii) said oral lesion is not caused by a dental procedure.

10. The ECM for use of claim 9, wherein said oral lesion is caused by or is associated with administration of a chemotherapeutic agent to said individual.

11. The ECM for use of claim 10, wherein said chemotherapeutic agent is:

    (a) an alkylating agent, wherein preferably said alkylating agent is one or more of melphalan, busulfan, cisplatin, carboplatin, cyclophosphamide, dacarbazine, ifosfamide, or mechlorethamine;
    (b) an anti-metabolite, wherein preferably said anti-metabolite is one or more of 5-fluorouracil, methotrexate, gemcitabine, cytarabine, or fludarabine;
    (c) an antibiotic having anti-tumor effect, wherein preferably said antibiotic having anti-tumor effect is one or more of bleomycin, dactinomycin, daunorubicin, doxorubicin, or idarubicin; or
    (d) a mitotic inhibitor, wherein preferably said mitotic inhibitor is one or more of paclitaxel, docetaxel, etoposide, vinblastine, vincristine or vinorelbine.

12. The ECM for use of claim 10, wherein said oral lesion, or a plurality of said oral lesions, has caused or is expected to cause premature termination of a course of therapy comprising said chemotherapeutic agent.

13. The ECM for use of claim 9, wherein said oral lesion is:

    (a) caused by or is associated with administration of an antibody to said individual, wherein preferably said antibody is one or more of rituximab, ofatumumab, veltuzumab, ocrelizumab, adalimumab, etanercept, infliximab, certolizumab pegol, natalizumab or golimumab;

(b) caused by or is associated with hematopoietic stem cell transplantation, or bone marrow transplantation, to said individual;

(c) caused by or is associated with graft-versus-host disease in said individual;

(d) caused by or is associated with radiation that has been administered to said individual;

(e) caused by a desquamating oral disorder; or

(f) an aphthous ulcer.

14. The ECM for use of any of claims 8 to 13, wherein said ECM comprises a plurality of stem cells, wherein preferably said stem cells are CD10+, CD34-CD105+, CD100+ placental stem cells.

15. The ECM for use of claim 8, wherein said ECM is used as

(i) a nerve guide,

(ii) a tendon wrap, or

(iii) a dural replacement.


**Patentansprüche**

1. Verfahren zur Herstellung einer extrazellulären Matrix- (ECM) Zusammensetzung, umfassend in der Reihenfolge:

Dezellularisieren eines Gewebes zur Herstellung dezellularisierter ECM; Gefrieren der ECM; Gefriertrocknen der ECM; Rehydratisieren der ECM; und
Trocknen der ECM bei einer Temperatur von 40 °C bis 70 °C zur Herstellung der ECM-Zusammensetzung, wobei das Verfahren keine Modifizierung der ECM mit einer Protease umfasst.

2. Verfahren nach Anspruch 1, wobei das Trocknen der ECM zwischen 50 °C bis 70 °C ausgeführt wird.

3. Verfahren nach Anspruch 1 oder Anspruch 2, wobei die ECM dezellularisiert wird durch

(i) osmotischen Schock;

(ii) Verwendung eines Detergens; oder

(iii) Verwendung einer Kombination aus osmotischem Schock und Detergens.

4. Verfahren nach Anspruch 1 Punkt (iii), wobei die ECM zusätzlich unter Verwendung einer Base dezellularisiert wird.

5. Verfahren nach Anspruch 3 Punkt (ii) oder Punkt (iii) oder Anspruch 4, wobei das Detergens Desoxycholinsäure ist.

6. Verfahren nach Anspruch 4, wobei die Base Ammoniumhydroxid, Kaliumhydroxid oder Natriumhydroxid ist, wobei die Base vorzugsweise bei einer Konzentration von weniger als 0,5 M verwendet wird.

7. Verfahren nach Anspruch 1, wobei die extrazelluläre Matrix Collagen und eines oder mehrere von Laminin, Fibronectin, Elastin und Glycosaminoglycan umfasst.

8. Extrazelluläre Matrix (ECM) zur Verwendung in einem Verfahren zum Behandeln einer Läsion in einem Individuum, wobei das Verfahren Verabreichen der ECM an das Individuum oder Kontaktieren des Individuums mit der ECM umfasst, wobei die ECM durch das Verfahren nach einem der Ansprüche 1-7 zubereitet worden ist.

9. ECM zur Verwendung nach Anspruch 8, wobei die Läsion eine orale Läsion ist, wobei vorzugsweise

(i) die orale Läsion durch ein dentales Verfahren verursacht wird; oder

(ii) die orale Läsion durch kein dentales Verfahren verursacht wird.

10. ECM zur Verwendung nach Anspruch 9, wobei die orale Läsion verursacht wird durch oder verbunden ist mit einer Verabreichung eines chemotherapeutischen Mittels an das Individuum.

11. ECM zur Verwendung nach Anspruch 10, wobei das chemotherapeutische Mittel ist:

(a) ein Alkylierungsmittel, wobei das Alkylierungsmittel vorzugsweise eines oder mehrere von Melphalan, Busulfan, Cisplatin, Carboplatin, Cyclophosphamid, Dacarbazin, Ifosfamid oder Mechlorethamin ist;

(b) ein Antimetabolit, wobei der Antimetabolit vorzugsweise einer oder mehrere von 5-Fluoruracil, Methotrexat, Gemcitabin, Cytarabin oder Fludarabin ist;

(c) ein Antibiotikum mit Antitumorwirkung, wobei das Antibiotikum mit Antitumorwirkung vorzugsweise eines oder mehrere von Bleomycin, Dactinomycin, Daunorubicin, Doxorubicin oder Idarubicin ist; oder

(d) ein Mitoseinhibitor, wobei der Mitoseinhibitor vorzugsweise einer oder mehrere von Paclitaxel, Docetaxel, Etoposid, Vinblastin, Vincristin oder Vinorelbin ist.

12. ECM zur Verwendung nach Anspruch 10, wobei die orale Läsion oder eine Mehrzahl der oralen Läsionen verursacht oder erwartet wird, eine vorzeitige Beendigung eines das chemotherapeutische Mittel umfassenden Therapieverlaufs zu verursachen.

13. ECM zur Verwendung nach Anspruch 9, wobei die orale Läsion ist:

(a) verursacht durch eine oder verbunden ist mit einer Verabreichung eines Antikörpers für das Individuum, wobei der Antikörper vorzugsweise einer oder mehrere von Rituximab, Ofatumumab, Veltuzumab, Ocrelizumab, Adalimumab, Etanercept, Infliximab, Certolizumab Pegol, Natalizumab oder Golimumab ist;

(b) verursacht durch eine oder verbunden mit einer blutbildenden Stammzellen-Transplantation oder Knochenmark-Transplantation für das Individuum ist;

(c) verursacht durch eine oder verbunden ist mit einer Transplantat-Wirts-Erkrankung in dem Individuum;

(d) verursacht durch eine oder verbunden mit einer Strahlung ist, die an das Individuum verabreicht wurde;

(e) verursacht durch eine desquamierende orale Störung; oder

(f) eine Aphthe.

14. ECM zur Verwendung nach einem der Ansprüche 8 bis 13, wobei die ECM eine Mehrzahl von Stammzellen umfasst, wobei die Stammzellen vorzugsweise CD10+, CD34-CD105+, CD100+ Plazentastammzellen sind.

15. ECM zur Verwendung nach Anspruch 8, wobei die ECM verwendet wird als

(i) eine Nervenleitung,

(ii) eine Sehnenscheide, oder

(iii) ein Dural-Ersatz.

**Revendications**

1. Procédé de production d'une composition de matrice extracellulaire (ECM), comprenant dans l'ordre :

une décellularisation d'un tissu pour produire une ECM décellularisée ; une congélation de l'ECM ; une lyophilisation de l'ECM ; et

un séchage de l'ECM à une température de 40° C à 70° C pour produire ladite composition d'ECM,

dans lequel ledit procédé ne comprend pas une modification de l'ECM par une protéase.

2. Procédé selon la revendication 1, dans lequel ledit séchage de l'ECM est effectué entre 50° C et 70° C.

3. Procédé selon la revendication 1 ou la revendication 2, dans lequel ladite ECM est décellularisée par

(i) un choc osmotique ;

(ii) une utilisation d'un détergent ; ou

(iii) une utilisation d'une combinaison d'un choc osmotique et d'un détergent.

4. Procédé selon le point (iii) de la revendication 3, dans lequelladite ECM est en outre décellularisée par une utilisation d'une base.

5. Procédé selon le point (ii) ou le point (iii) de la revendication 3 ou la revendication 4, dans lequel ledit détergent est l'acide désoxycholique.

**6.** Procédé selon la revendication 4, dans lequel ladite base est l'hydroxyde d'ammonium, l'hydroxyde de potassium, ou l'hydroxyde de sodium, de préférence dans lequel ladite base est utilisée à une concentration inférieure à 0,5 M.

**7.** Procédé selon la revendication 1, dans lequel ladite matrice extracellulaire comprend le collagène et un ou plusieurs parmi la laminine, la fibronectine, l'élastine, et une glycosaminoglycane.

**8.** Matrice extracellulaire (ECM) destinée à être utilisée dans une méthode de traitement d'une lésion chez un individu, dans laquelle la méthode comprend une administration de ladite ECM à l'individu, ou une mise en contact de l'individu avec ladite ECM, dans laquelle l'ECM est préparée par le procédé selon l'une quelconque des revendications 1 à 7.

**9.** ECM destinée à être utilisée selon la revendication 8, dans laquelle la lésion est une lésion buccale, dans laquelle, de préférence,

> (i) ladite lésion buccale est provoquée par une intervention dentaire ; ou
> (ii) ladite lésion buccale n'est pas provoquée par une intervention dentaire.

**10.** ECM destinée à être utilisée selon la revendication 9, dans laquelle ladite lésion buccale est provoquée par ou est associée à une administration d'un agent de chimiothérapie audit individu.

**11.** ECM destinée à être utilisée selon la revendication 10, dans laquelle ledit agent de chimiothérapie est :

> (a) un agent alkylant, dans laquelle, de préférence, ledit agent alkylant est un ou plusieurs parmi le melphalan, le busulfan, le cisplatine, le carboplatine, le cyclophosphamide, la dacarbazine, l'ifosfamide, ou la mechloréthamine ;
> (b) un antimétabolite, dans laquelle, de préférence, ledit antimétabolite est un ou plusieurs parmi le 5-fluorouracile, le méthotrexate, la gemcitabine, la cytarabine, ou la fludarabine ;
> (c) un antibiotique ayant un effet antitumoral, dans laquelle, de préférence, ledit antibiotique ayant un effet antitumoral est un ou plusieurs parmi la bléomycine, la dactinomycine, la daunorubicine, la doxorubicine, ou l'idarubicine ; ou
> (d) un inhibiteur mitotique, dans laquelle, de préférence, ledit inhibiteur mitotique est un ou plusieurs parmi le paclitaxel, le docétaxel, l'étopiside, la vinblastine, la vincristine ou la vinorelbine.

**12.** ECM destinée à être utilisée selon la revendication 10, dans laquelle ladite lésion buccale, ou une pluralité desdites liaisons buccales, a provoqué ou est censée provoquer l'arrêt prématuré du cours d'une thérapie comprenant ledit agent de chimiothérapie.

**13.** ECM destinée à être utilisée selon la revendication 9, dans laquelle ladite lésion buccale est :

> (a) provoquée par ou est associée à une administration d'un anticorps audit individu, dans laquelle, de préférence, ledit anticorps est un ou plusieurs parmi le rituximab, l'ofatumumab, le veltuzumab, l'ocrelizumab, l'adalimumab, l'etanercept, l'infliximab, le certolizumab pegol, le natalizumab ou le golimumab ;
> (b) provoquée par ou est associée à une transplantation de cellules souches hématopoïétiques, ou une transplantation de moelle osseuse, audit individu ;
> (c) provoquée par ou est associée à une maladie du greffon contre l'hôte dans ledit individu ;
> (d) provoquée par ou est associée à un rayonnement qui a été administré audit individu ;
> (e) provoquée par un trouble buccal desquamant ; ou
> (f) un ulcère aphteux.

**14.** ECM destinée à être utilisée selon l'une quelconque des revendications 8 à 13, dans laquelle ladite ECM comprend une pluralité de cellules souches, dans laquelle, de préférence, lesdites cellules souches sont des cellules souches placentaires CD10+, CD34-CD105+, CD100+.

**15.** ECM destinée à être utilisée selon la revendication 8, dans laquelle ladite ECM est utilisée en tant que :

> (i) guide nerveux,
> (ii) enveloppe pour tendon, ou
> (iii) remplaçant dural.

Figure 1

Figure 2

Figure 3A

Figure 3B

## bFGF Secreted by AMDACs on ECM vs. on Culture Plate

Figure 3C

## IL-8 Secretion by AMDACs on ECM vs. on Culture Plate

Figure 3D

**Figure 3E**

**Figure 3F**

Figure 3G

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4852640 A
- US 5428022 A
- US 5660692 A
- US 5008116 A
- US 5880242 A
- US 6117979 A
- US 20030049301 A
- US 20040048796 A
- US 20030187515 A
- US 4511653 A
- US 4582640 A
- US 5436135 A
- US 6548077 B
- US 4185011 A
- US 4597762 A

- US 5412076 A
- US 5763579 A
- US 4642117 A
- US 7045148 B
- US 7468276 B
- US 8057788 B
- US 8202703 B
- US 20070275362 A
- US 3157524 A
- US 4320201 A
- US 3800792 A
- US 4837285 A
- US 5116620 A
- US 8367409 B

**Non-patent literature cited in the description**

- **MCPHERSON et al.** *J. Biomedical Materials Res.,* 1986, vol. 20, 79-92
- **ZEEMAN et al.** *J Biomed Mater Res.,* 2000, vol. 51 (4), 541-8
- **VAN WACHEM et al.** *J Biomed Mater Res.,* 2000, vol. 53 (1), 18-27
- **VAN WACHEM et al.** *J Biomed Mater Res.,* 1999, vol. 47 (2), 270-7
- **ZEEMAN et al.** *J Biomed Mater Res.,* 1999, vol. 46 (3), 424-33
- **ZEEMAN et al.** *Biomaterials,* 1999, vol. 20 (10), 921-31
- **ORBAN et al.** *J. Biomedical Materials Res.,* 2004, vol. 68 (4), 756-62
- **COTORRUELO et al.** *Clin. Lab.,* 2002, vol. 48 (5 6), 271-81
- **MAINE et al.** *Expert Rev. Mol. Diagn.,* 2001, vol. 1 (1), 19-29
- **NIELSEN et al.** *J. Clin. Microbiol.,* 1987, vol. 25 (8), 1406-10
- **LAYNE, E.** *Spectrophotometric and Turbidimetric Methods for Measuring Proteins, Methods in Enzymology,* 1957, vol. 3, 447-455
- **STOSCHECK, C M.** *Quantitation of Protein, Methods in Enzymology,* 1990, vol. 182, 50-69
- **SCOPES, R K.** *Analytical Biochemistry,* 1974, vol. 59, 277
- **STOSCHECK, C M.** *Quantitation of Protein, Methods in Enzymology,* 1990, vol. 182, 50-69

- **BRADFORD, M.** *Analytical Biochemistry,* 1976, vol. 72, 248
- **WINKLEMAN, J.** *Cancer Research,* 1962, vol. 22, 589-596
- Biomaterials. Stockton Press, 1991, 55-122
- The Pharmacological Basis of Therapeutics. Macmillan Publishing
- Katzung, Basic & Clinical Pharmacology. Appleton & Lang, Norwalk, Conn, 1995
- **GRAHAM.** *Med. Device Technol.,* 1998, vol. 9 (1), 18-22
- **PEPPAS et al.** *Eur. J. Pharm. Biopharm.,* 2000, vol. 50 (1), 27-46
- **NGUYEN et al.** *Biomaterials,* 2002, vol. 23 (22), 4307-14
- **HENINCL et al.** *Adv. Drug Deliv. Rev,* 2002, vol. 54 (1), 13-36
- **SKELHORNE et al.** *Med. Device. Technol.,* 2002, vol. 13 (9), 19-23
- **SCHMEDLEN et al.** *Biomaterials,* 2002, vol. 23, 4325-32
- **KAMARCH.** *Methods Enzymol,* 1987, vol. 151, 150-165
- **SONIS et al.** *Cancer,* 1999, vol. 85 (10), 2103-2113
- **DOILLON et al.** *Biomaterials,* 1987, vol. 8, 195-200
- **DOILLON ; SILVER.** *Biomaterials,* 1986, vol. 7, 3-8

- **QUTEISH et al.** *J. Clin. Periodontol.,* 1992, vol. 19 (7), 476-84
- **CHUNG et al.** *J. Periodontol.,* 1990, vol. 61 (12), 732-6
- **MATTSON et al.** *J. Periodontol.,* 1995, vol. 66 (7), 635-45
- **BENQUE et al.** *J. Clin. Periodontol.,* 1997, vol. 24 (8), 544-9
- **MATTSON et al.** *J. Periodontol.,* 1999, vol. 70 (5), 510-7
- **PAUL et al.** *Int. J. Periodontics Restorative Dent.,* 1992, vol. 12, 123-31
- **WANG et al.** *J. Periodontol.,* 1994, vol. 65, 1029-36
- **BLUMENTHAL.** *J. Periodontol.,* 1993, vol. 64, 925-33
- **BLACK et al.** *J. Periodontol.,* 1994, vol. 54, 598-604
- **YUKNA et al.** *J. Periodontol.,* 1995, vol. 67, 650-7
- **SHIEH et al.** *J. Periodontol.,* 1997, vol. 68, 770-8
- **ZAHEDI et al.** *J. Periodontol.,* 1998, vol. 69, 975-81
- **OZCAN et al.** *J. Marmara Univ. Dent. Fa.,* 1997, vol. 2, 588-98
- **WANG et al.** *J. Dent. Res.,* 1997, vol. 78, 119